(19) Europäisches Patentamt
European Patent Office
Office européen des brevets

(11) EP 1 077 938 B1

(12) **EUROPÄISCHE PATENTSCHRIFT**

(45) Veröffentlichungstag und Bekanntmachung des Hinweises auf die Patenterteilung:
**13.04.2005 Patentblatt 2005/15**

(21) Anmeldenummer: **99923526.0**

(22) Anmeldetag: **05.05.1999**

(51) Int Cl.7: **C07D 207/20**, C07D 409/10, C07D 405/10, C07C 271/18, C07C 205/45

(86) Internationale Anmeldenummer:
**PCT/EP1999/003062**

(87) Internationale Veröffentlichungsnummer:
**WO 1999/059967 (25.11.1999 Gazette 1999/47)**

(54) **2-(2-METHYLPHENYL)-3,4-DIHYDRO-2H-PYRROL-DERIVATE**

2-(2-METHYLPHENYL)-3,4-DIHYDRO-2H-PYRROL DERIVATIVES

DERIVES DE 2-(2-METHYLPHENYL)-3,4-DIHYDRO-2H-PYRROL

(84) Benannte Vertragsstaaten:
**AT BE CH DE DK ES FI FR GB GR IE IT LI NL PT SE**

(30) Priorität: **18.05.1998 DE 19822245**

(43) Veröffentlichungstag der Anmeldung:
**28.02.2001 Patentblatt 2001/09**

(73) Patentinhaber: **Bayer CropScience AG**
**40789 Monheim (DE)**

(72) Erfinder:
- **PLANT, Andrew**
**D-51373 Leverkusen (DE)**
- **BACKHAUS, Dirk**
**D-50672 Köln (DE)**
- **ERDELEN, Christoph**
**D-42799 Leichlingen (DE)**
- **TURBERG, Andreas**
**D-42781 Haan (DE)**
- **MENCKE, Norbert**
**D-51381 Leverkusen (DE)**

(56) Entgegenhaltungen:
**WO-A-98/22438**

- **E. PROFFT ET AL.: JOURNAL FÜR PRAKTISCHE CHEMIE, 4. REIHE, Bd. 1, 1954, Seiten 57-86, XP002113754 in der Anmeldung erwähnt**
- **W. KOLLER ET AL.: CHEMISCHE BERICHTE, Bd. 96, 1963, Seiten 93-113, XP002113755 in der Anmeldung erwähnt**
- **C. F. H. ALLEN ET AL.: JOURNAL OF THE AMERICAN CHEMICAL SOCIETY, Bd. 51, Nr. 7, 1929, Seiten 2153-57, XP002113756**
- **R. WEIL ET AL.: BULL. SOC. CHIM. FRANCE, Nr. 1-2, 1974, Seiten 258-62, XP002113757 in der Anmeldung erwähnt**
- **H. QUAST ET AL.: CHEMISCHE BERICHTE, Bd. 116, 1983, Seiten 3931-46, XP002113758 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 077 938 B1

Printed by Jouve, 75001 PARIS (FR)

## Beschreibung

Die Erfindung betrifft neue (2-(2-Methylphenyl)-3,4-dihydro-2H-pyrrol-Derivate, mehrere Verfahren zu ihrer Herstellung und ihre Verwendung als Schädlingsbekämpfungsmittel.

Bisher sind nur wenige substituierte cyclische α,α'-Diphenylimine bekannt geworden: Drei im 2-Phenylring alkoxysubstituierte 2,5-Diphenyl-1-pyrroline [5-(2,5-Dimethoxyphenyl)-2-phenyl-3,4-dihydro-2H-pyrrol und 5-(4-Methoxyphenyl)-2-phenyl-3,4-dihydro-2H-pyrrol aus Chem. Ber. 96, 93 (1963) und die entsprechende 4-Propoxy-Verbindung aus J. Prakt. Chem., Serie 4, 1, 57 (1954)] sowie das nicht weiter substituierte 2,6-Diphenyl-3,4,5,6-tetrahydropyridin [vgl. z.B. Bull. Soc. Chim. Fr. **1974**, 258 u. Chem. Ber. **116**, 3931 (1983)].

Über deren Eignung zur Verwendung als Schädlingsbekämpfungsmittel ist nichts bekannt.

Es wurden neue 2-(2-Methylphenyl)-3,4-dihydro-2H-pyrrol-Derivate der Formel (I)

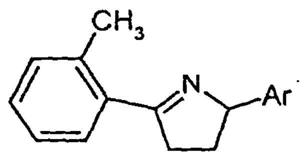

(I)

gefunden, in welcher

Ar für den Rest

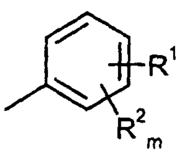

steht, in welchem

m für 0, 1, 2, 3 oder 4 steht,

$R^1$ für einen Substituenten in meta- oder para-Position aus der Reihe Wasserstoff, F, Cl, Br, Cyano, Tri-($C_1$-$C_6$-alkyl)-silyl, -CO-$NR^4R^5$, Tetrahydropyranyl oder eine der folgenden Gruppierungen

(l) -X-A

(m) -B-Z-D

(n) -Y-E

steht,

$R^2$ für Wasserstoff, Halogen, Cyano, Nitro, $C_1$-$C_{16}$-Alkyl, $C_1$-$C_{16}$-Alkoxy, $C_1$-$C_6$-Halogenalkyl, $C_1$-$C_6$-Halogenalkoxy, $C_1$-$C_8$-Alkoxy-$C_1$-$C_8$-alkoxy oder -$S(O)_oR^3$,

mit der Maßgabe, dass $R^1$ nicht für Wasserstoff steht, wenn m = 1 und $R^2$ = Iod, steht,

o für 0, 1 oder 2 steht,

$R^3$ für gegebenenfalls durch Fluor oder Chlor substituiertes $C_1$-$C_6$-Alkyl steht,

$R^4$ und $R^5$ unabhängig voneinander für Wasserstoff, $C_1$-$C_6$-Alkyl, $C_1$-$C_6$-Halogenalkyl oder für jeweils gegebenenfalls einfach bis dreifach durch Reste aus der Liste $W^1$ substituiertes Phenyl oder Phenyl-$C_1$-$C_4$-alkyl stehen,

X für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder Di-$C_1$-$C_4$-alkylsilylen steht,

A für jeweils gegebenenfalls einfach bis vierfach durch Reste aus der Liste $W^1$ substituiertes Phenyl, Naphthyl oder Tetrahydronaphthyl oder für jeweils gegebenenfalls einfach bis vierfach durch Reste aus der Liste $W^2$ substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesonder Furyl, Benzofuryl, Thienyl, Benzothienyl, Oxazolyl, Benzoxazolyl, Thiazolyl, Benzothiazolyl, Pyrrolyl, Pyridyl, Pyrimidyl, 1,3,5-Triazinyl, Quinolinyl, Isoquinolinyl, Indolyl, Purinyl, Benzodioxolyl, Indanyl, Benzodioxanyl oder Chromanyl) steht,

B für gegebenenfalls einfach oder zweifach durch Reste aus der Liste $W^1$ substituiertes p-Phenylen steht,

Z für Sauerstoff oder Schwefel steht,

D für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, jeweils gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl oder $C_3$-$C_8$-Cycloalkyl-$C_1$-$C_6$-alkyl, für jeweils gegebenenfalls durch Halogen oder $C_1$-$C_4$-Alkyl substituiertes $C_5$-$C_8$-Cycloalkenyl oder $C_5$-$C_8$-Cycloalkenyl-$C_1$-$C_4$-alkyl, für jeweils gegebenenfalls durch Nitro, Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl-$C_1$-$C_6$-alkyl, Naphthyl-$C_1$-$C_6$-alkyl, Tetrahydronaphthyl-$C_1$-$C_6$-alkyl oder 5- oder 6-ringgliedriges Hetaryl-$C_1$-$C_6$-alkyl mit 1 oder 2 Heteroatomen aus der Reihe Stickstoff, Sauerstoff und Schwefel (insbesondere Furylmethyl, Thienylmethyl, Pyrrolylmethyl, Oxazolylmethyl, Isoxazolylmethyl, Thiazolylmethyl oder Pyridylmethyl), für -CO-$R^6$, -CO-$NR^7R^8$ oder für die Gruppierung

$$-(CH_2)_p-(CR^9R^{10})_q-(CH_2)_r-G$$

steht,

Z und D können auch gemeinsam für jeweils gegebenenfalls durch Nitro, Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenoxy-$C_1$-$C_4$-alkyl stehen,

Y für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder für gegebenenfalls einfach oder zweifach durch Reste aus der Liste $W^1$ substituiertes p-Phenylen steht,

E für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl, für gegebenenfalls durch Halogen oder $C_1$-$C_4$-Alkyl substituiertes $C_5$-$C_8$-Cycloalkenyl, für gegebenenfalls einfach bis vierfach durch Reste aus der Liste $W^1$ substituiertes Phenyl oder für gegebenenfalls einfach bis vierfach durch Reste aus der Liste $W^2$ substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung

$$-(CH_2)_p-(CR^9R^{10})_q-(CH_2)_r-G$$

steht,

$R^6$ für $C_1$-$C_{12}$-Alkyl, $C_1$-$C_{12}$-Alkoxy, $C_2$-$C_{12}$-Alkenyl, $C_2$-$C_{12}$-Alkenyloxy, jeweils gegebenenfalls durch Halogen, $C_1$-$C_4$-Alkyl, $C_2$-$C_4$-Alkenyl, $C_1$-$C_4$-Halogenalkyl oder $C_2$-$C_4$-Halogenalkenyl substituiertes $C_3$-$C_8$-Cycloalkyl, $C_3$-$C_8$-Cycloalkyloxy oder $C_3$-$C_8$-Cycloalkyl-$C_1$-$C_6$-alkyloxy oder für jeweils gegebenenfalls einfach bis vierfach durch Nitro, Halogen, $C_1$-$C_{12}$-Alkyl, $C_1$-$C_{12}$-Alkoxy, $C_1$-$C_{12}$-Halogenalkyl oder $C_1$-$C_{12}$-Halogenalkoxy substituiertes Phenyl oder Naphthyl steht,

$R^7$ für Wasserstoff oder $C_1$-$C_{12}$-Alkyl steht,

$R^8$ für $C_1$-$C_{12}$-Alkyl, $C_1$-$C_{12}$-Halogenalkyl, jeweils gegebenenfalls durch Halogen, $C_1$-$C_4$-Alkyl, $C_2$-$C_4$-Alkenyl, $C_1$-$C_4$-Halogenalkyl oder $C_2$-$C_4$-Halogenalkenyl substituiertes $C_3$-$C_8$-Cycloalkyl oder $C_3$-$C_8$-Cycloalkyl-$C_1$-$C_6$-alkyl oder für jeweils gegebenenfalls einfach bis vierfach durch Halogen, $C_1$-$C_{12}$-Alkyl, $C_1$-$C_{12}$-Alkoxy, $C_1$-$C_{12}$-Halogenalkyl oder $C_1$-$C_{12}$-Halygenalkoxy substituiertes Phenyl oder Phenyl-$C_1$-$C_6$-alkyl steht,

p, q und r unabhängig voneinander für 0, 1, 2 oder 3 stehen, wobei deren Summe kleiner als 6 und größer als 1 ist,

$R^9$ und $R^{10}$ unabhängig voneinander für Wasserstoff oder $C_1$-$C_4$-Alkyl stehen,

G für Cyano, für einen gegebenenfalls einfach bis dreifach durch Halogen, $C_1$-$C_4$-Alkyl oder $C_1$-$C_4$-Halogenalkyl und gegebenenfalls an der Verknüpfungsstelle durch den Rest $R^{11}$ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder l,3-Thioxan-2-yl) oder eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

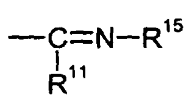

(f)

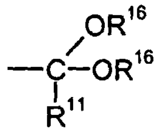

(g)

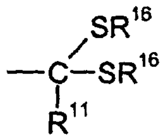

(h)

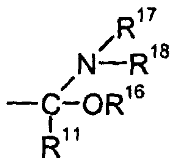

(i)

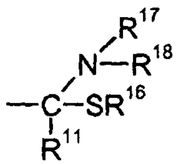

(j)

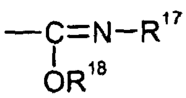

(k)

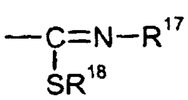

steht,

$R^{11}$ für Wasserstoff, $C_1$-$C_6$-Alkyl, $C_2$-$C_6$-Alkenyl, $C_1$-$C_4$-Halogenalkyl, $C_2$-$C_6$-Halogenalkenyl, gegebenenfalls durch Halogen, $C_1$-$C_4$-Alkyl oder $C_1$-$C_4$-Halogenalkyl substituiertes $C_3$-$C_6$-Cycloalkyl oder für gegebenenfalls einfach bis fünffach durch $C_1$-$C_4$-Alkylcarbonylamino, $C_1$-$C_4$-Allcylcarbonyl-$C_1$-$C_4$-alkylamino und/oder Reste aus der Liste $W^3$ substituiertes Phenyl steht,

$R^{12}$ für Wasserstoff, $C_1$-$C_4$-Alkyl, $C_2$-$C_6$-Alkenyl, $C_1$-$C_4$-Halogenalkyl, $C_2$-$C_6$-Halogenalkenyl, jeweils

gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl oder für gegebenenfalls einfach bis vierfach durch Reste aus der Liste $W^3$ substituiertes $C_6$-$C_{10}$-Aryl-$C_1$-$C_4$-alkyl (insbesondere Phenyl-$C_1$-$C_4$-alkyl oder Naphthyl-$C_1$-$C_4$-alkyl) steht,

$R^{13}$ und $R^{14}$ unabhängig voneinander für Wasserstoff, $C_1$-$C_4$-Alkyl, $C_3$-$C_6$-Alkenyl, $C_1$-$C_4$-Halogenalkyl, $C_3$-$C_6$-Halogenalkenyl, $C_1$-$C_4$-Alkoxy, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl, für jeweils gegebenenfalls einfach bis fünffach durch Reste aus der Liste $W^3$ substituiertes Phenyl oder Phenyl-$C_1$-$C_4$-alkyl, für $-OR^{12}$ oder $-NR^{11}R^{12}$ oder gemeinsam für eine Alkylenkette mit 4 bis 6 Gliedern, in welcher gegebenenfalls eine Methylengruppe durch Sauerstoff ersetzt ist, stehen,

$R^{15}$ für $-OR^{12}$, $-NR^{11}R^{12}$ oder $-N(R^{11})-COOR^{12}$ steht,

$R^{16}$, $R^{17}$ und $R^{18}$ unabhängig voneinander für $C_1$-$C_6$-Alkyl stehen,

$W^1$ für Wasserstoff, Halogen, Cyano, Formyl, Nitro, $C_1$-$C_6$-Alkyl, Tri-$C_1$-$C_4$-alkylsilyl, $C_1$-$C_{16}$-Alkoxy, $C_1$-$C_6$-Halogenalkyl, $C_1$-$C_6$-Halogenalkoxy, $C_2$-$C_6$-Halogenalkenyloxy, $C_1$-$C_6$-Alkylcarbonyl, $C_1$-$C_{16}$-Alkoxycarbonyl, Pentafluorthio oder $-S(O)_oR^3$ steht,

$W^2$ für Halogen, Cyano, Formyl, Nitro, $C_1$-$C_6$-Alkyl, Tri-$C_1$-$C_4$-alkylsilyl, $C_1$-$C_{16}$-Alkoxy, $C_1$-$C_6$-Halogenalkyl, $C_1$-$C_6$-Halogenalkoxy, $C_1$-$C_6$-Alkylcarbonyl, $C_1$-$C_{16}$-Alkoxycarbonyl, Pentafluorthio, $-S(O)_oR^3$ oder $-C(R^{11})=N-R^{15}$ steht,

$W^3$ für Halogen, Cyano, Nitro, $C_1$-$C_4$-Alkyl, $C_1$-$C_4$-Alkoxy, $C_1$-$C_4$-Halogenalkyl, $C_1$-$C_4$-Halogenalkoxy, Di-$C_1$-$C_4$-alkylamino, $-S(O)_oR^3$, $-COOR^{19}$ oder $-CONR^{20}R^{21}$ steht,

$R^{19}$ für Wasserstoff, $C_1$-$C_4$-Alkyl, $C_1$-$C_4$-Halogenalkyl, gegebenenfalls durch Halogen, $C_1$-$C_4$-Alkyl oder $C_1$-$C_4$-Halogenalkyl substituiertes $C_3$-$C_7$-Cycloalkyl oder für gegebenenfalls einfach bis fünffach durch Reste aus der Liste $W^4$ substituiertes Phenyl steht,

$R^{20}$ und $R^{21}$ unabhängig voneinander für Wasserstoff, $C_1$-$C_4$-Alkyl, $C_3$-$C_6$-Alkenyl, $C_1$-$C_4$-Halogenalkyl, $C_3$-$C_6$-Halogenalkenyl, $C_1$-$C_4$-Alkoxy, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl oder für jeweils gegebenenfalls einfach bis fünffach durch Reste aus der Liste $W^4$ substituiertes Phenyl oder Phenyl-$C_1$-$C_4$-alkyl, für $-OR^{16}$ oder $-NR^{17}R^{18}$ oder gemeinsam für eine Alkylenkette mit 4 bis 6 Gliedern, in welcher gegebenenfalls eine Methylengruppe durch Sauerstoff ersetzt ist, stehen, und

$W^4$ für Halogen, Cyano, Nitro, $C_1$-$C_6$-Alkyl, $C_1$-$C_6$-Alkoxy, $C_1$-$C_6$-Halogenalkyl, $C_1$-$C_6$-Halogenalkoxy, Di-$C_1$-$C_4$-alkylamino, $C_1$-$C_6$-Alkoxycarbonyl, Di-$C_1$-$C_6$-alkylaminocarbonyl oder $-S(O)_oR^3$ steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen Verbindungen der Formel (I) nach einem der im folgenden beschriebenen Verfahren erhält.

A) Cyclische Imine der Formel (I)

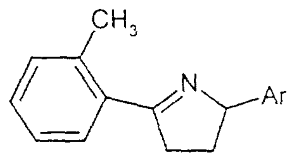

(I).

in welcher
Ar die oben angegebenen Bedeutungen hat,
lassen sich herstellen. indem man a) Aminoketonderivate der Formel (VIII)

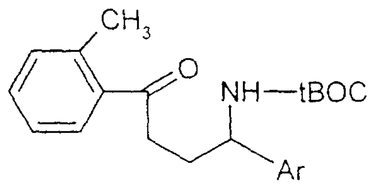

(II).

in welcher

Ar die oben angegebenen Bedeutungen hat,

mit einer Säure umsetzt und anschließend gegebenenfalls in Gegenwart eines Säurebindemittels cyclokondensiert, oder

b) die Nitrogruppe von Nitroketonen der Formel (XVIII),

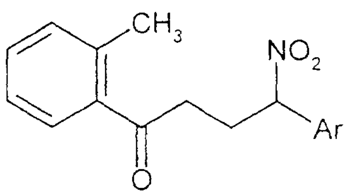

(XVIII)

in welcher
Ar die oben genannten Bedeutungen hat, reduziert, wobei

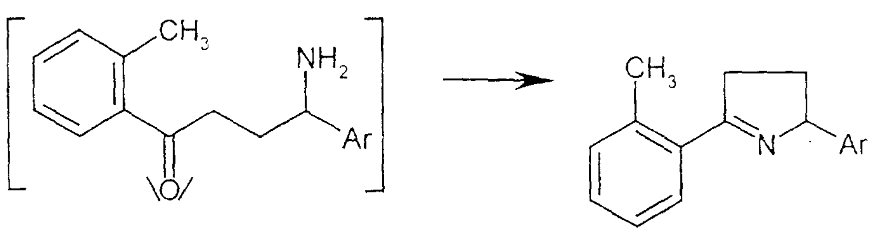

(II) (I)

intermediär ein Aminoketon der Formel (II) auftritt, welches jedoch, insbesondere in saurem Medium, in situ zu (I) cyclokondensiert wird, oder

c) Imine der Formel (XXVII)

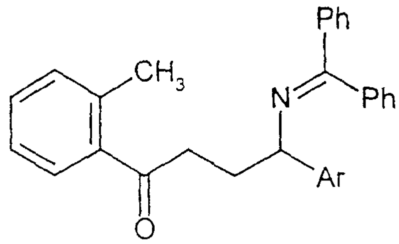

(XXVII)

in welcher

Ar die oben genannten Bedeutungen hat, mit wäßrigen Säuren hydrolysiert

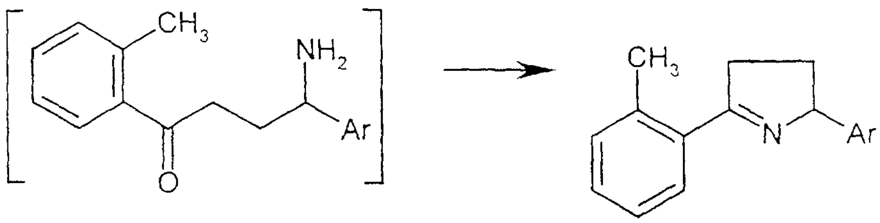

(II) (I)

wobei
intermediär ein Aminoketon der Formel (II) auftritt, welches jedoch in situ zu (I) cyclokondensiert wird.

B) Cyclische Imine der Formel (I) lassen sich auch herstellen, indem man cyclische *O*-Methylsulfonyloxime der Formel (III)

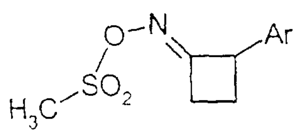

(III),

in welcher

Ar die oben angegebenen Bedeutungen hat,

mit der Aryl-Grignard-Verbindung der Formel (IV)

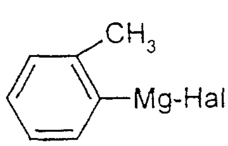

(IV),

in welcher

Hal für Chlor, Brom oder Iod steht,

in Gegenwart eines Verdünnungsmittels umsetzt.

C) Cyclische Imine der Formel (I-b)

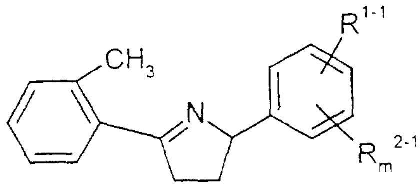

(I-b)

in welcher

m die oben angegebenen Bedeutungen hat.

$R^{1-1}$ für A oder eine der folgenden Gruppierungen

(m) -B-Z-D

(n-a)

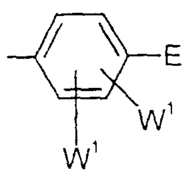

steht, wobei

A, B, D, E, $W^1$ und Z die oben angegebenen Bedeutungen haben und

$R^{2-1}$ für Wasserstoff. Fluor, Cyano, Nitro, Alkyl, Alkoxy, Halogenalkyl, Halogenalkoxy, Alkoxyalkoxy oder $-SR^3$ steht, wobei

$R^3$ die oben angegebene Bedeutung hat,

lassen sich herstellen, indem man Verbindungen der Formel (V)

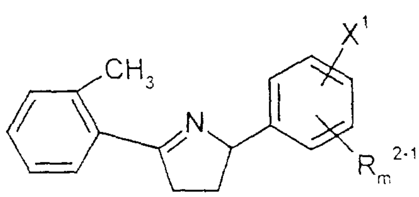

(V),

in welcher

$R^{2-1}$ und m die oben angegebenen Bedeutungen haben und

$X^1$ für Brom. Iod oder $-OSO_2CF_3$ steht

mit Boronsäuren der Formel (VI)

$$R^{1-1}\text{-}B(OH)_2 \qquad (VI),$$

in welcher

$R^{1-1}$ die oben angegebenen Bedeutungen hat,

in Gegenwart eines Katalysators und in Gegenwart eines Säurebindemittels und in Gegenwart eines Lösungsmittels kuppelt.

D) Cyclische Imine der Formel (I-c)

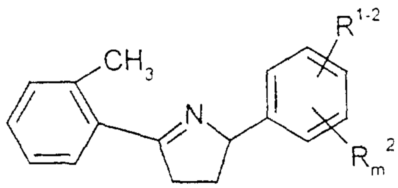

(I-c),

in welcher

$R^2$ und m die oben angegebenen Bedeutungen haben,
$R^{1-2}$ für eine der folgenden Gruppierungen

(m-b) $-B-Z-D^1$

(n-b) $-Y^1-E^1$

steht, in denen
B und Z die oben angegebenen Bedeutungen haben,

$Y^1$ für Sauerstoff oder Schwefel steht und

$D^1$ und $E^1$ für die Gruppierung

$$-(CH2)_p-(CR^9R^{10})_q(CH_2)_r-G$$

stehen, in der
$R^9$, $R^{10}$, G, p, q und r die oben angegebenen Bedeutungen haben,
lassen sich herstellen, indem man cyclische Imine der Formel (I-d)

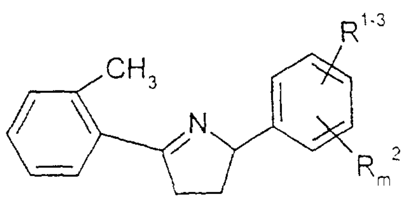

(I-d),

in welcher

$R^2$ und m die oben angegebenen Bedeutungen haben und
$R^{1-3}$ für eine der folgenden Gruppierungen

(m-c) -B -Z-H

(n-c) $-Y^1-H$

steht, in denen
B, $Y^1$ und Z die oben angegebenen Bedeutungen haben,
mit Verbindungen der Formel (VII)

$$Ab-(CH_2)_p-(CR^9R^{10})_q-(CH_2)_r-G \quad (VII),$$

in welcher

$R^9$, $R^{10}$, G, p, q und r die oben angegebenen Bedeutungen haben und
Ab für eine Abgangsgruppe steht,

kondensiert.

E) Cyclische Imine der Formel (I-e)

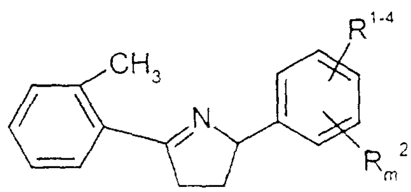
(I-e),

in welcher

$R^2$ und m die oben angegebenen Bedeutungen haben und

$R^{1-4}$ für eine den Rest G enthaltenden Gruppierung aus der Beschreibung der erfindungsgemäßen Verbindungen der Formel (I) steht, wobei

G für eine der oben genannten Gruppierungen (e) bis (k) steht,

lassen sich herstellen durch allgemein übliche und bekannte Derivatisierungen der entsprechenden Keto-Derivate. Carbonsäure-Derivate oder Nitrile, d.h. Verbindungen der Formel (I), in denen G für Cyano oder eine der Gruppierungen (a) bis (d) steht.

Weiterhin wurde gefunden, daß die neuen Verbindungen der Formel (I) bei guter Pflanzenverträglichkeit eine sehr gute Wirksamkeit als Schädlingsbekämpfungsmittel, insbesondere gegen Arthropoden in der Landwirtschaft aber auch gegen Parasiten in der Nutz- und Hobbytierhaltung aufweisen.
Die erfindungsgemäßen Verbindungen sind durch die Formel (I) allgemein definiert. Bevorzugte Substituenten bzw. Bereiche der in den oben und nachstehend erwähnten Formeln aufgeführten Reste werden im folgenden erläutert.

m steht bevorzugt für 0, 1,2 oder 3.

Ar steht besonders bevorzugt für den Rest

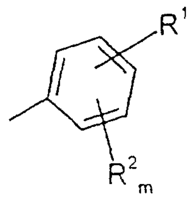

m steht besonders bevorzugt für 0, 1 oder 2.

$R^1$ steht besonders bevorzugt für einen Substituenten in meta- oder para-Position aus der Reihe Wasserstoff, Fluor, Chlor, Brom, Cyano, Tri-($C_1$-$C_4$-alkyl)-silyl, -CO-$NR^4R^5$, Tetrahydropyranyl oder eine der folgenden Gruppierungen

(l) -X-A

(m) -B-Z-D

(n) -Y-E .

$R^2$ steht besonders bevorzugt für Wasserstoff, Fluor, Chlor, Brom, Iod, Cyano, Nitro, $C_1$-$C_{16}$-Alkyl, $C_1$-$C_{16}$-Alkoxy, jeweils durch Fluor oder Chlor substituiertes $C_1$-$C_6$-Alkyl oder $C_1$-$C_6$-Alkoxy, für $C_1$-$C_8$-Alkoxy-$C_1$-$C_8$-alkoxy, oder -$S(O)_oR^3$,

mit der Maßgabe, daß $R^1$ nicht für Wasserstoff steht, wenn m = 1 und $R^2$ = Iod ist.

o steht besonders bevorzugt für 0, 1 oder 2.

$R^3$ steht besonders bevorzugt für $C_1$-$C_4$-Alkyl oder jeweils durch Fluor oder Chlor substituiertes Methyl oder Ethyl.

$R^4$ und $R^5$ stehen unabhängig voneinander besonders bevorzugt für Wasserstoff, $C_1$-$C_6$-Alkyl, durch Fluor oder Chlor substituiertes $C_1$-$C_6$-Alkyl oder für jeweils gegebenenfalls einfach oder zweifach durch Reste aus der Liste $W^1$ substituiertes Phenyl oder Benzyl.

X steht besonders bevorzugt für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder Di-$C_1$-$C_4$-alkylsilylen.

A steht besonders bevorzugt für jeweils gegebenenfalls einfach bis dreifach durch Reste aus der Liste $W^1$ substituiertes Phenyl, Naphthyl oder Tetrahydronaphthyl oder für jeweils gegebenenfalls einfach bis dreifach durch Reste aus der Liste $W^2$ substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält. (insbesondere Furyl, Benzofuryl, Thienyl, Benzothienyl, Oxazolyl, Benzoxazolyl. Thiazolyl, Benzthiazolyl, Pyrrolyl, Pyridyl, Pyrimidyl, 1,3,5-TriazinyL Chinolinyl, Isochinolinyl, Indolyl, Puriny], Benzodioxolyl, Indanyl, Benzodioxanyl oder Chromanyl).

B steht besonders bevorzugt für gegebenenfalls einfach oder zweifach durch Reste aus der Liste $W^1$ substituiertes p-Phenylen.

Z steht besonders bevorzugt für Sauerstoff oder Schwefel.

D steht besonders bevorzugt für Wasserstoff, $C_1$-$C_{16}$-Alkyl, $C_2$-$C_{16}$-Alkenyl, $C_2$-$C_6$-Alkinyl, jeweils

durch Fluor oder Chlor substituiertes $C_1$-$C_4$-Alkyl oder $C_2$-$C_4$-Alkenyl, für jeweils gegebenenfalls durch Fluor, Chlor. Brom, $C_1$-$C_4$-Alkyl, $C_2$-$C_4$-Alkenyl, durch Fluor oder Chlor substituiertes $C_2$-$C_4$-Alkenyl, durch Phenyl, Styryl, jeweils durch Fluor, Chlor oder Brom substituiertes Phenyl oder Styryl substituiertes $C_3$-$C_6$-Cycloalkyl oder $C_3$-$C_6$-Cycloalkyl-$C_1$-$C_4$-alkyl, für jeweils gegebenenfalls durch Fluor, Chlor, Brom Oder $C_1$-$C_4$-Alkyl substituiertes $C_5$-$C_6$-Cycloalkenyl oder $C_5$-$C_6$-Cycloalkenyl-$C_1$-$C_4$-alkyl, für jeweils gegebenenfalls durch Nitro, Fluor, Chlor, Brom, $C_1$-$C_6$-Alkyl, $C_1$-$C_6$-Alkoxy, jeweils durch Fluor oder Chlor substituiertes $C_1$-$C_4$-Alkyl oder $C_1$-$C_4$-Alkoxy substituiertes Phenyl-$C_1$-$C_4$-alkyl, Naphthyl$C_1$-$C_4$-alkyl, Tetrahydronaphthyl-$C_1$-$C_6$-alkyl oder 5- oder 6-ringgliedriges Hetaryl-$C_1$-$C_4$-alkyl mit 1 oder 2 Heteroatomen aus der Reihe Stickstoff, Sauerstoff und Schwefel, insbesondere Furylmethyl, Thienylmethyl, Pyrrolylmethyl, Oxazolylmethyl, Isoxazolylmethyl, Thioazolylmethyl oder Pyridylmethyl) für -CO-$R^6$, -CO-$NR^7R^8$ oder die Gruppierung

$$—(CN_2)_p—(CR^9R^{10})_q—(CH_2)_r—G\ .$$

Z und D — stehen auch <u>besonders bevorzugt</u> gemeinsam für gegebenenfalls durch Nitro, Fluor, Chlor, Brom, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy substituiertes Phenoxy-$C_1$-$C_3$-alkyl.

Y — steht <u>besonders bevorzugt</u> für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder für gegebenenfalls einfach oder zweifach durch Reste aus der Liste $W^1$ substituiertes p-Phenylen.

E — steht <u>besonders bevorzugt</u> für Wasserstoff, $C_1$-$C_{16}$-Alkyl, $C_2$-$C_{16}$-Alkenyl. $C_2$-$C_6$-Alkinyl, jeweils durch Fluor oder Chlor substituiertes $C_1$-$C_4$-Alkyl oder $C_2$-$C_4$-Alkenyl, für gegebenenfalls durch Fluor, Chlor, Brom, $C_1$-$C_4$-Alkyl, $C_2$-$C_4$-Alkenyl, durch Fluor oder Chlor substituiertes $C_2$-$C_4$-Alkenyl, durch Phenyl, Styryl oder jeweils durch Fluor, Chlor oder Brom substituiertes Phenyl oder Styryl substituiertes $C_3$-$C_6$-Cycloalkyl, für gegebenenfalls durch Fluor, Chlor, Brom oder $C_1$-$C_4$-Alkyl substituiertes $C_5$-$C_6$-Cycloalkenyl, für gegebenenfalls einfach bis dreifach durch Reste aus der Liste $W^1$ substituiertes Phenyl oder für jeweils gegebenenfalls einfach oder zweifach durch Reste aus der Liste $W^2$ substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung

$$-(CH_2)_p-CR^9R^{10})_q-(CH_2)_r-G$$

$R^6$ — steht <u>besonders bevorzugt</u> für $C_1$-$C_6$-Alkyl, $C_1$-$C_6$-Alkoxy, $C_2$-$C_6$-Alkenyl, $C_2$-$C_6$-Alkenyloxy, jeweils gegebenenfalls durch Fluor, Chlor, $C_1$-$C_3$-Alkyl oder jeweils durch Fluor oder Chlor substituiertes $C_1$-$C_2$-Alkyl oder $C_2$-$C_3$-Alkenyl substituiertes $C_3$-$C_6$-Cycloalkyl, $C_3$-$C_6$-Cycloalkyloxy oder $C_3$-$C_6$-Cycloalkyl-$C_1$-$C_2$-alkyloxy oder für gegebenenfalls einfach oder zweifach durch Fluor, Chlor, Brom, Iod, $C_1$-$C_4$-Alkyl, $C_1$-$C_4$-Alkoxy oder jeweils durch Fluor oder Chlor substituiertes $C_1$-$C_3$-Alkyl oder $C_1$-$C_4$-Alkoxy substituiertes Phenyl.

$R^7$ — steht <u>besonders bevorzugt</u> für Wasserstoff oder $C_1$-$C_4$-Alkyl.

$R^8$ — steht <u>besonders bevorzugt</u> für $C_1$-$C_4$-Alkyl oder für jeweils gegebenenfalls einfach oder zweifach durch Fluor, Chlor, Brom, $C_1$-$C_4$-Alkyl oder jeweils durch Fluor oder Chlor substituiertes $C_1$-$C_4$-Alkyl oder $C_1$-$C_4$-Alkoxy substituiertes Phenyl oder Benzyl.

p, q und r — stehen <u>besonders bevorzugt</u> unabhängig voneinander für 0, 1, 2 oder 3, wobei deren Summe kleiner 6 und größer 1 ist.

$R^9$ und $R^{10}$ — stehen unabhängig voneinander <u>besonders bevorzugt</u> für Wasserstoff oder $C_1$-$C_4$-Alkyl.

G — steht <u>besonders bevorzugt</u> für Cyano, für einen gegebenenfalls einfach bis dreifach durch Fluor,

Chlor, Brom, $C_1$-$C_4$-Alkyl oder durch Fluor oder Chlor substituiertes $C_1$-$C_4$-Alkyl und gegebenenfalls an der Verknüpfungsstelle durch den Rest $R^{11}$ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

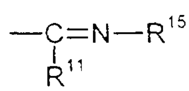

(f)

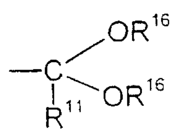

(g)

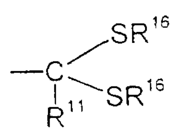

(h)

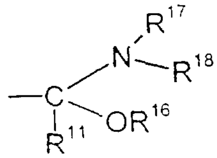

(i)

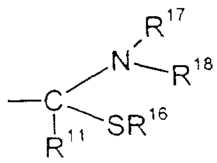

(j)

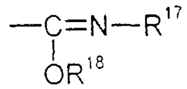

(k)

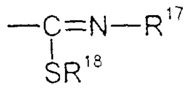

$R^{11}$ steht <u>besonders bevorzugt</u> für Wasserstoff, $C_1$-$C_6$-Alkyl, $C_2$-$C_6$-Alkenyl, jeweils durch Fluor oder Chlor substituiertes $C_1$-$C_4$-Alkyl oder $C_2$-$C_6$-Alkenyl, gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder für gegebenenfalls einfach bis dreifach durch $C_1$-$C_4$-Alkylcarbonylamino, $C_1$-$C_4$-Alkylcarbonyl-$C_1$-$C_4$-alkylamino und/oder Reste aus der Liste $W^3$ substituiertes Phenyl.

$R^{12}$ steht <u>besonders bevorzugt</u> für Wasserstoff, $C_1$-$C_4$-Alkyl, $C_3$-$C_6$-Alkenyl, jeweils durch Fluor oder Chlor substituiertes $C_1$-$C_4$-Alkyl oder $C_3$-$C_6$-Alkenyl, jeweils gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder $C_3$-$C_6$-Cycloalkyl-$C_1$-$C_4$-alkyl oder für jeweils gegebenenfalls einfach bis dreifach durch Reste aus der Liste $W^3$ substituiertes Phenyl-$C_1$-$C_4$-alkyl oder Naphthyl-$C_1$-$C_4$-alkyl.

$R^{13}$ und $R^{14}$ stehen unabhängig voneinander <u>besonders bevorzugt</u> für Wasserstoff, $C_1$-$C_4$-Alkyl, $C_3$-$C_6$-Alkenyl, jeweils durch Fluor oder Chlor substituiertes $C_1$-$C_4$-Alkyl oder $C_3$-$C_6$-Alkenyl, für $C_1$-$C_4$-Alkoxy, jeweils gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder $C_3$-$C_6$-Cycloalkyl-$C_1$-$C_4$-alkyl, für jeweils gegebenenfalls einfach bis dreifach durch Reste aus der Liste $W^3$ substituiertes Phenyl oder Phenyl-$C_1$-$C_4$-alkyl, für $-OR^{12}$ oder $-NR^{11}R^{12}$ oder gemeinsam für $-(CH_2)_5-$, $-(CH_2)_6-$ oder $-(CH_2)_2-O-(CH_2)_2$.

$R^{15}$ steht <u>besonders bevorzugt</u> für$-OR^{12}$, $-NR^{11}R^{12}$ oder $-N(R^{11})-COOR^{12}$.

$R^{16}$, $R^{17}$ und $R^{18}$ stehen unabhängig voneinander <u>besonders bevorzugt</u> für $C_1$-$C_4$-Alkyl.

$W^1$ steht <u>besonders bevorzugt</u> für Wasserstoff, Fluor, Chlor, Brom, Iod, Cyano, Formyl, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für $C_1$-$C_4$-Alkylcarbonyl, $C_1$-$C_4$-Alkoxycarbonyl oder $-S(O)_oR^3$.

$W^2$ steht <u>besonders bevorzugt</u> für Fluor, Chlor, Brom, Cyano, Formyl, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für $C_1$-$C_4$-Alkylcarbonyl, $C_1$-$C_4$-Alkoxycarbonyl, $-S(O)_oR^3$ oder $-C(R^{11})=N-R^{15}$.

$W^3$ steht <u>besonders bevorzugt</u> für Fluor, Chlor, Brom, Cyano, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für Di-$C_1$-$C_4$-alkylamino, -S

$(O)_oR^3$, $-COOR^{19}$ oder $-CONR^{20}R^{21}$.

$R^{19}$ steht besonders bevorzugt für Wasserstoff, $C_1$-$C_4$-Alkyl, durch Fluor oder Chlor substituiertes $C_1$-$C_4$-Alkyl, gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder für gegebenenfalls einfach bis dreifach durch Reste aus der Liste $W^4$ substituiertes Phenyl.

$R^{20}$ und $R^{21}$ stehen unabhängig voneinander besonders bevorzugt für Wasserstoff, $C_1$-$C_4$-Alkyl, $C_3$-$C_6$-Alkenyl, jeweils durch Fluor oder Chlor substituiertes $C_1$-$C_4$-Alkyl oder $C_3$-$C_6$-Alkenyl, für $C_1$-$C_4$-Alkoxy, jeweils gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder $C_3$-$C_6$-Cycloalkyl-$C_1$-$C_4$-alkyl oder für jeweils gegebenenfalls einfach bis dreifach durch Reste aus der Liste $W^4$ substituiertes Phenyl oder Phenyl-$C_1$-$C_4$-alkyl, für $OR^{16}$ oder $-NR^{17}R^{18}$ oder gemeinsam für $-(CH_2)_5-$, $-(CH_2)_6-$ oder $-(CH_2)_2-O-(CH_2)_2$.

$W^4$ steht besonders bevorzugt für Fluor, Chlor, Brom, Cyano, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, Di-$C_1$-$C_4$-alkylamino, $C_1$-$C_4$-Alkoxycarbonyl, Di-$C_1$-$C_6$-alkylaminocarbonyl oder $-S(O)_oR^3$.

Ar steht ganz besonders bevorzugt für den Rest

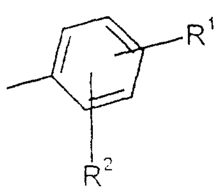

$R^1$ steht ganz besonders bevorzugt für einen Substituenten in meta- oder para-Position aus der Reihe Wasserstoff, Fluor, Chlor, Brom, Cyano, $-CO-NR^4R^5$, Tetrahydropyranyl oder eine der folgenden Gruppierungen

(l) —X—A

(m-a)

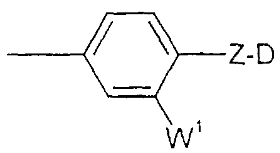

(n) —Y—E.

$R^2$ steht ganz besonders bevorzugt für Wasserstoff, Fluor, Chlor, Brom, Iod, Methyl, Ethyl, Methoxy, Ethoxy, Methylthio, Ethylthio, Trifluormethyl, Difluormethoxy, Trifluormethoxy oder Trifluormethylthio,

mit der Maßgabe, daß $R^1$ nicht für Wasserstoff steht, wenn m = 1 und $R^2$ = Iod ist.

o steht ganz besonders bevorzugt für 0 oder 2.

$R^3$ steht ganz besonders bevorzugt für Methyl, Ethyl, n-Propyl, Isopropyl, Difluormethyl oder Trifluormethyl.

$R^4$ und $R^5$ stehen unabhängig voneinander ganz besonders bevorzugt für Wasserstoff, Methyl, Ethyl, n-Propyl, Isopropyl, n-Butyl, Isobutyl, sec.-Butyl, tert.-Butyl oder für jeweils gegebenenfalls einfach durch einen Rest aus der Liste $W^1$ substituiertes Phenyl oder Benzyl.

X steht ganz besonders bcvorzugt für eine direkte Bindung, Sauerstoff, Schwefel, Carbonyl, $-CH_2-$, $-(CH_2)_2-$, -CH=CH- (E oder Z), -C≡C-, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, $C_1$-$C_4$-Alkylendioxy, insbesondere $-OCH_2O-$, $-O(CH_2)_2O-$oder $-OCH(CH_3)O-$.

A steht ganz besonders bevorzugt für gegebenenfalls einfach oder zweifach durch Reste aus der Liste $W^1$ substituiertes Phenyl oder für jeweils gegebenenfalls einfach oder zweifach durch Reste aus der Liste $W^2$ substituiertes Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl.

Z steht ganz besonders bevorzugt für Sauerstoff oder Schwefel.

D steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, $-CH=CCl_2$, Phenyl, Styryl, jeweils durch Fluor, Chlor oder Brom substituiertes Phenyl oder 4-Chlorstyryl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl, Cyclohexenyl, Cyclohexenylmethyl oder Cyclopentenylmethyl, für jeweils gegebenenfalls einfach oder zweifach durch Nitro, Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Trifluormethyl, Trifluormethoxy, Difluormethoxy oder Chlordifluormethoxy substituiertes Benzyl, Phenethyl, Naphthylmethyl, Tetrahydronaphthylmethyl, Furylmethyl, Thienylmethyl, Pyrrolylmethyl, Oxazolylmethyl, Isoxazolylmethyl, Thiazolylmethyl oder Pyridylmethyl, für $-CO-R^6$, $-CO-NR^7R^8$ oder die Gruppierung

$$-(CH_2)_p-(CR^9R^{10})_q-CH_2)_r-G,$$

Z und D stehen auch ganz besonders bevorzugt gemeinsam für gegebenenfalls einfach oder zweifach durch Nitro, Fluor, Chlor, Brom, Methyl, Ethyl, n-Propyl, i-Propyl, Methoxy, Ethoxy, n-Propoxy, i-Propoxy, Trifluormethyl, Trifluormethoxy, Difluormethoxy oder Chlordifluormethoxy substituiertes Phenoxymethyl.

Y steht ganz besonders bevorzugt für eine direkte Bindung, Sauerstoff. Schwefel, Carbonyl, $-CH_2-$, $-(CH_2)_2-$, -CH=CH- (E oder Z), -C C-, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, $C_1$-$C_4$-Alkylendioxy, insbesondere $-OCH_2O-$ oder $-O(CH_2)_2O-$ oder für gegebenenfalls einfach durch einen Rest aus der Liste $W^1$ substituiertes p-Phenylen.

E steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2.2-Dimethylethenyl, $-CH=CCl_2$, Phenyl, Styryl, jeweils durch Fluor, Chlor oder Brom substituiertes Phenyl oder durch 4-Chlorstyryl substituiertes Cyclopropyl, Cyclobutyl, Cyclopentyl oder Cyclohexyl, für jeweils gegebenenfalls durch Fluor, Chlor, Methyl, Ethyl, n-Propyl, Isopropyl, n-Butyl, Isobutyl,

sec.-Butyl oder tert.-Butyl substituiertes Cyclopentenyl oder Cyclohexenyl, für gegebenenfalls einfach oder zweifach durch Reste aus der Liste $W^1$ substituiertes Phenyl, für jeweils gegebenenfalls einfach oder zweifach durch Reste aus der Liste $W^2$ substituiertes Furyl, Thienyl, Pyrrolyl, Oxazolyl, Isoxazolyl, Thiazolyl oder Pyridyl, oder für die Gruppierung

$$-(CH_2)_p-(CR^9R^{10})_q-(CH_2)_r-G.$$

$R^6$ steht ganz besonders bevorzugt für Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Cyclopropyl, Cyclohexyl, Cyclohexyloxy, Cyclohexylmethyloxy, Phenyl, 2-Chlorphenyl, 3-Chlorphenyl, 2,6-Difluorphenyl, 2,4-Dichlorphenyl, 3,4-Dichlorphenyl, 2-Trifluormethoxyphenyl oder 4-Trifluormethoxyphenyl.

$R^7$ steht ganz besonders bevorzugt für Wasserstoff.

$R^8$ steht ganz besonders bevorzugt für Methyl, Ethyl oder gegebenenfalls einfach durch Chlor substituiertes Phenyl.

p, q und r stehen ganz besonders bevorzugt unabhängig voneinander für 0, 1, 2 oder 3, wobei deren Summe kleiner 4 und größer 1 ist.

$R^9$ und $R^{10}$ stehen unabhängig voneinander ganz besonders bevorzugt für Wasserstoff, Methyl, Ethyl, n-Propyl, Isopropyl, n-Butyl, Isobutyl, sec.-Butyl, tert.-Butyl.

G steht ganz besonders bevorzugt für Cyano, für jeweils gegebenenfalls einfach bis dreifach durch Fluor, Chlor, Brom, Methyl, Ethyl, n-Propyl, Isopropyl oder Trifluormethyl und gegebenenfalls an der Verknüpfungsstelle durch den Rest $R^{11}$ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1.3-Dithian-2-yl oder 1,3-Thioxan-2-yl oder eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

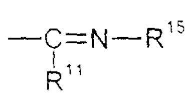

(f)

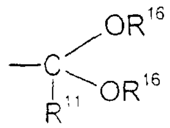

(g)

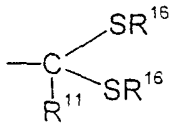

(h)

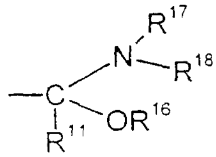

(i)

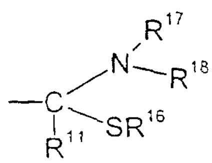

$R^{11}$ steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $C_3$-$C_6$-Alkenyl, einfach bis dreifach durch Fluor oder Chlor substituiertes $C_3$-$C_6$-Alkenyl, für jeweils gegebenenfalls einfach oder zweifach durch Fluor, Chlor, Methyl, Ethyl, n-Propyl, Isopropyl, $-CF_3$, $-CHF_2$, $-CClF_2$, $-CF_2CHFCl$, $-CF_2CH_2F$, $-CF_2CHF_2$, $-CF_2CCl_3$ oder $-CH_2CF_3$ substituiertes Cyclopropyl, Cyclopentyl oder Cyclohexyl oder für gegebenenfalls einfach oder zweifach durch Methylcarbonylamino, Ethylcarbonylamino, Methylcarbonyl-methylamino und/oder Reste aus der Liste $W^3$ substituiertes Phenyl.

$R^{12}$ steht ganz besonders bevorzugt für Wasserstoff, Methyl, Ethyl, n-Propyl, Isopropyl, n-Butyl, Isobutyl, sec.-Butyl, tert.-Butyl, $-CH_2CF_3$, Allyl, jeweils gegebenenfalls einfach oder zweifach durch Fluor, Chlor, Methyl, Ethyl, n-Propyl, Isopropyl $-CF_3$, $-CHF_2$, $-CClF_2$, $-CF_2CHFCl$, $-CF_2CH_2F$, $-CF_2CHF_2$, $-CF_2CCl_3$ oder $-CH_2CF_3$ substituiertes Cyclopropyl, Cyclopentyl, Cyclohexyl, Cyclopropylmethyl, Cyclopentylmethyl, Cyclohexylmethyl, Cyclopropylethyl, Cyclopentylethyl oder Cyclohexylethyl oder für jeweils gegebenenfalls einfach oder zweifach durch Reste aus der Liste $W^3$ substituiertes Benzyl oder Phenethyl.

$R^{13}$ und $R^{14}$ stehen unabhängig voneinander ganz besonders bevorzugt für Wasserstoff, Methyl, Ethyl, n-Propyl, Isopropyl, n-Butyl, Isobutyl, sec.-Butyl, tert.-Butyl, $-CH_2CF_3$, Methoxy, Ethoxy, Allyl, für jeweils gegebenenfalls einfach oder zweifach durch Fluor, Chlor, Methyl, Ethyl, n-Propyl, Isopropyl oder Trifluormethyl substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste

aus der Liste $W^3$ substituiertes Phenyl, Benzyl oder Phenethyl, für $-OR^{12}$ oder $-NR^{11}R^{12}$.

$R^{15}$ steht ganz besonders bevorzugt für $-OR^{12}$, $-NR^{11}R^{12}$ oder $-N(R^{11})-COOR^{12}$.

$R^{16}$, $R^{17}$ und $R^{18}$ stehen unabhängig voneinander besonders bevorzugt für Methyl, Ethyl, n-Propyl oder Isopropyl.

$W^1$ steht ganz besonders bevorzugt für Wasserstoff, Fluor, Chlor, Brom, Cyano, Formyl, Nitro, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, Trifluormethoxy, Difluormethoxy, Chlordifluormethoxy, Acetyl, Propionyl, Butyryl, Isobutyryl, Methoxycarbonyl, Ethoxycarbonyl, n-Propoxycarbonyl, Isopropoxycarbonyl, n-Butoxycarbonyl, Isobutoxycarbonyl, sec.-Butoxycarbonyl, tert.-Butoxycarbonyl oder $-S(O)_oR^3$.

$W^2$ steht ganz besonders bevorzugt für Fluor, Chlor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, $-CH=N-OCH_3$, $-CH=N-OC_2H_5$, $-CH=N-OC_3H_7$, $-C(CH_3)=N-OCH_3$, $-C(CH_3)=N-OC_2H_5$, $-C(CH_3)=N-OC_3H_7$, $-C(C_2H_5)=N-OCH_3$, $-C(C_2H_5)=N-OC_2H_5$ oder $-C(C_2H_5)=N-OC_3H_7$.

$W^3$ steht ganz besonders bevorzugt für Fluor, Chlor, Cyano, Nitro, Methyl, Ethyl, Methoxy, Ethoxy, Methylthio, Trifluormethyl, Trifluormethoxy, Trifluormethylthio, Dimethylamino, Diethylamino, $-COOR^{19}$ oder $-CONR^{20}R^{21}$.

$R^{19}$ steht ganz besonders bevorzugt für Wasserstoff, Methyl, Ethyl, n-Propyl, Isopropyl, tert.-Butyl, $-CH_2CF_3$, für jeweils gegebenenfalls einfach oder zweifach durch Fluor, Chlor, Methyl, Ethyl, n-Propyl, Isopropyl oder $-CF_3$ substituiertes Cyclopropyl, Cyclopentyl oder Cyclohexyl oder für gegebenenfalls einfach oder zweifach durch Reste aus der Liste $W^4$ substituiertes Phenyl.

$R^{20}$ und $R^{21}$ stehen unabhängig voneinander ganz besonders bevorzugt für Wasserstoff, Methyl, Ethyl, n-Propyl, Isopropyl, n-Butyl, Isobutyl, sec.-Butyl, tert.-Butyl, $-CH_2CF_3$, Methoxy, Ethoxy, Allyl, für jeweils gegebenenfalls einfach oder zweifach durch Fluor oder Chlor substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste $W^4$ substituiertes Phenyl, Benzyl oder Phenethyl, für $-OR^{16}$ oder $-NR^{17}R^{18}$.

$W^4$ steht ganz besonders bevorzugt für Fluor, Chlor, Brom, Cyano, Nitro, Methyl, Ethyl, tert.-Butyl, Methoxy, Ethoxy, Metlylthio, Trifluormethyl, Trifluormethoxy oder Trifluormethylthio.

Weiterhin bevorzugt sind Verbindungen der Formel (I-a)

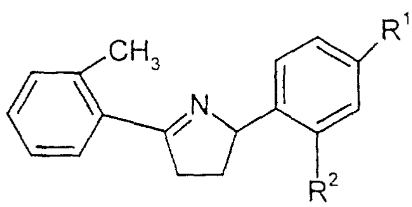

(I-a)

in welcher

$R^2$ die oben genannten allgemeinen, bevorzugten, besonders bevorzugten oder ganz besonders bevorzugten Bedeutungen hat,

$R^1$ für Wasserstoff oder einfach oder zweifach durch Reste aus der Liste $W^1$ substituiertes Phenyl oder eine der folgenden Gruppierungen

(m-b) -B-O-D

(l) -Y-E

steht,

B für gegebenenfalls einfach durch einen Rest aus der Liste $W^1$ substituiertes p-Phenylen steht,

Y für eine direkte Bindung oder für gegebenenfalls einfach oder zweifach durch Reste aus der Liste $W^1$ substituiertes p-Phenylen steht und

D und E die oben genannten ganz besonders bevorzugten Bedeutungen haben, wobei

G für Cyano oder eine der folgenden Gruppierungen

(a)

$$-CO-R^{11}$$

(b)

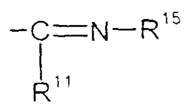

steht, in denen

$R^{11}$ und $R^{15}$ die oben genannten allgemeinen, bevorzugten, besonders bevorzugten oder ganz besonders bevorzugten Bedeutungen haben und

$W^1$ die oben genannte allgemeine, bevorzugte, besonders bevorzugte oder ganz besonders bevorzugte Bedeutung hat.

Weiterhin bevorzugt sind Verbindungen der Formel (I-f)

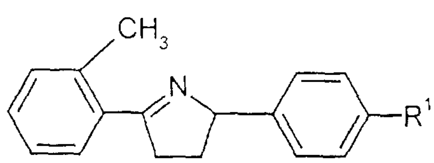

(I-f)

in welcher

$R^1$ für Wasserstoff, oder

a) einfach oder zweifach durch Reste aus der Liste $W^2$ substituiertes Phenyl oder

b) einfach oder zweifach durch Reste aus der Liste $W^2$ substituiertes Hetaryl (insbesondere Furyl, Thienyl, Pyrrolyl, Oxazolyl, Isoxazolyl, Thiazolyl oder Pyridyl, speziell Thienyl) steht.

Weiterhin bevorzugt sind Verbindungen der Formel (I-g)

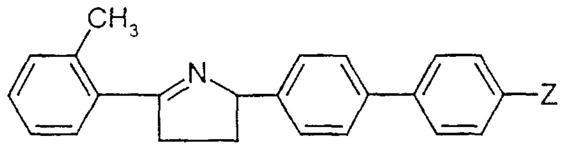

(I-g)

in welcher

Z für Wasserstoff, Fluor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, $-CH{=}N{-}OCH_3$, $-CH{=}N{-}OC_2H_5$, $-CH{=}N{-}OC_3H_7$, $-C(CH_3){=}N{-}$, $OCH_3$, $-C(CH_3){=}N{-}OC_2H_5$, $-C(CH_3){=}N{-}OCt_3H_7$, $-C(C_2H_5){=}N{-}OC_2H_5$ oder $-C(C_2H_5){=}N{-}OC_3H_7$ steht.

Weiterhin bevorzugt sind die in Tabelle 1 aufgeführten Verbindungen der Formel (I-f).

## Tabelle 1

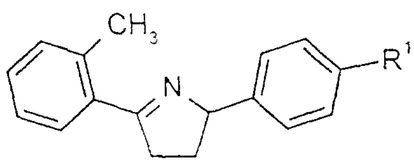

(I-f)

| Bsp.-Nr. | $R^1$ |
|---|---|
| I-1 | $(CH_2)_3CH_3$ |
| I-2 | 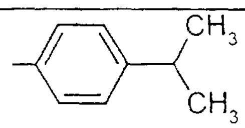 |
| I-3 | 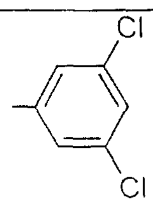 |

| Bsp.-Nr. | $R^1$ |
|---|---|
| I-4 | 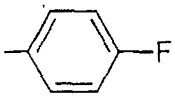 |
| I-5 | 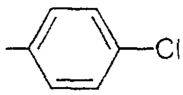 |
| I-6 | 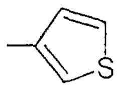 |
| I-7 | 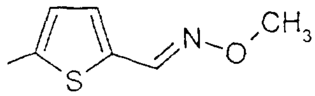 |
| I-8 | 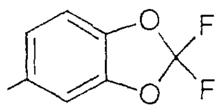 |
| I-9 | 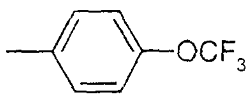 |
| I-10 | 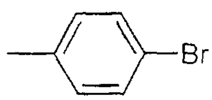 |
| I-11 | 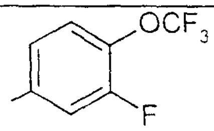 |
| I-12 | 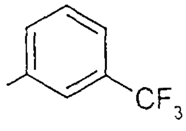 |
| I-13 | 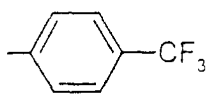 |
| I-14 | 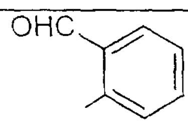 |
| I-15 | 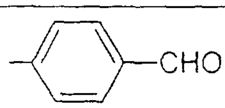 |

| Bsp.-Nr. | $R^1$ |
|---|---|
| I-16 | 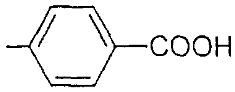 |
| I-17 | 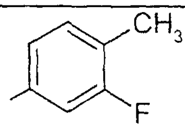 |
| I-18 | 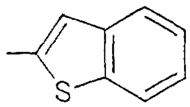 |
| I-19 | 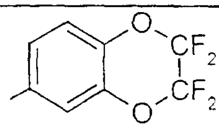 |
| I-20 | 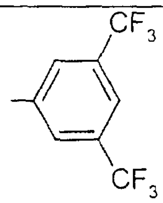 |
| I-21 | 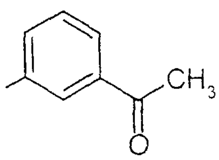 |
| I-22 | -H |
| I-23 | 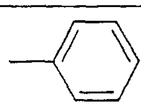 |
| I-24 | 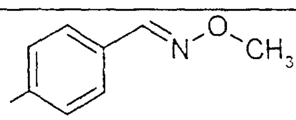 |
| I-25 | 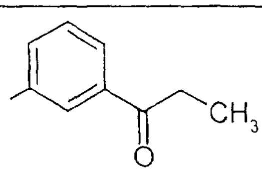 |
| I-26 | 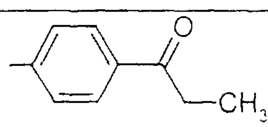 |

| Bsp.-Nr. | $R^1$ |
| --- | --- |
| I-27 | 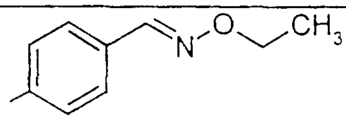 |
| I-28 | 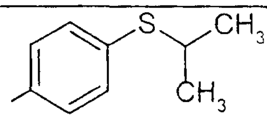 |
| I-29 | 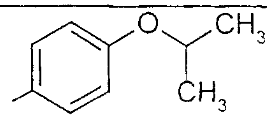 |
| I-30 | 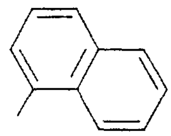 |
| I-31 | 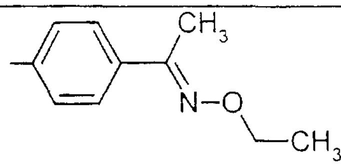 |
| I-32 | 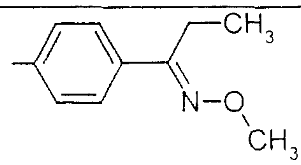 |
| I-33 | 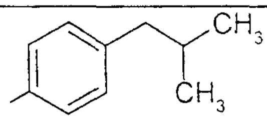 |
| I-34 | 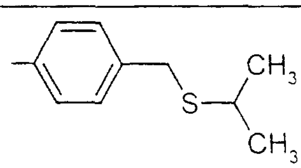 |
| I-35 | 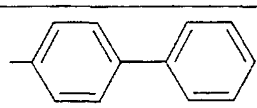 |
| I-36 | 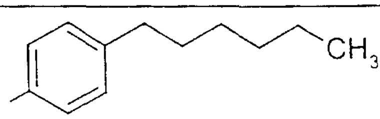 |

| Bsp.-Nr. | $R^1$ |
|---|---|
| I-37 | 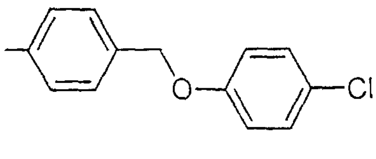 |
| I-38 | 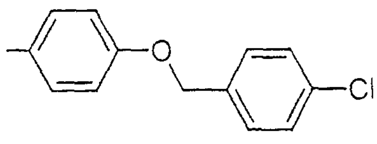 |
| I-39 | 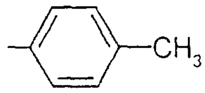 |
| I-40 | 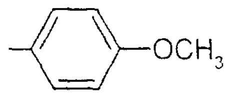 |
| I-41 | 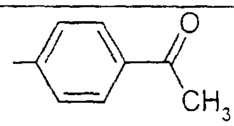 |
| I-42 | 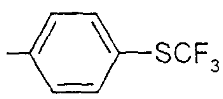 |
| I-43 | 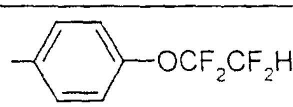 |

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise m Reste $R^2$ für m >1, können gleich oder verschieden sein.

Verwendet man beispielsweise tBOC-[1-(4-Ethyl-2-Methyl-phenyl)-3-(2-Methylphenylcarboxyl-)-1-propyl]-amin als Ausgangsstoff, so kann der Reaktionsablauf des erfindungsgemäßen Verfahrens (A) a) durch das folgende Formelschema miedergegeben werden:

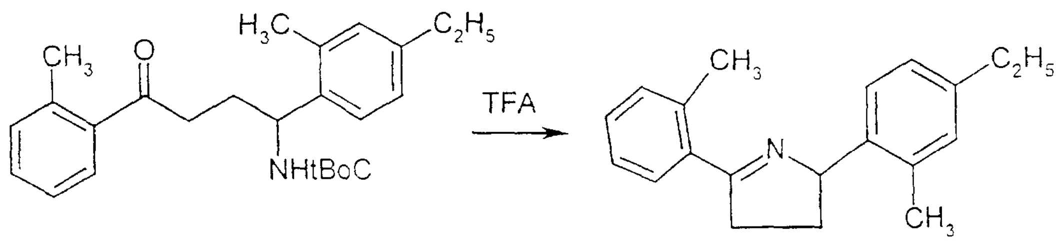


Verwendet man beispielsweise 1-(4-Ethyl-2-Methyl-phenyl)-1-Nitro-3-(2-Methylphenylcarboxyl-)-propan als Ausgangsstoffe, so kann der Reaktionsablauf des erfindungsgemäßen Verfahrens (A) b) durch das folgende Formelschema wiedergegeben werden:

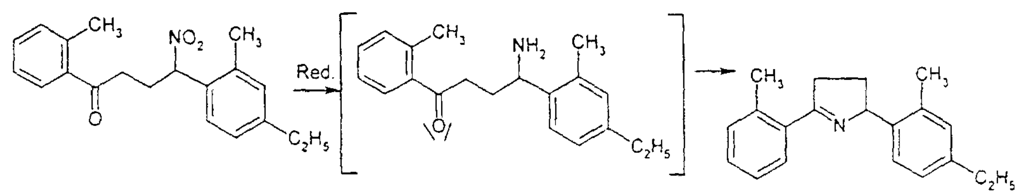


Verwendet man beispielsweise 1-(4-Ethyl-2-Methyl-phenyl)-1-(Diphenylmethylenimino)-3-(-2-Methyl-phenylcarboxyl-)-propan als Ausgangsstoff, so kann der Reaktionsablauf des erfindungsgemäßen Verfahrens (A) c) durch das folgende Formelschema wiedergegeben werden:

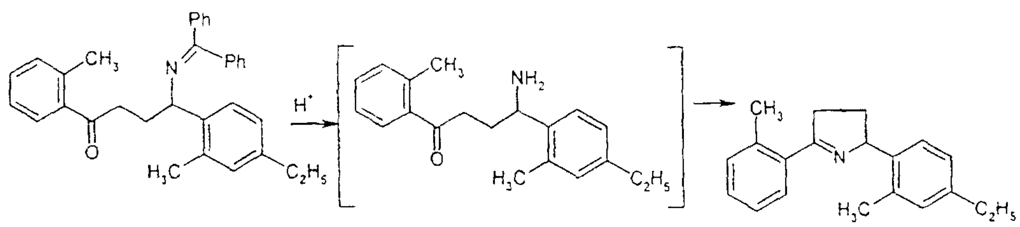


Verwendet man beispielsweise 2-(4-Methoxyphenyl)-cyclobutanon-O-methanssulfonyloxim und 2-Tolylmagnesiumbromid als Ausgangsstoffe, so kann der Reaktionsablauf des erfindungsgemäßen Verfahrens (B) durch das folgende Formelschema wiedergegeben werden:

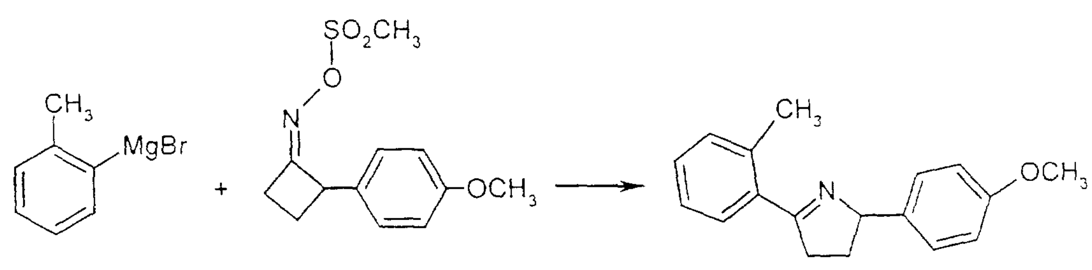

Verwendet man beispielsweise 2-(2-Methylphenyl)-5-(4-iodphenyl)-3,4-dihydro-2Hpyrrol und 4-Methoxyphenylboronsäure als Ausgangsstoffe, so kann der Reaktionsablauf des erfindungsgemäßen Verfahrens (C) durch das folgende Formelschema wiedergegeben werden:

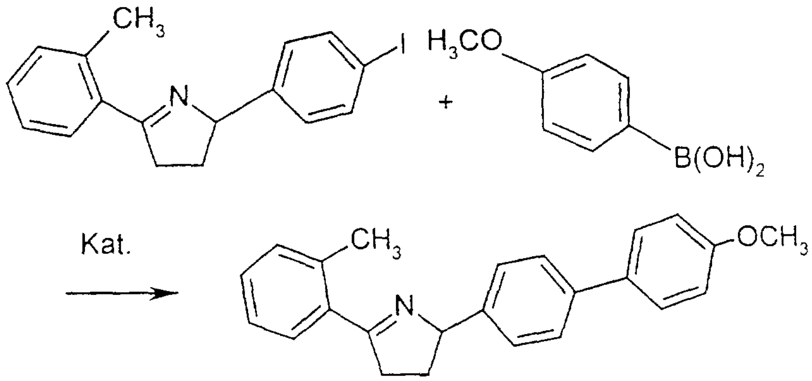


Verwendet man beispielsweise 2-(2-Methyl-phenyl)-5-(3'-chlor-4'-hydroxybiphenyl-4-yl)-3,4-dihydro-2H-pyrrol und α-Bromvaleriansäuremethylester als Ausgangsstoffe, so kann der Reaktionsablauf des erfindungsgemäßen Verfahrens (D) durch das folgende Formelschema wiedergegeben werden:

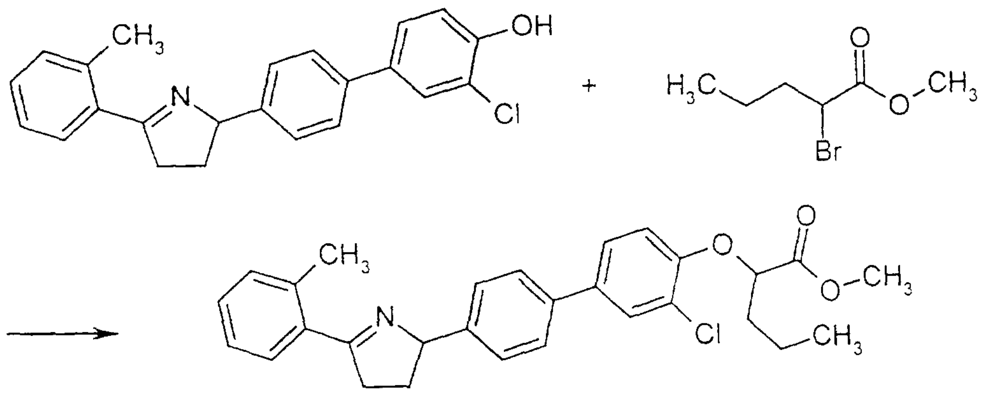

Verwendet man beispielsweise 6-(4'-Cyclopropylcarbonylmethoxy-3-trifluormethoxy-biphenyl-4-yl)-2-(2-methylphenyl)-3,4-dihydro-2H-pyrrol und *O*-Methylhydroxylamin als Ausgangsstoffe, so kann der Reaktionsablauf des erfindungsgemäßen Verfahrens (E) durch das folgende Formelschema wiedergegeben werden:

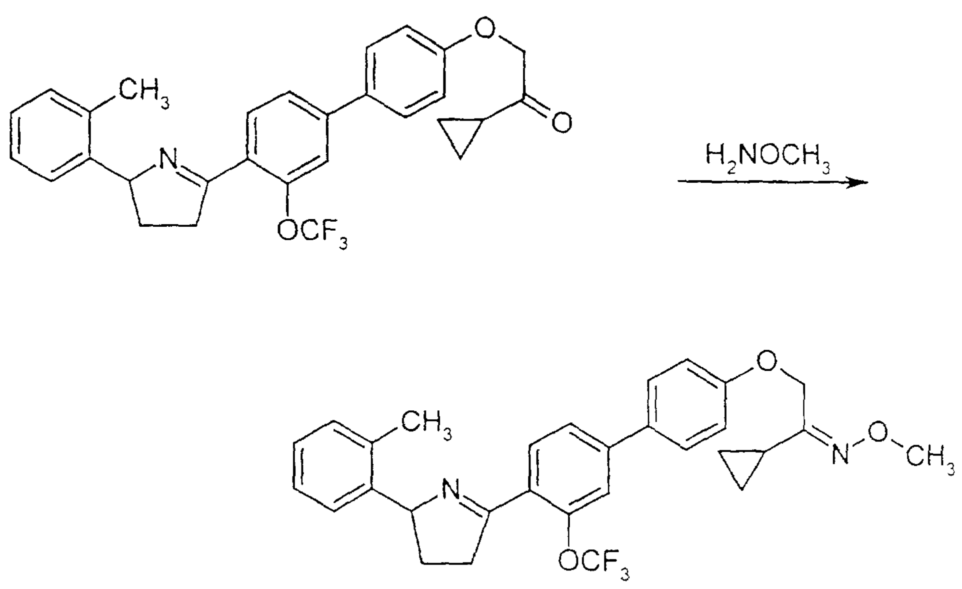


Die zur Durchführung des erfindungsgemäßen Verfahrens (A) a) benötigten Aminoketonderivate sind durch die Formel (VIII) allgemein definiert. In dieser Formel hat Ar vorzugsweise diejenigen Bedeutungen, die bereits im Zusammenhang mit der Beschreibung der cyclischen Imine der Formel (I) als bevorzugt genannt wurden. Die Aminoketonderivate der Formel (VIII) sind neu.

Aminoketon-Derivate der Formel (VIII) lassen sich z.B. herstellen, indem man BOCgeschützte Lactame der Formel (IX) mit metallierten Aromaten der Formel (X) bei Temperaturen zwischen 0°C und 80°C gemäß dem folgenden Reaktionsschema umsetzt:

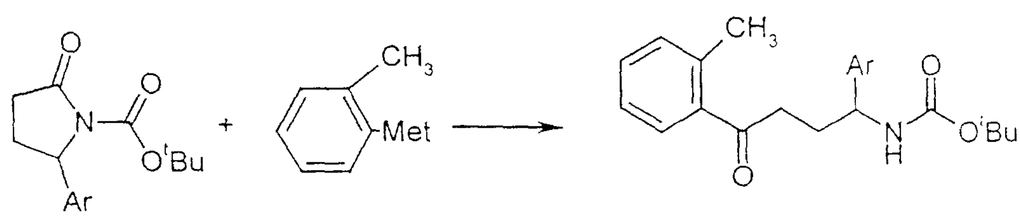

(IX) (X) (VIII)

In der Formel (X) steht Met Für einen einwertigen Metallrest wie Li. MgI. MgBr oder MgCl.

Metallierte Aromaten der Formel (X) sind zum Teil bekannt oder können nach bekannten Methoden wie z.B. Lithiierung oder Grignard-Reaktion aus den entsprechenden Aromaten oder Halogenaromaten hergestellt werden.

Geschützte Lactame der Formel (IX) erhält man beispielsweise, indem man Lactame der Formel (XI) nach üblichen Methoden wie z.B. Metallierung mit Butyllithium und Umsetzung mit Di-tert.-butyldicarbonat BOC-schützt (vgl. z.B. T. W. Greene, P. G. M. Wuts. Protective Groups in Organic Synthesis, 2. Ed., John Wiley & Sons, New York 1991).

Lactame der Formel (XI) lassen sich z.B. von ω-Alkoxylactamen der Formel (XII) ausgehend nach zwei Methoden herstellen. Man kann diese mit Aromaten der Formel (XIII) in Gegenwart eines sauren Katalysators, wie beispielsweise Schwefelsäure, Essigsäure oder Aluminiumchlorid und gegebenenfalls in Gegenwart eines Verdünnungsmittels, wie beispielsweise Dichlormethan oder Acetonitril gemäß dem folgenden Reaktionsschema umsetzen:

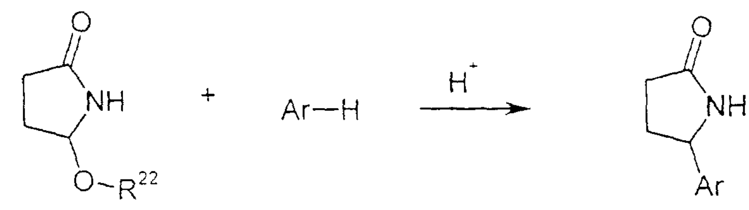

(XII) (XIII) (XI)

Alternativ kann man mit Aryl-Grignard-Verbindungen der Formel (XIV) in Gegenwart eines Verdünnungsmittels, wie beispielsweise Tetrahydrofuran gemäß dem folgenden Reaktionsschema umsetzen [vgl. Org. Prep. Proced. Int. **25**, 255 (1993)]:

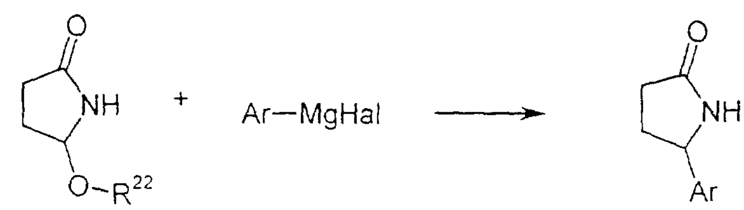

(XII) (XIV) (XI)

In Formel (XII) steht $R^{22}$ für Methyl oder Ethyl. In Formel (XIV) steht Hal für Chlor, Brom oder Iod.

Die ω-Alkoxylactame der Formel (XII) sind bekannt, z.T. kommerziell erhältlich und lassen sich z.B. aus den entsprechenden unsubstituierten Imiden durch kathodische oder Natriumboranat-Reduktion oder aus den unsubstituierten Lactamen durch anodische Oxidation, jeweils in Gegenwart von Methanol oder Ethanol herstellen (vgl. z.B. J. Org. Chem. **56,** 1822 (1991); Synthesis **1980,** 315).

Die Aromaten der Formel (XIII) sind Benzolderivate, die allgemein bekannt sind oder nach einer weiten Palette allgemein bekannter Methoden der organischen Chemie hergestellt werden können.

Die Aryl-Grignard-Verbindungen der Formel (XIV) können nach üblicher Weise aus den entsprechenden Arylhalogeniden und Magnesium hergestellt werden. Arylhalogenide sind allgemein bekannte Verbindungen der organischen Chemie.

Lactame der Formel (XI) lassen sich z.B. auch herstellen, indem man substituierte Benzoylcarbonsäuren der Formel (XV) mit einem Reagenz, bereitet aus Ammoniumcarbonat und Ameisensäure bei Siedetemperatur gemäß folgendem Reaktionsschema cyclisiert [vgl. Recl. Trav. Chim. Bays-Bas **81**, 788 (1962)]:

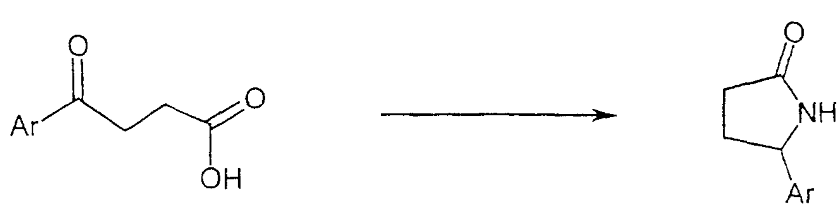

(XV) (XI)

Die hierfür benötigten ω-Benzoylcarbonsäuren der Formel (XV) lassen sich z.B. herstellen, indem man die Dicarbonsäureanhydride der Formel (XVI) mit Aromaten der Formel (XIII) in Gegenwart einer Lewis-Säure wie beispielsweise Aluminiumchlorid und gegebenenfalls in Gegenwart eines Verdünnungsmittels wie beispielsweise Benzol gemäß dem folgenden Reaktionsschema umsetzt [vgl. Recl. Trav. Chim. Bays-Bas 81, 788 (1962)]:

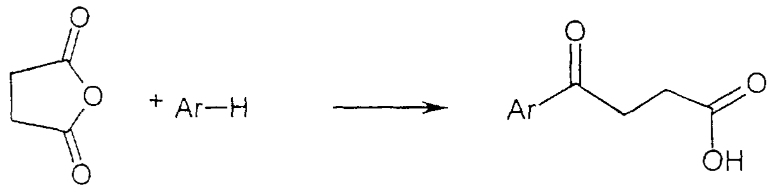

(XVI) (XIII) (XV)

Das hierfür benötigte Anhydrid (Bernsteinsäureanhydrid) ist kommerziell erhältlich.

Ausgehend von den Lactamen der Formel (XI) können alle Verfahrensschritte bis zur Darstellung der cyclischen Imine der Formel (1), einschließlich der Cyclokondensation analog Verfahren A a) auch als "Eintopfreaktion" durchgeführt werden (s. Beispiel 1-9 "Eintopfvariante").

Für den Fall, daß Ar im erfindungsgemäßen Wirkstoff der Formel (I) wie in der weiter oben angegebenen Formel (I-b) für ein gegebenenfalls substituiertes Biphenylyl steht, kann man die entsprechenden Biphenyllactame der Formel (XI-a) in einer vorteilhaften Variante des hier beschriebene Verfahrens herstellen, indem man analog zum oben und weiter unten beschriebenen Verfahren (C) bestimmte Phenyllactame der Formel (XVII) mit Boronsäuren der Formel (VI) gemäß dem folgenden Reaktionsschema umsetzt:

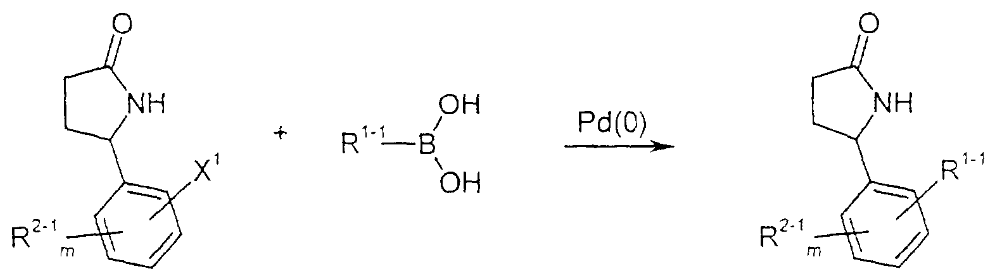


(XVII) (VI) (XI-a)

Die Phenyllactame der Formel (XVII), in denen $X^1$ für Brom oder Iod steht, sind eine Teilmenge der Verbindungen der Formel (XI), deren Herstellung oben angegeben ist. Die Phenyllactame der Formel (XVII), in denen $X^1$ für Trifluormethansulfonyl steht, lassen sich analog wie bei Verfahren (C) beschrieben aus den entsprechenden Verbindungen der Formel (XI), in denen Ar durch $R^1$ = Hydroxy substituiert ist, herstellen.

Die zur Durchführung des Verfahrens (A) b) benötigten Nitroketone sind durch die Formel (XVIII) allgemein definiert. In dieser Formel hat Ar vorzugsweise diejenigen Bedeutungen, die bereits im Zusammenhang mit der Beschreibung der cyclischen Imine der Formel (I) als bevorzugt genannt werden. Die Nitroketone der Formel (XVIII) sind neu.

Nitroketone der Formel (XVIII) lassen sich z.B. herstellen, indem man das ω-Chloralkylphenylketon der Formel (XXI) in Gegenwart eines Verdünnungsmittels wie z.B. Methanol, Ethanol, einem anderen niederen aliphatischen Alkohol oder auch Tetrahydrofuran und in Gegenwart eines Säurebindemittels wie z.B. Natriumhydrid oder eines Alkalialkoholates, vorzugsweise des entsprechenden als Verdünnungsmittel eingesetzten Alkohols, gemäß dem folgenden Reaktionsschema kondensiert:

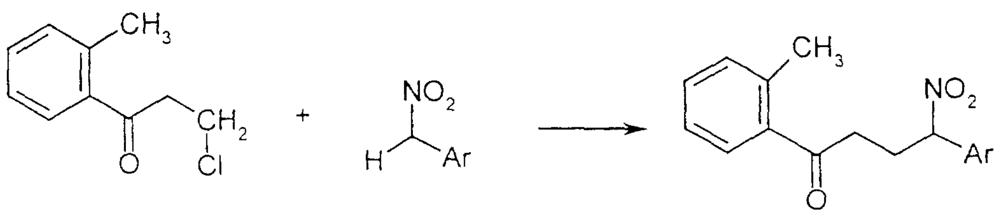

(XXI) (XIX) (XVIII)

Das ω-Chloralkylphenylketon der Formel (XXI), läßt sich auf bekannte Weise herstellen, wie z.B. durch Friedel-Crafts-Acylierung von Toluol mit 3-Chlorpropionsäurechlorid.

Die Nitromethylbenzole der Formel (XIX) sind bekannt oder lassen sich auf bekannte Weise herstellen, wie z.B. durch Nitrieren entsprechender Toluole in der Seitenkette oder Umsetzung entsprechender Benzylhalogenide mit Silbernitrit [vgl. hierzu z.B. J. Am. Chem. Soc. **77**, 6269 (1955); J. Am. Chem. Soc **86**, 2681 (1964); Houben-Weyl, Methoden der Organischen Chemie, Georg Thieme Verlag Stuttgart, Band **10/1**, 46-57 (Halogensubstitution), Band **E16**, 145-154 (Beide Methoden)]. Die hierfür benötigten Toluole oder Benzylhalogenide sind allgemein bekannte Verbindungen der organischen Chemie.

Die Nitroketone der Formel (XVIII), lassen sich z.B. durch Michael-Additionen von Nitromethylbenzolen der Formel (XIX) an Phenylvinylketon der Formel (XX) in Gegenwart eines Verdünnungsmittels wie z.B. Methanol, Ethanol oder einem anderen niederen aliphatischen Alkohol und in Gegenwart eines Säurebindemittels wie z.B. vorzugsweise eines Alkalialkoholates des entsprechenden als Verdünnungsmittel eingesetzten Alkohols gemäß dem folgenden Reaktionsschema herstellen (vgl. z.B. J. Prakt. Chem., Serie 4, **1**, 57 (1955); Houben-Weyl, Methoden der Organischen Chemie, Georg Thieme Verlag Stuttgart, Band **10/1,** 199-206):

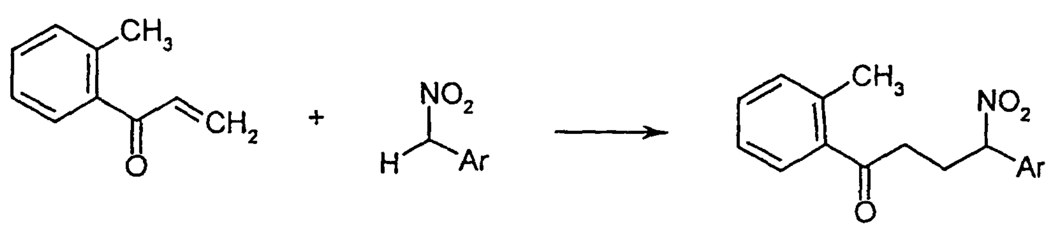

(XX) (XIX) (XVIII)

Das Phenylvinylketon der Formel (XX) läßt sich z.B. herstellen, indem man Chlorwasserstoff aus β-Chlorpropiophenon der Formel (XXI), welches sich z.B. durch Friedel-Crafts-Acylierung von Toluol (XXII) mit 3-Chlorpropionsäurechlorid erhalten läßt, in Gegenwart eines Säurebindemittels wie beispielsweise Kaliumacetat in Gegenwart eines Verdünnungsmittels wie beispielsweise Methanol gemäß folgendem Reaktionsschema abspaltet [vgl. z.B. J. Prakt. Chem., Serie 4, **1**, 57 (1954)]:

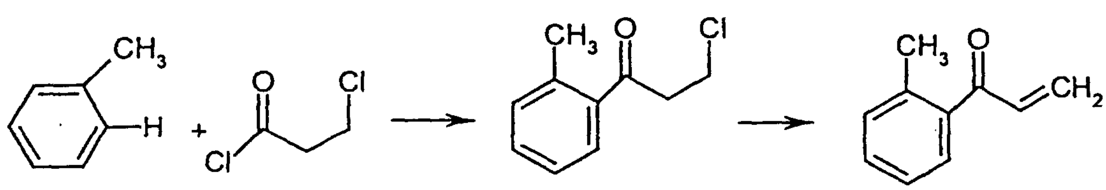

(XXII) (XXI) (XX)

Toluol der Formel (XXII) ist kommerziell erhältlich.

Das Phenylvinylketon der Formel (XX) läßt sich auch herstellen, indem man *O*-Methyl-methyl-2-methyl-benzohydroxamat der Formel (XXIII) mit Vinylmagnesiumbromid gemäß folgendem Reaktionsschema umsetzt:

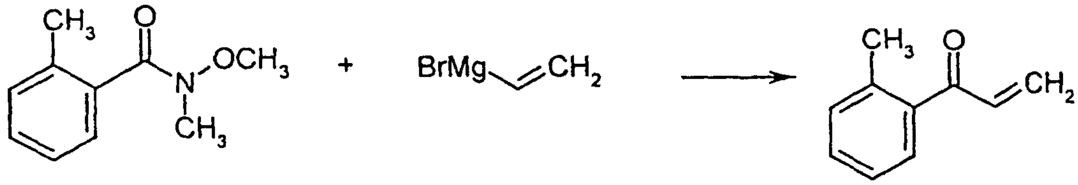

(XXIII) (XX)

Das O-Methyl-methyl-2-Methyl-benzohydroxamat der Formel (XXIII) kann nach bekannten Methoden z.B. aus den entsprechenden Benzoesäure-Derivaten hergestellt werden [vgl. z.B. Tetrahedron Lett. **22**, 3815 (1981)].

Da das Phenylvinylketon der Formel (XX) empfindlich ist, setzt man es in einer bevorzugten Variante zur Herstellung der Nitroketone der Formel (XVIII) direkt mit Nitromethylbenzolen der Formel (XIX) weiter um.

Nitroketone der Formel (XVIII) lassen sich auch herstellen, indem man gemäß nachfolgendem Reaktionsschema Enamine von Methylphenylketonen der Formel (XXVI) an α-Nitrostyrole der Formel (XXVII) addiert und das Reaktionsprodukt sauer hydrolisiert:

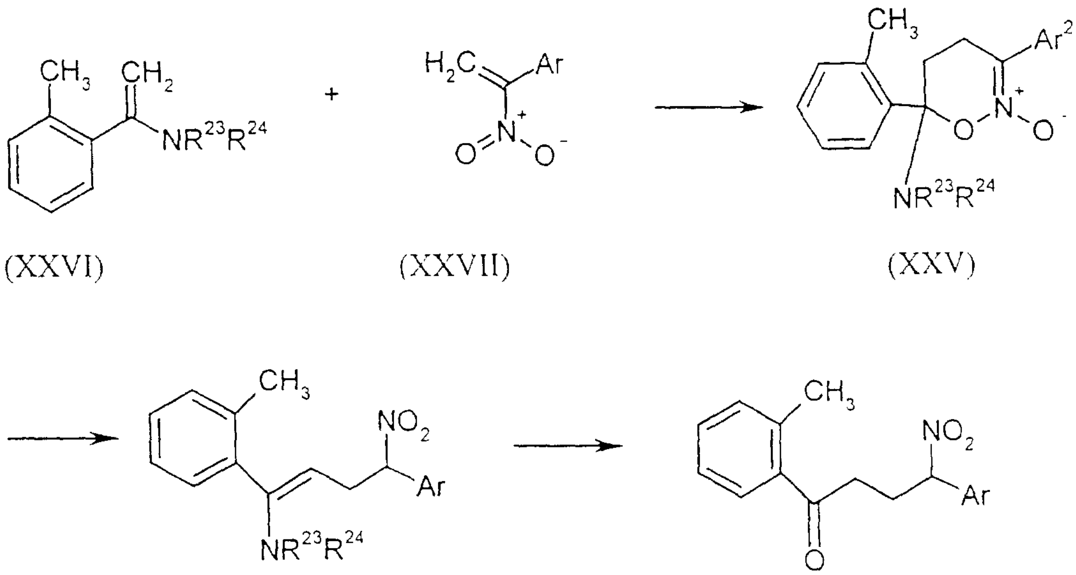

(XXVI) (XXVII) (XXV)

(XXIV) (XVIII)

In den Formeln (XXIV), und (XXVI) stehen $R^{23}$ und $R^{24}$ gemeinsam mit dem Stickstoffatom, an das sie gebunden sind für einen cyclischen Aminorest, wie z.B. 1-Pyrrolidino, 1-Piperidino oder 4-Morpholino.

Die Addition verläuft zumeist in einer [4+2]-Cycloaddition zu isolierbaren 1,2-Oxazin-N-oxid-Derivaten der Formel (XXV) und wird gegebenenfalls in Gegenwart eines unpolaren Verdünnungsmittel wie z.B. Diethylether bei beispielsweise -80° bis +20°C durchgeführt. Zur Hydrolyse verwendet man z.B wäßrige Mineralsäuren wie Salzsäure gegebenenfalls in Gegenwart von Methanol oder Ethanol [vgl. z.B. Helv. Chim. Acta **68**, 162 (1985); Tetrahedron **45**, 2099 (1989)]. In vielen Fällen ist es vorteilhaft, zunächst die Ringöffnung zu Verbindungen der Formel (XXIV) durch einfaches Lösen des 1,2-Oxazin-N-oxid-Derivates in Methanol oder Ethanol durchzuführen, da sonst unerwünschte Nef-Reaktion zu der entsprechenden Diketo-Verbindung als Konkurrenzreaktion auftritt [vgl. z.B. Tetrahedron **45**, 2099 (1989)].

Die Enamine der Formel (XXVI) sind teilweise bekannt oder lassen sich z.B aus den entsprechend substituierten Acetophenonen und den cyclischen Aminen nach Standardverfahren herstellen (z.B. Org. Syntheses Vol. 58, 56, John Wiley & Sons, New York). Die hierfür benötigten Acetophenone sind teilweise käuflich, bekannt oder lassen sich nach bekannten Methoden der Aromatenchemie herstellen.

Die Nitrostyrole der Formel (XXVII) sind teilweise bekannt oder lassen sich z.B. durch Formylierung der Nitromethylbenzole der oben angegebenen Formel (XIX) herstellen (vgl. z.B. Houben-Weyl, Methoden der Organischen Chemie, Georg Thieme Verlag, Stuttgart, Band **E16**, 215).

Die zur Durchführung des Verfahrens (A) c) benötigten Imine sind durch die Formel (XXVIII) allgemein definiert. In dieser Formel hat Ar vorzugsweise diejenigen Bedeutungen, die bereits im Zusammenhang mit der Beschreibung der cyclischen Imine der Formel (I) als bevorzugt genannt werden.

Die Imine der Formel (XXVIII), lassen sich z.B. herstellen, indem man Michael-Additionen von *N*-Diphenylmethylenbenzylaminen der Formel (XXIX) an das Phenylvinylketon der Formel (XX) gemäß dem folgendem Reaktionsschema durchführt:

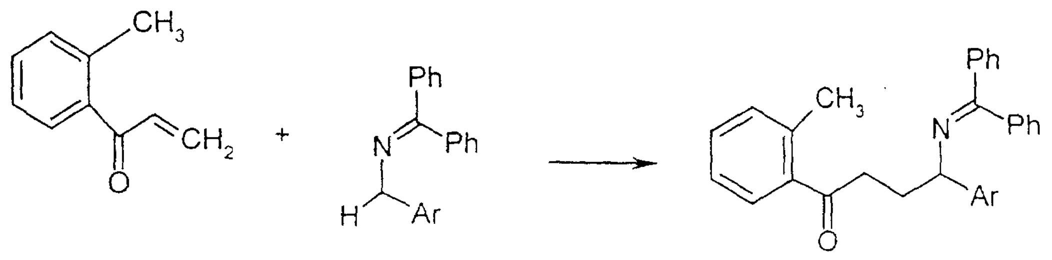

(XX) (XXIX) (XXVIII)

Die Addition wird in Gegenwart eines Säurebindemittels und in Gegenwart eines Verdünnungsmittels wie z. B. Acetonitril oder Dichlormethan und gegebenenfalls in Gegenwart eines Reaktionshilfsmittels z.B. bei Raumtemperatur durchgeführt. Als Säurebindemittel dient vorzugsweise wäßriges Alkali wie 50%-ige Natronlauge in Gegenwart eines Phasentransferkatalysators wie z.B. Triethylbenzylammoniumchlorid als Reaktionshilfsmittel [vgl. z.B. Synth. Commun.**17**, 211 (1987)].

Die Herstellung des Phenylvinylketons der Formel (XX) ist weiter oben beschrieben. Die *N*-Diphenylmethylenbenzylamine der Formel (XXIX) erhält man z.B. durch Umsetzung der entsprechenden Benzylamine mit Benzophenon (vgl. z.B. Tetrahedron. Lett. **1978**, 2641 ). Die hierfür benötigten Benzylamine sind bekannt oder können nach bekannten Methoden wie z.B. Aminolyse der entsprechenden Benzylhalonenide (siehe oben) hergestellt werden.

Die zur Durchführung des erfindungsgemäßen Verfahrens (B) benötigten cyclischen *O*-Methansulfonyloxime sind durch die Formel (III) allgemein definiert. In dieser Formel hat Ar vorzugsweise diejenigen Bedeutungen, die bereits im Zusammenhang mit der Beschreibung der cyclischen Imine der Formel (I) als bevorzugt genannt wurden. Die *O*-Methansulfonyloxime der Formel (III) sind neu.

Die *O*-Methylsulfonyloxime der formel (III) lassen sich herstellen, indem man z.B. gemäß dem nachfolgenden Reaktionsschema cyclische α-Arylketone der Formel (XXXI) zunächst nach allgemein bekannten Methoden in ihre Oxime der Formel (XXX) überführt und diese anschließend mit Methansulfonylchlorid analog der Mesylierung von Alkoholen umsetzt:

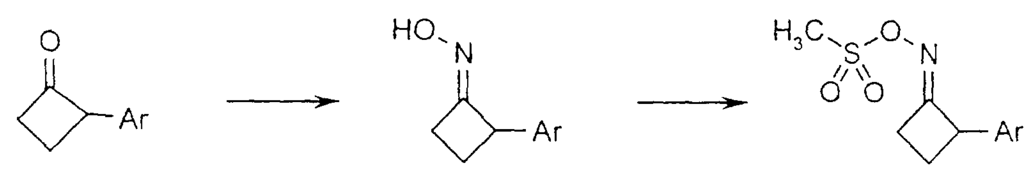

(XXXI) (XXX) (III)

Cyclische α-Arylketone der Formel (XXXI) lassen sich z.B. herstellen, indem man gemäß nachfolgendem Reaktionsschema 1-Aryleycloalkene der Formel (XXXIII) nach üblichen Methoden, wie beispielsweise mit m-Chlorperbenzoesäure, zu Oxiranen der Formel (XXXII) epoxidiert und diese anschließend durch saure Aufarbeitung isomerisiert [vgl. z.B. Tetrahedron **30**, 2027 (1974)]:

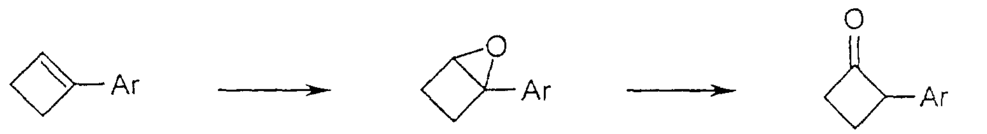

(XXXIII) (XXXII) (XXXI)

Natürlich kann man auch auf anderen Wegen erhaltene Oxirane der Formel (XXXII) z.B. durch Schütteln einer Lösung in Chloroform mit 20%-iger Schwefelsäure zu cyclischen α-Arylketonen der Formel (XXXI) isomerisieren.

1-Arylcycloalkene der Formel (XXXIII) lassen sich z.B. herstellen, indem man gemäß dem nachfolgenden Reaktionsschema weiter vorne beschriebene Aryl-Grignard-Verbindungen der Formel (XIV) mit Cyclobutanon der Formel (XXXV) unter üblichen Grignard-Bedingungen umsetzt und die z.B. auf diese Weise erhabenen cyclischen Benzylalkohole der Formel (XXXIV) dehydratisiert:

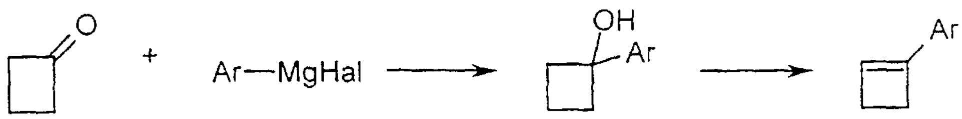

(XXXV) (XIV) (XXXIV) (XXXIII)

Die Dehydratisierung kann man beispielsweise durchführen, indem man den Alkohol in einem wenig polaren Lösungsmittel wie Hexan löst und bei z.B. 0° bis 20°C mit halbkonzentrierter Schwefelsäure verrührt [vgl. z.B. Tetrahedron **30**, 2027 (1974)].

Cyclobutanon der Formel (XXXV) ist kommerziell erhältlich.

Die weiterhin zur Durchführung des erfindungsgemäßen Verfahrens (B) benötigten Aryl-Grignard-Verbindungen sind durch die Formel (IV) definiert.

Die Aryl-Grignard-Verbindungen der Formel (IV) lassen sich durch Grignard-Reaktion aus o-Tolylhalogeniden und Magnesium herstellen. o-Tolylhalogenide sind allgemein bekannte Verbindungen der organischen Chemie.

Die zur Durchführung des erfindungsgemäßen Verfahrens (C) benötigten cyclischen Imine der Formel (V) sind, soweit $X^1$ für Brom oder Iod steht. Teilmengen der erfindungsgemäßen Verbindungen der allgemeinen Formel (I) und lassen sich beispielsweise nach den Verfahren (A) oder (B) herstellen. Für den Fall, daß $X^1$ für Trifluormethansulfonyl steht, lassen sie sich die Verbindungen der Formel (V-a) durch Umsetzung von Hydroxyverbindungen der Formel (I-f), welche ebenfalls nach den Verfahren (A) oder (B) hergestellt werden können, mit Trifluormethansulfonylchlorid oder Trifluormethansulfonsäureanhydrid in Gegenwart eines Säurebindemittels wie z.B. Pyridin und gegebenenfalls in Gegenwart eines Verdünnungsmittels gemäß folgendem Reaktionsschema herstellen:

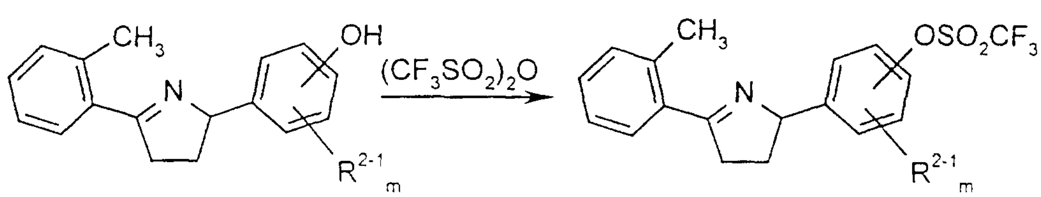


(I-f) (V-a)

Die ebenfalls zur Durchführung des erfindungsgemäßen Verfahrens (C) benötigten Boronsäuren sind durch die Formel (VI) allgemein definiert. In dieser Formel hat $R^{1-1}$ vorzugsweise diejenige Bedeutung, die bereits im Zusammenhang mit der Beschreibung der cyclischen Imine der Formel (1-b) als bevorzugt genannt wurden.

Aromatische Boronsäuren der Formel (VI) sind bekannt oder können nach bekannten Methoden hergestellt werden [vgl. Chem. Rev. **45,** 2457 (1995); Pure Appl. Chem. **66**, 213 (1994)].

Die zur Durchführung des erfindungsgemäßen Verfahrens (D) benötigten cyclischen Imine der Formel (I-d) sind Teilmengen der erfindungsgemäßen Verbindungen der allgemeinen Formel (1) und lassen sich beispielsweise nach den Verfahren (A) bis (C) herstellen.

Die weiteren zur Durchführung des erfindungsgemäßen Verfahrens (D) benötigten Verbindungen sind durch die Formel (VII) definiert. In dieser Formel haben $R^9$, $R^{10}$, G, p, q und r vorzugsweise diejenigen Bedeutungen, die bereits im Zusammenhang mit der Beschreibung der cyclischen Imine der Formel (I) als bevorzugt genannt wurden, Ab steht für eine übliche Abgangsgruppe wie z.B. Halogen, insbesondere Chlor oder Brom; Alkylsulfonyloxy, insbesondere Methylsulfonyloxy; oder gegebenenfalls substituiertes Arensulfonyloxy, insbesondere Phenylsulfonyloxy, p-Chlorsulfonylovy oder p-Tolylsulfonyloxy.

Die Verbindungen der Formel (VII) sind allgemein bekannte Verbindungen der Organischen Chemie.

Zur Durchführung des erfindungsgemäßen Verfahrens A) a) eignen sich organische oder anorganische Bronstedt-Säure wie beispielsweise Fluorwasserstoff, Chlorwasserstoff, Schwefelsäure, Phosphorsäure, Ameisensäure, Essigsäure, Benzoesäure, Citronensäure, Trifluoressigsäure, Methansulfonsäure, Trifluormethansulfonsäure oder Toluolsulfonsäure.

Insbesondere eignet sich die zur Abspaltung der tert.-Butoxycarbonyl-Aminoschutzgruppe üblicherweise eingesetzte Acidolyse mit Trifluoressigsäure (vgl. z.B. T.W. Greene, P.G.M. Wuts, Protective Groups in Organic Synthesis. 2. Ed.. John Wiley & Sons, New York 1991).

Das erfindungsgemäße Verfahren (A) a) wird gegebenenfalls in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydroxide, -amide, -alkoholate, -acetate, -carbonate oder -hydrogencarbonate, wie beispielsweise Natrium-, Kalium- oder Ammoniumhydroxid, Natriumamid, Lithiumdiisopropylamid, Natrium-methylat, Natrium-ethylat, Kalium-tert.-butylat, Natrium-, Kalium-, Calcium- oder Ammoniumacetat. Natrium-. Kalium- oder Ammoniumcarbonat, Natriumhydrogen- oder Kaliumhydrogencarbonat, sowie tertiäre Amine, wie Trimethylamin, Triethylamin, Tributylamin. N.N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (A) a) wird gegebenenfalls in Gegenwart eines Verdünnungsmittels durchgeführt. Hierfür kommen Wasser, organische Lösungsmittel und Mischungen davon in Betracht. Beispielhaft seien genannt: aliphatische. alicyclische oder aromatische Kohlenwasserstoffe, wie beispielsweise Petrolether, Hexan, Heptan, Cyclohexan, Methylcyclohexan, Benzol, Toluol, Xylol oder Decalin, halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether. Diethylenglykolmonoethylether.

Bei der Durchführung des Verfahrens (A) a) wird die Säure im allgemeinen im Überschuß eingesetzt.

Das erfindungsgemäße Verfahren (A) b) wird als katalytische Hydrierung oder nach anderen allgemein bekannten Methoden zur Reduktion von Nitrogruppen durchgeführt (vgl. z.B. Houben-Weyl, Methoden der Organischen Chemie, Georg thieme Verlag Stuttgart, Band **11/1**, 394 409 und Band **4/1c**, 490 506).

Das erfindungsgemäße Verfahren (A) c) wird als Hydrolyse nach allgemein bekannten Methoden z.B. mit wäßriger Salzsäure durchgeführt.

Als Verdünnungsmittel zur Durchführung der Verfahren (A) b) und (A) c) kommen die oben für das Verfahren (A) a) genannten Verdünnungsmittel in Frage.

Als Verdünnungsmittel zur Durchführung des erfindungsgemäßen Verfahrens (B) kommen inerte organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol.

Bevorzugt setzt man eine Lösung der Grignard-Verbindung der Formel (IV) in einem Ether und eine Lösung des *O*-Methylsulfonyloxim der Formel (III) in einem Kohlenwasserstoff ein.

Die Reaktionstemperatur kann bei dem erfindungsgemäßen Verfahren (B) innerhalb eines größeren Bereiches variiert werden. Im allgemeinen arbeitet man bei Temperaturen zwischen -100°C und +50°C, bevorzugt zwischen

-80°C und +30°C.

Bei der Durchführung des erfindungsgemäßen Verfahrens (B) werden Grignard-Verbindung der Formel (IV) und *O*-Nlethylsulfonyloxim der Formel (III) im molaren Verhältnis von 1: 1 bis 3:1, vorzugsweise 1:1 bis 2:1 eingesetzt.

Zur Durchführung des erfindungsgemäßen Verfahrens (C) sind Palladium(0)-Komplexe als Katalysator geeignet. Bevorzugt wird beispielsweise Tetrakis(triphenylphosphin)palladium.

Als Säureakzeptoren zur Durchführung des erfindungsgemäßen Verfahrens (C) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (C) kommen Wasser, organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen: Ether, wie Diethyl-, Diisopropyl-, Methyl-t-butyl-, Methyl-t-amylether, Dioxan, Tetrahydrofuran. 1,2-Dimethoxyethan, 1,2-Diethoxyethan, Diethylenglykoldimethylether oder Anisol; Alkohole, wie Methanol, Ethanol, n- oder 1-Propanol, n-, iso-, sek- oder tert-Butanol, Ethandiol, Propan-1,2-diol, Ethoxyethanol, Methoxyethanol, Diethylenglyholmonomethylether, Diethylenglykolmonoethylether.

Die Reaktionstemperatur kann bei dem erfindungsgemäßen Verfahren (C) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (C) werden Boronsäuren der Formel (VI) und Verbindungen der Formel (V) im molaren Verhältnis von 1:1 bis 3:1, vorzugsweise 1:1 bis 2:1 eingesetzt. Vom Katalysator setzt man im allgemeinen 0,005 bis 0,5 Mol. vorzugsweise 0,01 mol bis 0,1 Mol pro Mol der Verbindung der Formel (V) ein. Die Base setzt man im allgemeinen in einem Überschuß ein.

Das erfindungsgemäße Verfahren (D) wird vorzugsweise in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natrium-, Kalium- oder Ammoniumhydroxid, Natriumamid, Lithiumdiisopropylamid, Natrium-methylat, Natrium-ethylat, Kalium-tert.-butylat, Natrium-, Kalium-, Calcium- oder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (D) kann in Gegenwart eines geeigneten Phasentransferkatalysators durchgeführt werden. Als Beispiele für solche Katalysatoren seien genannt: Tetrabutylammoniumiodid, -bromid oder -chlorid. Tributylmethylphosphoniumbromid, Trimethyl-$C_{13}$/$C_{15}$-alkylammoniumchlorid oder -bromid, Dibenzyldimethylammonium-methylsulfat, Dimethyl-$C_{12}$/$C_{14}$-alkylbenzylammoniumchlorid, 15-Krone-5, 18-Krone-6 oder Tris-[2-(2-methoxyethoxy)-ethyl]-amin.

Das erfindungsgemäße Verfahren (D) wird vorzugsweise in Gegenwart eines Verdünnungsmittels durchgeführt. Hierfür kommen z.B. alle bei Verfahren (A) aufgelisteten Lösungsmittel in Frage.

Die Reaktionstemperatur kann bei dem erfindungsgemäßen Verfahren (D) innerhalb eines größeren Bereiches variiert werden. Im allgemeinen arbeitet man bei Temperaturen zwischen -20°C und +100°C, bevorzugt zwischen 0°C und 60°C.

Bei der Durchführung des erfindungsgemäßen Verfahrens (D) arbeitet man im allgemeinen mit ungefähr äquimolaren Mengen der Edukte. Man kann jedoch auch einen Überschuß der Verbindung der Formel (VII) verwenden.

Die Umsetzungen gemäß dem erfindungsmäßen Verfahren E) sind dem Fachmann bekannte Derivatisierungsreaktionen insbesondere von Carbonsäureestern und Ketonen (vgl. z.B. Houben-Weyl, Methoden der Organischen Chemie, Georg Thieme Verlag, Stuttgart, Bd. VII/2b, insbesonders 1912 ff; Bd. VIII zu Carbonsäureestern und deren Derivaten; Bd. E5, insbesondere S. 812 ff und die darin zitierte Literatur).

Die Umsetzungen der erfindungsgemäßen Verfahren können bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch Entfernung der flüchtigen Bestandteile gegebenenfalls im Vakuum gereinigt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus. Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica. Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata,. Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich insbesondere durch hervorragende Wirkung gegen Larven des Meerettichblattkäfers (Phaedon cochleariae), Raupen des Eulenfalters (Spodoptera frugiperda), Larven der Grünen Reiszikade (Nephotettix cincticeps), Pfirsichblattläuse (Myzus persicae) und alle Stadien der gemeinen Spinnmilbe (Tetranychus urticae).

Die Wirkstoffe können in die üblichen formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. und bevorzugt durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel. wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester. Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose. natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Werkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Besonders günstige Mischpartner sind z.B. die folgenden:

**Fungizide:**

2-Aminobutan: 2-Anilino-4-methyl-6-cyclopropyl-pyrimidin; 2'.6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorobenzol, Hexaconazol, Hymexazol,

Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat. Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol.
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon.
Quintozen (PCNB).
Schwefel und Schwefel-Zubereitungen.
Tebuconazol, Tecloftalam, Tecnazen, Tetraconazol, Thiabendazol, Thicyofen, Thiophanat-methyl, Thiram, Tolclophos-methyl, Tolylfluanid, Triadimefon. Triadimenol, Triazoxid, Trichlamid, Tricyclazol, Tridemorph, Triflumizol, Triforin, Triticonazol,
Validamycin A, Vinclozolin,
Zineb, Ziram.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypemethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemeton M, Oxydeprofos,
Parathion A, Parathion M, Permethrin, Phenthoat, Phorat, Phosalon, Phosmet, Phosphamidon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiometon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in seinen handelsüblichen Formulierungen sowie in den aus

diesen Formulierungen bereiteten Anwendungsformen in Mischung mit Synergisten vorliegen. Synergisten sind Verbindungen, durch die die Wirkung der Wirkstoffe gesteigert wird. ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.
Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp.,

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen alle larvalen Stadien der Fliege *Lucilia cuprina* und alle Entwicklungsstadien der Zecke *Amblyomma variegatum*.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten. Kapseln, Tränken. Drenchen. Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.
Beispielhaft und vorzugsweise - ohne jedoch zu limitieren - seien die folgenden Insekten genannt:

Käfer wie

Hylotrupes bajulus, Chlorophorus pilosis, Anobium punctatum, Xestobium ruFovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis. Xyleborus spec. Tryptodendron spec. Apate monachus, Bostrychus capucins, Heterobostrychus brunneus, Sinoxylon spec. Dinoderus minutus

Hautflügler wie

Sirex juvencus, Urocerus gigas, Urocerus gigas taignus, Urocerus augur

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw, dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten. Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,000 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0.0001 bis 20 Gew.-%. vorzugsweise 0.001 bis 10 Gew.-%. des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise Monochlornaphthalin, verwendet.

Die organischen schwerflüchtigen, öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glykolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide, wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

**Herstellungsbeispiele**

**Beispiel I-9**

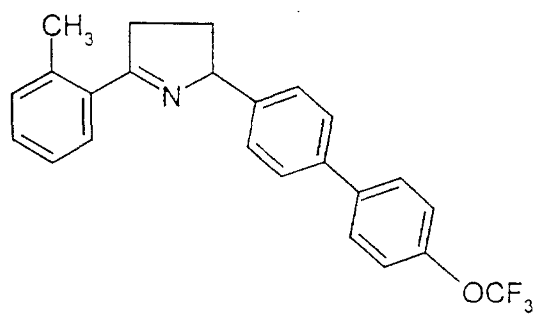

210 mg (0,4 mmol) 1-tert.-Butyloxycarbonylamino-1-[4'-'Trifluormethoxybiphenyl-4-yl-]-3-[O-Methylbenzoyl]-propan wurden in 5 ml $CH_2Cl_2$ vorgelegt und die Lösung auf 0°C gekühlt. Anschließend wurde 1 ml Trifluoressigsäure zugegeben und 4 h bei Raumtemperatur gerührt. Dann wurde das Lösungsmittel im Vakuum entfernt.

Zur Aufarbeitung wurde das Produktgemisch mehrfach mit Toluol versetzt, welches anschließend wieder im Vakuum entfernt wurde. Schließlich wurde der Rückstand in Ether aufgenommen, zweimal mit Wasser gewaschen, getrocknet und eingeengt.

Man erhielt 150 mg (93,1 % d. Th.) 2-[2-Methylphenyl]-5-[4'-Trifluormethoxybiphenyl-4-yl-]-3,4-Dihydro-2H-pyrrol als Öl.
$^1$H-NMR (400 MHz, $CDCl_3$, TMS): δ [ppm] = 1,88 (m, 1H, CHNC<u>H</u>H), 2.61 (m, 1H, CHNCH<u>H</u>), 2.62 (s. 3H, C<u>H</u>$_3$), 3.10 (m, 2H, CNC<u>H</u>$_2$), 5.38 (t, 1H, CHN), 7.20-7.35 (5H, ArH), 7.46 (m, 2H, ArH), 7.53 (m, JH, ArH), 7.60 (m. 2H, ArH)
HPLC log P* (neutral) = 6.05
LC-MS: 396 M⊕ bei 10.53 min.
* log P = negativer dekadischer Logarithmus des Alkan/Wasser Verteilungskoeffizienten, bestimmt durch HPLC Analytik mit Wasser/Acetonitril als Laufmittel auf 125 × 4,0 mm Kromasil 120°C 18 (5 µm); Fluß: 1,5 ml/min.

**Beispiel 1-9 "Eintopfvariante":**

0,964 g (3 mmol) XI-a-2 wurden in 20 ml THF vorgelegt und auf -78°C abgekühlt. Danach wurden bei -78°C 2,05 ml (3,3 mmol) n-Butyllithium (1,6 N Lösung in Hexan) zugetropft und 30 min bei -78°C gerührt. Anschließend wurden bei -78°C 0,72 g (3,3 mmol) Ditertiärbutyldicarbonat in 6 ml THF zugetropft. Nach wenigen Minuten Nachrühren bei -78°C wurde die Mischung auf 0°C erwärmt und wieder auf -78°C abgekühlt. Nun wurden 19,5 ml (3,9 mmol) o-Tolylmagnesiumbromid (2.0 M in $Et_2O$) bei -78°C zugetropft und unter Rühren auf 0°C erwärmt. Bei 0°C wurden 7,5 ml Trifluoressigsäure zugesetzt und die Mischung noch 4 h bei Raumtemperatur gerührt. Das Tetrahydrofuran wurde am Rotationsverdampfer entfernt, der Rückstand in 25 ml Methylenchlorid aufgenommen und mit 7,5 ml Trifluoressigsäure versetzt.

Es wurde weitere 6 h bei Raumtemperatur gerührt. Anschließend wurden alle flüchtigen Bestandteile am Rotationsverdampfer entfernt und der Rückstand an Kieselgel mit Essigester/Cyclohexan 1:2 chromatographiert.

Es wurden zwei Fraktionen isoliert. Die zweite enthält 434 mg des gewünschten Produkts I-9.

Die erste Fraktion enthielt das Trifluoracetat von 1-9 und wurde weiter aufgearbeitet:

Zunächst wurde die Fraktion in Essigester aufgenommen und mit $NaHCO_3$-Lösung ausgeschüttelt, dann wurde mit 100 ml Essigester mit 20 % NaOH versetzt und über Nacht bei Raumtemperatur gerührt.

Die Lösung wurde dreimal mit gesättigter Kochsalzlösung gewaschen, getrocknet und das Solvent am Rotationsverdampfer entfernt. Der Rückstand wurde dreimal mit Toluol versetzt, welches anschließend wieder im Vakuum entfernt wurde.

Nach Entfernen aller flüchtigen Bestandteile im Vakuum wurde nochmals an Kieselgel mit Essigester/Cyclohexan 1:3 chromatographiert.

Es wurden zwei Fraktionen isoliert, die erste enthielt weitere 36 mg 1-9, die zweite 61 mg des Trifluoracetats.

Es wurde eine Gesamtausbeute von 470 (434 + 36) mg (46 % d. Th.) I-9 erhalten.
(Physikalische Daten s. "Stufenvariante")

**Beispiel I-43**

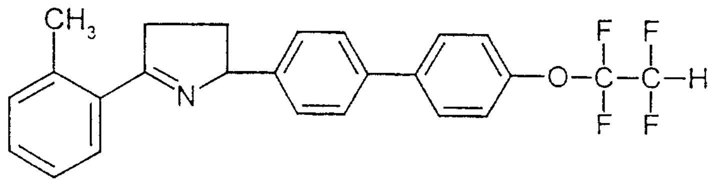

1 g der Verbindung (VIII-43) wurden in 10 ml $CH_2Cl_2$ vorgelegt und auf 0°C gekühlt. 1.39 ml Trifluoressigsäure wurden zugegeben und die Mischung anschließend über Nacht bei Raumtemperatur gerührt. Die Triflouressigsäure wurde unter Vakuum abrotiert und der Rückstand in Essigester aufgenommen. Mit 1N NaOH wurde der pH-Wert auf 11 gestellt. Die organische Phase wurde mit Wasser gewaschen, über $MgSO_4$ getrocknet, filtriert und eingeengt.

Es wurde 0,23 g eines Feststoffs erhalten.
Fp.: 87°C
HPLC: LogP (pH 2,3) = 3.02

**Herstellung der Ausgangsprodukte**

**γ-Ethoxy-γ-butyrolactam**

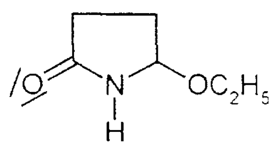

9.91 g Succinimid wurden bei 0°C in 415 ml Ethanol vorgelegt und portionsweise mit insgesamt 5,53 g Natriumboranat versetzt. Es wurden bei dieser Temperatur über 4½ Stunden alle 15 Minuten 2 bis 3 Tropfen 2N ethanolischer Chlorwasserstoff zugetropft. Anschließend wurde mit weiterer Säure auf pH 3 angesäuert. Nach 1 Stunde Rühren bei 0°C wurde mit 1%-iger ethanolischer Kalilauge neutralisiert, weitere 15 Minuten gerührt und eingedampft. Der Rückstand wurde in Wasser aufgenommen und dreimal mit Dichlormethan extrahiert. Nach Trocknen über Natriumsulfat und Einengen wurden 7,16 g (55 % d.Th.) γ-Ethoxy-γ-butyrolactam erhalten.

**Beispiel XI-1**

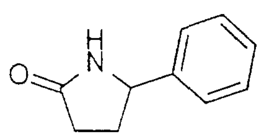

6.45 g γ-Ethoxy-butyrolactam und 50 ml konz. Schwefelsäure wurden bei 0°C vorgelegt und 18,8 ml Benzol zugesetzt. Nach dem Auftauen wurde 4 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Es wurden 8.1 g (100 % d.Th.) γ-Phenyl-γ-butyrolactam erhalten.
$^1$H-NMR (400 MHz, $d_6$-DMSO) δ [ppm]: 1,75 (m, 1H); 2,23 (t, 2H); 2,45 (m, 1H); 4,67 (t, 1 H); 7,26-7,39 (m, 5H); 8,08 (br, 1H)

**Beispiel XI-2**

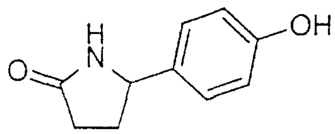

(XI-2a)

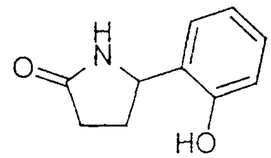

(XI-2b)

12,9 g γ-Ethoxy-γ-butyrolactam, 10 ml konz. Schwefelsäure und 90 ml Eisessig wurden bei 0°C vorgelegt und portionsweise mit insgesamt 18,8 g Phenol versetzt. Nach dem Auftauen wurde 2 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Aus der wäßrigen Phase kristallisierte nach einiger Zeit γ-2-Hydroxyphenyl-γ-butyrolactam (XI-2b) vom Schmelzpunkt 220°C aus (6,4 g 36 % d.Th). Der Eindampfrückstand wurde mit einem 1 : 1 -Gemisch Cyclohexan/Ethylacetat verrührt und lieferte nach Absaugen 4,65 g -γ-4-Hydrosyphenyl- γ-butvrolactam (X1-2a) vom Schmelzpunkt 183°C. Das Filtrat wurde eingedampft. Durch Umkristallisieren aus Dichlormethan/Hexan wurden weitere 3,35 g (gesamt: 45 % d.Th.) γ-4-Hydroxyphenyl-γ-butyrolactam erhalten.

**Beispiel XVII-2**

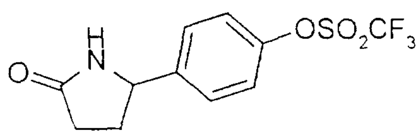

Zu 5,23 g γ-4-Hydroxyphenyl-γ-butyrolactam (z.B. aus Bsp. XI-2) in 60 ml Pyridin wurden bei 0°C 10 g Trifluormethansulfonsäureanhydrid getropft. Nach Rühren über Nacht bei Raumtemperatur wurde auf Eis gegossen, mit 10%-iger Salzsäure angesäuert und dreimal mit Ethylacetat extrahiert. Nach Trocknung und Abdampfen des Lösungsmittels wurden 6,4 g (70 % d.Th.) γ-4-Trifluormethylsulfonyloxyphenyl-γ-butyrolactam vom Schmelzpunkt 127°C erhalten.

**Beispiel XI-a-2**

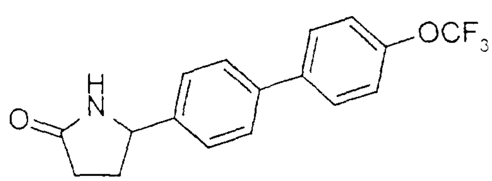

5,4 g γ-4-Trifluormethylsulfonyloxyphenyl-γ-butyrolactam (z.B. aus Bsp. XVII-2) wurden unter Argon in 43 ml Dimethoxyethan vorgelegt. Nacheinander gab man 5,87 g 4-Trifluormethoxyboronsäure sowie 1,01 g Tetrakis(triphenylphosphin)palladium zu. Nach 15 Minuten wurden 28 ml 2M Sodalösung zugegeben, auf 80°C erwärmt und über Nacht gerührt. Nach Beendigung der Reaktion wurde in Wasser/Ethylacetat aufgenommen, die Phasen getrennt und die wäßrige Phase zweimal mit Ethylacetat extrahiert. Die vereinigten organischen Phasen wurden mit gesättigter

Kochsalzlösung gewaschen und getrocknet. Durch Eindampfen wurden 5,5 g (98 % d.Th.) γ-4'-Trifluormethoxybiphenyl-4-yl-γ-butyrolactam vom Schmelzpunkt 128°C erhalten.

**Beispiel XI-3**

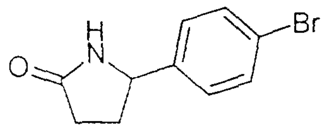

In einem 3 1-Dreihalskolben mit Rührer und Destillationsbrücke wurden 199,3 g Ammoniumformiat in 127,9 Ameisensäure vorgelegt und 210 g aus Toluol umkristallisierte 4-Brombenzoylpropionsäure zugegeben. Darauf wurde der Kolben in ein 200°C heißes Ölbad getaucht. Bei 60°C beginnt der Kolbeninhalt unter Gasentwicklung in Lösung zu gehen. Über ca. 2 h wird bei von 140 bis auf 167°C steigender Sumpftemperatur wird ausdestilliert. Nach Abkühlen auf unter 60°C wurden vorsichtig 1 1 Dichlormethan zugegeben und ausgefallenes Salz durch Absaugen über eine Filternutsche abgetrennt. Die organische Phase wurde mit 11 Wasser gewaschen, über Magnesiumsulfat getrocknet und im Vakuum eingeengt. Zur Reinigung wurde das Rohprodukt wurde über 1 kg Kieselgel mit Dichlormethan/Ethanol/Triethylamin (95:5:3) filtriert und anschließend aus Methyl-tert.butyl-ether kristallisiert. Es wurden 38 g (19 % d.Th.) γ-4-Bromphenyl-γ-butyrolactam vom Schmelzpunkt 142°C erhalten.

**Beispiel XI-43**

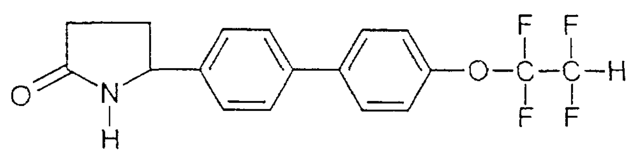

In einem Autoklav wurden 400 ml HF einkondensiert. Dann wurden 38,7 g γ-ethoxy-γ-butyrolactam und 40,6 g Tetrafluorethoxy-biphenyl gemeinsam in 100 ml $CH_2Cl_2$ gelöst und zugegeben. Das Reaktionsgemisch wurde über Nacht bei Raumtemperatur gerührt. HF wurde abgezogen, der Rückstand in $CH_2Cl_2$, aufgenommen und mit wässriger Natriumhydrogen-Lösung gewaschen. Die organische Phase wurde getrocknet, über $MgSO_4$ filtriert und eingeengt. Die Rohausbeute wurde aus 500 ml Toluol umkristallisiert. 20,9 g weißer Feststoff wurde erhalten.
HPLC: log.P (PH 2-3) = 2.79

**Beispiel IX-1**

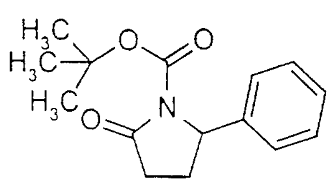

3,4 g γ-Phenyl-γ-butyrolactam (z.B aus Bsp. XI-1) wurden in 63 ml Tetrahydrofuran (THF) vorgelegt und bei -78°C mit 9,24 ml 2,4N Butyllithium-Lösung in n-Hexan versetzt. Nach halbstündigem Rühren bei dieser Temperatur wurde eine Lösung von 5,04 g Di-tert.-Butyldicarbonat in 20 ml THF unter weiterer Kühlung zugetropft, weitere 3 Stunden bei -78°C und dann über Nacht ohne Kühlung gerührt. Danach wurde mit gesättigter wäßriger Ammoniumchlorid-Lösung hydrolisiert, mit Wasser verdünnt und dreimal mit Ethylacetat extrahiert. Die vereinigten organischen Phasen wurden mit gesättigter Kochsalzlösung gewaschen und über Magnesiumsulfat getrocknet. Durch Eindampfen wurden 1,54 g (28 % d.Th.) *N*-$^t$Butoxycarbonyl-γ-phenyl-γ-butyrolactam erhalten.
$^1$H-NMR (400 MHz, $d_6$-DMSO) δ [ppm]: 1.18 (s. 9H); 1,73 (m, 1H); 2,40-2,60 (m, 3H); 5,10 (m, 1H), 7,24 (m, 2H); 7,30 (m, 1H); 7,38 (m, 2H)

**Beispiel IX-2**

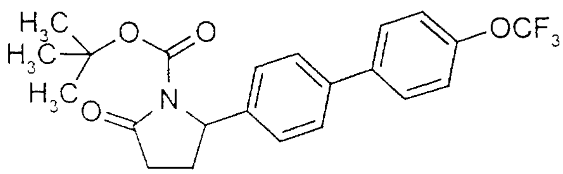

1.7 g γ-4'-Trifluormethoxybiphenyl-4-yl-γ-butyrolactam (z.B. aus Bsp. XI-a-2) wurden in 30 ml Tetrahydrofuran (THF) vorgelegt und bei -78°C mit 2,42 ml 2,4N Butyllithium-Lösung in n-Hexan versetzt. Nach halbstündigem Rühren bei dieser Temperatur wurde eine Lösung von 1.27 Di-tert.-Butyldicarbonat in 10 ml THF unter weiterer Kühlung zugetropft. Dann wird die Kühlung entfernt und bei Raumtemperatur über Nacht gerührt. Danach wurde mit gesättigter wäßriger Ammoniumchlorid-Lösung hydrolisiert, mit 2N Salzsäure angesäuert und dreimal mit Dichlormethan extrahiert. Nach Trocknung und Eindampfen wurde das Produkt durch Säulenchromatographie gereinigt (stationäre Phase: Kieselgel; mobile Phase Gradient Cyclohexan:Ethylacetat = 5:1,3 bis 1,1:1). Es wurden 1,14 g (47% d.Th.) teilkristallines *N*-$^t$Butoxycarbonyl-γ-4'-trifluormethoxybiphenyl-4-yl-γ-butyrolactam erhalten.
$^1$H-NMR (400 MHz, $CDCl_3$) δ [ppm]: 1,22 (s, 9H); 1,79 (m, 1H); 2,48-2,60 (m, 3H): 5,17 (m, 1H); 7,36 (d, 2H); 7,46 (d, 2H); 7,71 (d, 211); 7,80 (d, 2H)

**Beispiel IX-3**

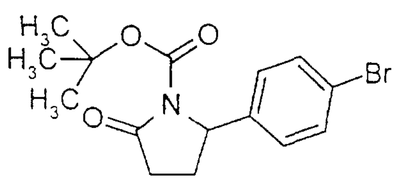

3,24 ml Diisopropylamin wurden in 90 ml THF bei -78°C vorgelegt und mit 9,24 ml 2,4N Butyllithium-Lösung in n-Hexan versetzt. Nach ½ h Rühren bei dieser Temperatur wurden eine Lösung von 5,02 g -4-Bromphenyl- -butyrolactam (z.B. aus Beispiel XI-3) in 20 ml THF zugetropft. Nach weiterer ½ h Rühren bei -78°C wurden 5,04 g Di-tert. -Butyldicarbonat in 20 ml THF zugetropft, auftauen gelassen und bei Raumtemperatur über Nacht gerührt. Danach wurde mit gesättigter wäßriger Ammoniumchlorid-Lösung hydrolisiert, mit 2N Salzsäure angesäuert und mit 150 ml Dichlormethan dreimal extrahiert. Nach Trocknung über Magnesiumsulfat und Eindampfen wurde das Produkt durch Kristallisieren aus Dichlormethan/Hexan gereinigt. Es wurden insgesamt 7,61 g (97 % d.Th.) kristallines *N*-$^t$Butoxycarbonyl-γ-4-bromphenyl-γ-butvrolactam erhalten. Die reinste Kristallfraktion (2.34 g) schmolz bei 122-124°C.

**Beispiel IX-43**

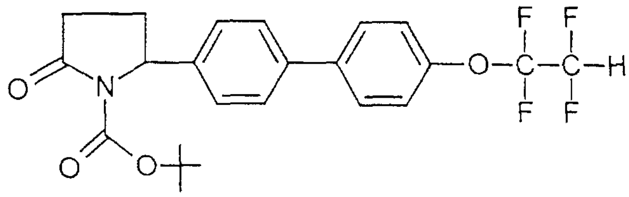

8.8 der Verbindung (XI-43) wurden mit 8,1 g Pyrokohlensäuretertiärbutylester und 0,2 g Kaliumfluorid in 80 ml Toluol auf 108°C 6 Stunden erhitzt, anschließend ließ man über Nacht bei Raumtemperatur stehen. 6,3 g ausgefallener Feststoff (der ausgefallene Feststoff entspricht ebenfalls dem gewünschten Produkt) würde abgesaugt. Die Toluol-Lösung wurde mit Wasser gewaschen, die organische Phase über $MgSO_4$ getrocknet, filtriert und einrotiert. Es wurden 6.1 g weißer Feststoff isoliert. Fp. 134°C; log P (pH 2,3) = 3,96).

**Beispiel VIII-2**

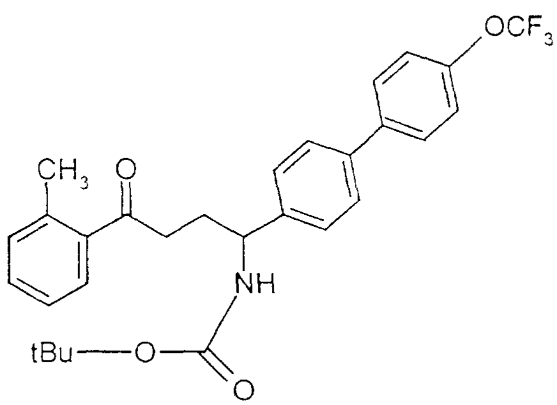

420 mg (1 mmol) N-tert.-Butoxycarbonyl-γ-4'-trifluormethoxybiphenyl-4-yl-γ-butyrolactam (aus Beispiel IX-2) wurden in 25 ml THF vorgelegt und auf -78°C abgekühlt.

Anschließend wurden bei -78°C unter Rühren 6,5 ml (13 mmol) o-Tolylmagnesiumbromid (2 M in $Et_2O$) zugetropft.

Danach wurde die Reaktionsmischung mit gesättigter Ammoniumchloridlösung hydrolysiert und mit Essigester extrahiert. Die organischen Phasen wurden vereinigt, getrocknet und eingeengt.

Abschließend wurde das Rohprodukt an Kieselgel mit Essigester/Cyclohexan 1:5 chromatographiert.

Es wurden 0,228 g (44,9 % d. Th.) 1-tert.-Butyloxycarbonylamino-1-[4'-Trifluormethoxybiphenyl-4-yl-]-3-[o-Methylbenzoyl]-propan als Öl erhalten.

$^1$H-NMR (400 MHz, $CDCl_3$, TMS): δ [ppm] = 1,19 (s, 9H, $[CH_3]_3$, 1.79, 2.12, 2.22, 2.53 (m, 1H, C$\underline{H}$H), 2.58 (s, 3H, C$\underline{H_3}$), 5.11 (d, 1H, CHN), 5.49 (s, 1H, NH), 7.15-7.65 (12H, ArH).

**Beispiel VIII-43**

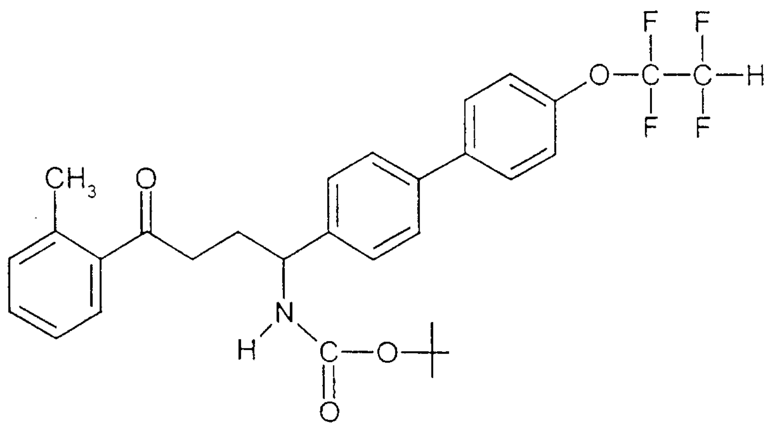

Wurde analog Beispiel VIII-2 hergestellt.
log P (pH 2,3) = 5.05
Fp. 134-135°C

**Biologische Beispiele**

**Beispiel A**

Heliothis armigera-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Sojatriebe (Glycine max) werden durch Tauchen in die Wirkstoffzubereitung der gewünschten Konzentration behandelt und mit Raupen des Baumwollkapselwurms (Heliothis armigera) besetzt, solange die Blätter noch feucht sind.
Nach der gewünschten Zeit wird die Abtötung in % bestimmt. Dabei bedeutet 100 %. daß alle Raupen abgetötet wurden; 0 % bedeutet, daß keine Raupen abgetötet wurden.
Bei diesem Test zeigt z.B. die Verbindung gemäß Herstellungsbeispiel I-9 in einer beispielhaften Wirkstoffkonzentration von 0,004 % eine Abtötung von 100 % nach 6 Tagen.

**Beispiel B**

Phaedon-Larven-Test

| | |
|---|---|
| Lösungsmittel | 7 Gewichtsteile Dimethylformamid |
| Emukgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf

die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine häferlarven abgetötet wurden.

Bei diesem Test zeigt z.B. die Verbindung gemäß Herstellungsbeispiel 1-9 bei einer beispielhaften Wirkstoffkonzentration von 0,004 % eine Abtötung von 100 % nach 6 Tagen, die Verbindung gemäß Hlerstellungsbeispiel I-43 zeigte dabei bei einer beispielhaften Wirkstoffkonzentration von 0,01 % eine Abtötung von 100 % nach 7 Tagen.

## Beispiel C

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet werden; 0 % bedeutet, daß keine Raupen abgetötet wurden.

Bei diesem Test zeigt z.B. die Verbindung gemäß Herstellungsbeispiel 1-9 bei einer beispielhaften Wirkstoffkonzentration von 0,004 % eine Abtötung von 100 % nach 6 Tagen.

## Beispiel D

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung gemäß Herstellungsbeispiel I-9 bei einer beispielhaften Wirkstoffkonzentration von 0,004 % eine Abtötung von 100 % nach 6 Tagen, die Verbindung gemäß Herstellungsbeispiel 1-43 zeigte dabei bei einer beispielhaften Wirkstoffkonzentration von 0,01 % eine Abtötung von 100 % nach 7 Tagen.

**Beispiel E**

**Tetranychus-Test** (OP-resistent/Tauchbelhandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die Verbindung gemäß Herstellungsbeispiel I-9 bei einer Wirkstoffkonzentration von 0,004 % eine Abtötung von 98 % nach 7 Tagen, die Verbindung gemäß Herstellungsbeispiel I-43 zeigte dabei bei einer beispielhaften Wirkstoffkonzentration von 0,01 % eine Abtötung von 100 % nach 7 Tagen.

**Patentansprüche**

**1.** Verbindungen der Formel (I)

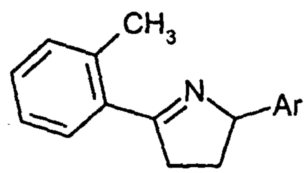

(I)

in welcher

Ar für den Rest

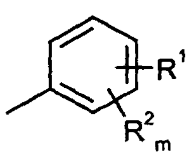

steht, in welchem

m für 0, 1, 2, 3 oder 4 steht,

$R^1$ für einen Substituenten in meta- oder para-Position aus der Reihe Wasserstoff, F, Cl, Br, Cyano, Tri-($C_1$-$C_6$-alkyl)-silyl, -CO-$NR^4R^5$, Tetrahydropyranyl oder eine der folgenden Gruppierungen

(l) -X-A

(m) -B-Z-D

(n) -Y-E

steht,

$R^2$ für Wasserstoff, Halogen, Cyano, Nitro, $C_1$-$C_{16}$-Alkyl, $C_1$-$C_{16}$-Alkoxy, $C_1$-$C_6$-Halogenalkyl, $C_1$-$C_6$-Halogenalkoxy, $C_1$-$C_8$-Alkoxy-$C_1$-$C_8$-alkoxy oder -$S(O)_oR^3$, mit der Maßgabe, dass $R^1$ nicht für Wasserstoff steht, wenn m = 1 und $R^2$ = Iod, steht,

o für 0, 1 oder 2 steht,

$R^3$ für gegebenenfalls durch Fluor oder Chlor substituiertes $C_1$-$C_6$-Alkyl steht,

$R^4$ und $R^5$ unabhängig voneinander für Wasserstoff, $C_1$-$C_6$-Alkyl, $C_1$-$C_6$-Halogenalkyl oder für jeweils gegebenenfalls einfach bis dreifach durch Reste aus der Liste W' substituiertes Phenyl oder Phenyl-$C_1$-$C_4$-alkyl stehen,

X für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder Di-$C_1$-$C_4$-alkylsilylen steht,

A für jeweils gegebenenfalls einfach bis vierfach durch Reste aus der Liste $W^1$ substituiertes Phenyl, Naphthyl oder Tetrahydronaphthyl oder für jeweils gegebenenfalls einfach bis vierfach durch Reste aus der Liste $W^2$ substituiertes 5- bis 10- gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, steht,

B für gegebenenfalls einfach oder zweifach durch Reste aus der Liste W' substituiertes p-Phenylen steht,

Z für Sauerstoff oder Schwefel steht,

D für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, jeweils gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl oder $C_3$-$C_8$-Cycloalkyl-$C_1$-$C_6$-alkyl, für jeweils gegebenenfalls durch Halogen oder $C_1$-$C_4$-Alkyl substituiertes $C_5$-$C_8$-Cycloalkenyl oder $C_5$-$C_8$-Cycloalkenyl-$C_1$-$C_4$-alkyl, für jeweils gegebenenfalls durch Nitro, Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl-$C_1$-$C_6$-alkyl, Naphthyl-$C_1$-$C_6$-alkyl, Tetrahydronaphthyl-$C_1$-$C_6$-alkyl oder 5- oder 6-ringgliedriges Hetaryl-$C_1$-$C_6$-alkyl mit 1 oder 2 Heteroatomen aus der Reihe Stickstoff, Sauerstoff und Schwefel, für -CO- $R^6$, -CO-$NR^7R^8$ oder für die Gruppierung

$$-(CH_2)_p-(CR^9R^{10})_q-(CH_2)_r-G$$

steht,

Z und D können auch gemeinsam für jeweils gegebenenfalls durch Nitro, Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenoxy-$C_1$-$C_4$-alkyl stehen,

Y für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder für gegebenenfalls einfach oder zweifach durch Reste aus der Liste $W^1$ substituiertes p-Phenylen steht,

E für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl, für gegebenenfalls durch Halogen oder $C_1$-$C_4$-Alkyl substituiertes $C_5$-$C_8$-Cycloalkenyl, für gegebenenfalls einfach bis vierfach durch Reste aus der Liste $W^1$ substituiertes Phenyl oder für gegebenenfalls einfach bis vierfach durch Reste aus der Liste $W^2$ substituiertes 5- oder 6-gliedriges Hetaryl mit 1 oder 2 Heteroatomen aus der Reihe Stickstoff, Sauerstoff und Schwefel oder für die Gruppierung

$$-(CH_2)p-(CR^9R^{10})q-(CH_2)_r-G$$

steht,

$R^6$ für $C_1$-$C_{12}$-Alkyl, $C_1$-$C_{12}$-Alkoxy, $C_2$-$C_{12}$-Alkenyl, $C_2$-$C_{12}$-Alkenyloxy, jeweils gegebenenfalls durch Halogen, $C_1$-$C_4$-Alkyl, $C_2$-$C_4$-Alkenyl, $C_1$-$C_4$-Halogenalkyl oder $C_2$-$C_4$-Halogenalkenyl substituiertes $C_3$-$C_8$-Cycloalkyl, $C_3$-$C_8$-Cycloalkyloxy oder $C_3$-$C_8$-Cycloalkyl-$C_1$-$C_6$-alkyloxy

oder für jeweils gegebenenfalls einfach bis vierfach durch Nitro, Halogen, $C_1$-$C_{12}$-Alkyl, $C_1$-$C_{12}$-Alkoxy, $C_1$-$C_{12}$-Halogenalkyl oder $C_1$-$C_{12}$-Halogenalkoxy substituiertes Phenyl oder Naphthyl steht,

$R^7$ für Wasserstoff oder $C_1$-$C_{12}$-Alkyl steht,

$R^8$ für $C_1$-$C_{12}$-Alkyl, $C_1$-$C_{12}$-Halogenalkyl, jeweils gegebenenfalls durch Halogen, $C_1$-$C_4$-Alkyl, $C_2$-$C_4$-Alkenyl, $C_1$-$C_4$-Halogenalkyl oder $C_2$-$C_4$-Halogenalkenyl substituiertes $C_3$-$C_8$-Cycloalkyl oder $C_3$-$C_8$-Cycloalkyl-$C_1$-$C_6$-alkyl oder für jeweils gegebenenfalls einfach bis vierfach durch Halogen, $C_1$-$C_{12}$-Alkyl, $C_1$-$C_{12}$-Alkoxy, $C_1$-$C_{12}$-Halogenalkyl oder $C_1$-$C_{12}$-Halogenalkoxy substituiertes Phenyl oder Phenyl-$C_1$-$C_6$-alkyl steht,

p, q und r unabhängig voneinander für 0, 1, 2 oder 3 stehen, wobei deren Summe kleiner als 6 und größer als 1 ist,

$R^9$ und $R^{10}$ unabhängig voneinander für Wasserstoff oder $C_1$-$C_4$-Alkyl stehen,

G für Cyano, für einen gegebenenfalls einfach bis dreifach durch Halogen, $C_1$-$C_4$-Alkyl oder $C_1$-$C_4$-Halogenalkyl und gegebenenfalls an der Verknüpfungsstelle durch den Rest $R^{11}$ substituierten 5- oder 6-gliedrigen Heterocyclus mit 1 bis 3 gleichen oder verschiedenen Heteroatomen aus der Reihe Stickstoff, Sauerstoff und Schwefel oder eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

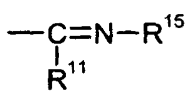

(f)

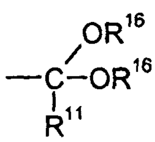

(g)

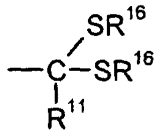

(h)

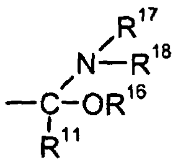

(i)

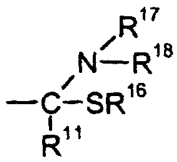

(j)

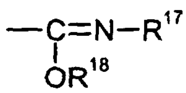

(k)

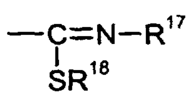

steht,

$R^{11}$ für Wasserstoff, $C_1$-$C_6$-Alkyl, $C_2$-$C_6$-Alkenyl, $C_1$-$C_4$-Halogenalkyl, $C_2$-$C_6$-Halogenalkenyl, gegebenenfalls durch Halogen, $C_1$-$C_4$-Alkyl oder $C_1$-$C_4$-Halogenalkyl substituiertes $C_3$-$C_6$-Cycloalkyl oder für gegebenenfalls einfach bis fünffach durch $C_1$-$C_4$-Alkylcarbonylamino, $C_1$-$C_4$-Alkylcarbonyl-$C_1$-$C_4$-alkylamino und/oder Reste aus der Liste $W^3$ substituiertes Phenyl steht,

$R^{12}$ für Wasserstoff, $C_1$-$C_4$-Alkyl, $C_2$-$C_6$-Alkenyl, $C_1$-$C_4$-Halogenalkyl, $C_2$-$C_6$-Halogenalkenyl, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl oder für gegebenenfalls einfach bis vierfach durch Reste aus der Liste $W^3$ substituiertes $C_6$-$C_{10}$-Aryl-$C_1$-$C_4$-alkyl steht,

$R^{13}$ und $R^{14}$ unabhängig voneinander für Wasserstoff, $C_1$-$C_4$-Alkyl, $C_3$-$C_6$-Alkenyl, $C_1$-$C_4$-Halogenalkyl, $C_3$-$C_6$-Halogenalkenyl, $C_1$-$C_4$-Alkoxy, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl, für jeweils gegebenenfalls einfach bis fünffach durch Reste aus der Liste $W^3$ substituiertes Phenyl oder Phenyl-$C_1$-$C_4$-alkyl, für -$OR^{12}$ oder -$NR^{11}R^{12}$ oder gemeinsam für eine Alkylenkette mit 4 bis 6 Gliedern, in welcher gegebenenfalls eine Methylengruppe durch Sauerstoff ersetzt ist, stehen,

$R^{15}$ für -$OR^{12}$, -$NR^{11}R^{12}$ oder -$N(R^{11})$-$COOR^{12}$ steht,

$R^{16}$, $R^{17}$ und $R^{18}$ unabhängig voneinander für $C_1$-$C_6$-Alkyl stehen,

$W^1$ für Wasserstoff, Halogen, Cyano, Formyl, Nitro, $C_1$-$C_6$-Alkyl, Tri-$C_1$-$C_4$-alkylsilyl, $C_1$-$C_{16}$-Alkoxy, $C_1$-$C_6$-Halogenalkyl, $C_1$-$C_6$-Halogenalkoxy, $C_2$-$C_6$-Halogenalkenyloxy, $C_1$-$C_6$-Alkylcarbonyl, $C_1$-$C_{16}$-Alkoxycarbonyl, Pentafluorthio oder -$S(O)_oR^3$ steht,

$W^2$ für Halogen, Cyano, Formyl, Nitro, $C_1$-$C_6$-Alkyl, Tri-$C_1$-$C_4$-alkylsilyl, $C_1$-$C_6$-Alkoxy, $C_1$-$C_6$-Halogenalkyl, $C_1$-$C_6$-Halogenalkoxy, $C_1$-$C_6$-Alkylcarbonyl, $C_1$-$C_{16}$-Alkoxycarbonyl, Pentafluorthio, -$S(O)_oR^3$ oder -$C(R^{11})$=N-$R^{15}$ steht,

$W^3$ für Halogen, Cyano, Nitro, $C_1$-$C_4$-Alkyl, $C_1$-$C_4$-Alkoxy, $C_1$-$C_4$-Halogenalkyl, $C_1$-$C_4$-Halogenalkoxy, Di-$C_1$-$C_4$-alkylamino, -$S(O)_oR^3$, -$COOR^{19}$ oder -$CONR^{20}R^{21}$ steht,

$R^{19}$ für Wasserstoff, $C_1$-$C_4$-Alkyl, $C_1$-$C_4$-Halogenalkyl, gegebenenfalls durch Halogen, $C_1$-$C_4$-Alkyl oder $C_1$-$C_4$-Halogenalkyl substituiertes $C_3$-$C_7$-Cycloalkyl oder für gegebenenfalls einfach bis fünffach durch Reste aus der Liste $W^4$ substituiertes Phenyl steht,

$R^{20}$ und $R^{21}$ unabhängig voneinander für Wasserstoff, $C_1$-$C_4$-Alkyl, $C_3$-$C_6$-Alkenyl, $C_1$-$C_4$-Halogenalkyl, $C_3$-$C_6$-Halogenalkenyl, $C_1$-$C_4$-Alkoxy, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl oder für jeweils gegebenenfalls einfach bis fünffach durch Reste aus der Liste $W^4$ substituiertes Phenyl oder Phenyl-$C_1$-$C_4$-alkyl, für -$OR^{16}$ oder -$NR^{17}R^{18}$ oder gemeinsam für eine Alkylenkette mit 4 bis 6 Gliedern, in welcher gegebenenfalls eine Methylengruppe durch Sauerstoff ersetzt ist, stehen, und

$W^4$ für Halogen, Cyano, Nitro, $C_1$-$C_6$-Alkyl, $C_1$-$C_6$-Alkoxy, $C_1$-$C_6$-Halogenalkyl, $C_1$-$C_6$-Halogenalkoxy, Di-$C_1$-$C_4$-alkylamino, $C_1$-$C_6$-Alkoxycarbonyl, Di-$C_1$-$C_6$-alkylaminocarbonyl oder -$S(O)_oR^3$ steht.

**2.** Verbindungen der Formel (I) gemäß Anspruch 1, in welcher

A für jeweils gegebenenfalls einfach bis vierfach durch Reste aus der Liste $W^1$ substituiertes Phenyl, Naphthyl oder Tetrahydronaphthyl oder für jeweils gegebenenfalls einfach bis vierfach durch Reste aus der Liste $W^2$ substituiertes Furyl, Benzofuryl, Thienyl, Benzothienyl, Oxazolyl, Benzoxazolyl, Thiazolyl, Benzothiazolyl, Pyrrolyl, Pyridyl, Pyrimidyl, 1,3,5-Triazinyl, Quinolinyl, Isoquinolinyl, Indolyl, Purinyl, Benzodioxolyl, Indanyl, Benzodioxanyl oder Chromanyl steht,

D für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, jeweils gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl oder $C_3$-$C_8$-Cycloalkyl-$C_1$-$C_6$-alkyl, für jeweils gegebenenfalls durch Halogen oder $C_1$-$C_4$-Alkyl substituiertes $C_5$-$C_8$-Cycloalkenyl oder $C_5$-$C_8$-Cycloalkenyl-$C_1$-$C_4$-alkyl, für jeweils gegebenenfalls durch Nitro, Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl-$C_1$-$C_6$-alkyl, Naphthyl-$C_1$-$C_6$-alkyl, Tetrahydronaphthyl-$C_1$-$C_6$-alkyl, Furylmethyl, Thienylmethyl, Pyrrolylmethyl, Oxazolylmethyl, Isoxazolylmethyl, Thiazolylmethyl oder Pyridylmethyl, für -CO-$R^6$, -CO- $NR^7R^8$ oder für die Gruppierung

$$-(CH_2)_p-(CR^9R^{10})_q-(CH_2)_r-G$$

steht,

E für Wasserstoff, $C_1$-$C_{16}$-Alkyl, $C_2$-$C_{16}$-Alkenyl, $C_2$-$C_6$-Alkinyl, $C_1$-$C_{16}$-Halogenalkyl, $C_2$-$C_{16}$-Halogenalkenyl, gegebenenfalls durch Halogen, $C_1$-$C_4$-Alkyl, $C_2$-$C_4$-Alkenyl, $C_2$-$C_4$-Halogenalkenyl, Phenyl, Styryl, Halogenphenyl oder Halogenstyryl substituiertes $C_3$-$C_8$-Cycloalkyl, für gegebenenfalls durch Halogen oder $C_1$-$C_4$-Alkyl substituiertes $C_5$-$C_8$-Cycloalkenyl, für gegebenenfalls einfach bis vierfach durch Reste aus der Liste $W^1$ substituiertes Phenyl oder für gegebenenfalls einfach bis vierfach durch Reste aus der Liste $W^2$ substituiertes Furyl, Thienyl, Pyrrolyl, Oxazolyl, Isoxazolyl, Thiazolyl oder Pyridyl oder für die Gruppierung

$$-(CH_2)_p-(CR^9R^{10})_q-(CH_2)_r-G$$

steht,

G für Cyano, für gegebenenfalls einfach bis dreifach durch Halogen, $C_1$-$C_4$-Alkyl oder $C_1$-$C_4$-Halogenalkyl und gegebenenfalls an der Verknüpfungsstelle durch den Rest $R^{11}$ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl oder eine der folgenden Gruppierungen:

(a)

$-CO-R^{11}$

(b)

$-CO-OR^{12}$

(c)

$-CO-NR^{13}R^{14}$

(d)

$-CS-NR^{13}R^{14}$

(e)

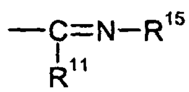

(f)

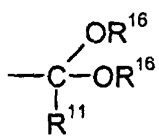

(g)

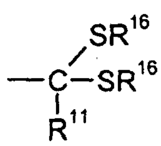

(h)

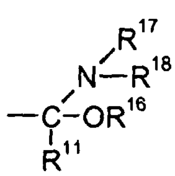

(i)

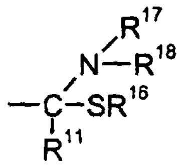

(j)

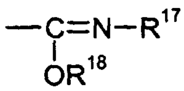

(k)

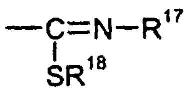

steht,

$R^{12}$ für Wasserstoff, $C_1$-$C_4$-Alkyl, $C_2$-$C_6$-Alkenyl, $C_1$-$C_4$-Halogenalkyl, $C_2$-$C_6$-Halogenalkenyl, jeweils gegebenenfalls durch Halogen, $C_1$-$C_4$-Alkyl oder $C_1$-$C_4$-Halogenalkyl substituiertes $C_3$-$C_6$-Cycloalkyl oder $C_3$-$C_6$-Cycloalkyl-$C_1$-$C_4$-alkyl oder für jeweils gegebenenfalls einfach bis vierfach durch Reste aus der Liste $W^3$ substituiertes Phenyl-$C_1$-$C_4$-alkyl oder Naphthyl-$C_1$-$C_4$-alkyl steht.

**3.** Verbindungen der Formel (I) gemäß Anspruch 1 oder 2, in welcher

m für 0, 1, 2 oder 3 steht.

**4.** Verbindungen der Formel (I) gemäß Anspruch 1, in welcher

Ar für den Rest

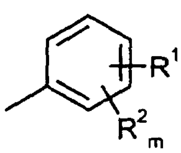

steht,

m für 0, 1 oder 2 steht,

$R^1$ für einen Substituenten in meta- oder para-Position aus der Reihe Wasserstoff, Fluor, Chlor, Brom, Cyano, Tri-($C_1$-$C_6$-alkyl)-silyl, -CO-$NR^4R^5$, Tetrahydropyranyl oder eine der folgenden Gruppierungen

(l) -X-A

(m) -B-Z-D

(n) -Y-E

steht,

$R^2$ für Wasserstoff, Fluor, Chlor, Brom, Iod, Cyano, Nitro, $C_1$-$C_{16}$-Alkyl, $C_1$-$C_{16}$-Alkoxy, jeweils durch Fluor oder Chlor substituiertes $C_1$-$C_6$-Alkyl oder $C_1$-$C_6$-Alkoxy, für $C_1$-$C_8$-Alkoxy-$C_1$-$C_8$-alkoxy oder -$S(O)_oR^3$,
mit der Maßgabe, dass $R^1$ nicht für Wasserstoff steht, wenn m = 1 und $R^2$ = Iod, steht,

o für 0, 1 oder 2 steht,

$R^3$ für $C_1$-$C_4$-Alkyl oder jeweils durch Fluor oder Chlor substituiertes Methyl oder Ethyl steht,

$R^4$ und $R^5$ unabhängig voneinander für Wasserstoff, $C_1$-$C_6$-Alkyl, durch Fluor oder Chlor substituiertes $C_1$-$C_6$-Alkyl oder für jeweils gegebenenfalls einfach oder zweifach durch Reste aus der Liste $W^1$ substituiertes Phenyl oder Benzyl stehen,

X für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder Di-$C_1$-$C_4$-alkylsilylen steht,

A für jeweils gegebenenfalls einfach bis dreifach durch Reste aus der Liste $W^1$ substituiertes Phenyl, Naphthyl oder Tetrahydronaphthyl oder für jeweils gegebenenfalls einfach bis dreifach durch Reste aus der Liste $W^2$ substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, steht,

B für gegebenenfalls einfach oder zweifach durch Reste aus der Liste W' substituiertes p-Phenylen steht,

Z für Sauerstoff oder Schwefel steht,

D für Wasserstoff, $C_1$-$C_{16}$-Alkyl, $C_2$-$C_{16}$-Alkenyl, $C_2$-$C_6$-Alkinyl, jeweils durch Fluor oder Chlor substituiertes $C_1$-$C_4$-Alkyl oder $C_2$-$C_4$-Alkenyl, für jeweils gegebenenfalls durch Fluor, Chlor, Brom, $C_1$-$C_4$-Alkyl, $C_2$-$C_4$-Alkenyl, durch Fluor oder Chlor substituiertes $C_2$-$C_4$-Alkenyl, durch Phenyl, Styryl, jeweils durch Fluor, Chlor oder Brom substituiertes Phenyl oder Styryl substituiertes $C_3$-$C_6$-Cycloalkyl oder $C_3$-$C_6$-Cycloalkyl-$C_1$-$C_4$-alkyl, für jeweils gegebenenfalls durch Fluor, Chlor, Brom oder $C_1$-$C_4$-Alkyl, substituiertes $C_5$-$C_6$-Cycloalkenyl oder $C_5$-$C_6$-Cycloalkenyl-$C_1$-$C_4$-alkyl, für jeweils gegebenenfalls durch Nitro, Fluor, Chlor, Brom, $C_1$-$C_6$-Alkyl, $C_1$-$C_6$-Alkoxy, jeweils durch Fluor oder Chlor substituiertes $C_1$-$C_4$-Alkyl oder $C_1$-$C_4$-Alkoxy substituiertes Phenyl-$C_1$-$C_4$-alkyl, Naphthyl-$C_1$-$C_4$-alkyl, Tetrahydronaphthyl-$C_1$-$C_6$-alkyl oder 5- oder 6-ringgliedriges Hetaryl-$C_1$-$C_4$-alkyl mit 1 oder 2 Heteroatomen aus der Reihe Stickstoff, Sauerstoff und Schwefel, für -CO-$R^6$, -CO-$NR^7R^8$ oder die Gruppierung

$$-(CH_2)_p-(CR^9R^{10})q-(CH_2)_r-G$$

steht,

Z und D können auch gemeinsam für gegebenenfalls durch Nitro, Fluor, Chlor, Brom, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy substituiertes Phenoxy-$C_1$-$C_3$-alkyl stehen,

Y für eine direkte Bindung, Sauerstoff, Schwefel, Carbonyl, Carbonyloxy, Oxycarbonyl, $C_1$-$C_4$-Alkylen, $C_2$-$C_4$-Alkenylen, $C_2$-$C_4$-Alkinylen, $C_1$-$C_4$-Alkylenoxy, $C_1$-$C_4$-Oxyalkylen, $C_1$-$C_4$-Thioalkylen, $C_1$-$C_4$-Alkylendioxy oder für gegebenenfalls einfach oder zweifach durch Reste aus der Liste $W^1$ substituiertes p-Phenylen steht,

E für Wasserstoff, $C_1$-$C_{16}$-Alkyl, $C_2$-$C_{16}$-Alkenyl, $C_2$-$C_6$-Alkinyl, jeweils durch Fluor oder Chlor substituiertes $C_1$-$C_4$-Alkyl oder $C_2$-$C_4$-Alkenyl, für jeweils durch Fluor, Chlor, Brom, $C_1$-$C_4$-Alkyl, $C_2$-$C_4$-Alkenyl, durch Fluor oder Chlor substituiertes $C_2$-$C_4$-Alkenyl, durch Phenyl, Styryl, jeweils durch Fluor, Chlor oder Brom substituiertes Phenyl oder Styryl substituiertes $C_3$-$C_6$-Cycloalkyl, für jeweils durch Fluor, Chlor, Brom oder $C_1$-$C_4$-Alkyl, substituiertes $C_5$-$C_6$-Cycloalkenyl, für gegebenenfalls einfach bis dreifach durch Reste aus der Liste $W^1$ substituiertes Phenyl oder für jeweils gegebenenfalls einfach oder zweifach durch Reste aus der Liste $W^2$ substituiertes 5- oder 6-gliedriges Hetaryl mit 1 oder 2 Heteroatomen aus der Reihe Stickstoff, Sauerstoff und Schwefel oder für die Gruppierung

$$-(CH_2)_p-(CR^9R^{10})_q-(CH_2)_r-G$$

steht,

$R^6$ für $C_1$-$C_6$-Alkyl, $C_1$-$C_6$-Alkoxy, $C_2$-$C_6$-Alkenyl, $C_1$-$C_6$-Alkenyloxy, jeweils gegebenenfalls

durch Fluor, Chlor, $C_1$-$C_3$-Alkyl oder jeweils durch Fluor oder Chlor substituiertes $C_1$-$C_2$-Alkyl oder $C_2$-$C_3$-Alkenyl substituiertes $C_3$-$C_6$-Cycloalkyl, $C_3$-$C_6$-Cycloalkyloxy oder $C_3$-$C_6$-Cycloalkyl-$C_1$-$C_2$-alkyloxy oder für gegebenenfalls einfach oder zweifach durch Fluor, Chlor, Brom, Iod, $C_1$-$C_4$-Alkyl, $C_1$-$C_4$-Alkoxy oder jeweils durch Fluor oder Chlor substituiertes $C_1$-$C_3$-Alkyl oder $C_1$-$C_4$-Alkoxy substituiertes Phenyl steht,

$R^7$ für Wasserstoff oder $C_1$-$C_4$-Alkyl steht,

$R^8$ für $C_1$-$C_4$-Alkyl oder für jeweils gegebenenfalls einfach oder zweifach durch Fluor, Chlor, Brom, $C_1$-$C_4$-Alkyl, oder jeweils durch Fluor oder Chlor substituiertes $C_1$-$C_4$-Alkyl oder $C_1$-$C_4$-Alkoxy substituiertes Phenyl oder Benzyl steht,

p, q und r unabhängig voneinander für 0, 1, 2 oder 3 stehen, wobei deren Summe kleiner als 6 und größer als 1 ist,

$R^9$ und $R^{10}$ unabhängig voneinander für Wasserstoff oder $C_1$-$C_4$-Alkyl stehen,

G für Cyano, für einen gegebenenfalls einfach bis dreifach durch Fluor, Chlor, Brom, $C_1$-$C_4$-Alkyl oder durch Fluor oder Chlor substituiertes $C_1$-$C_4$-Alkyl und gegebenenfalls an der Verknüpfungsstelle durch den Rest $R^{11}$ substituierten 5- oder 6- gliedrigen Heterocyclus mit 1 bis 3 gleichen oder verschiedenen Heteroatomen aus der Reihe Stickstoff, Sauerstoff und Schwefel oder eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

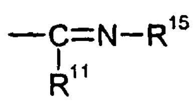

(f)

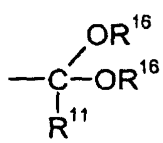

(g)

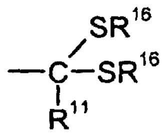

(h)

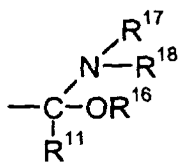

(i)

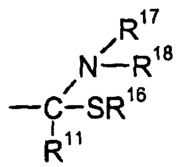

(j)

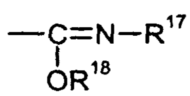

(k)

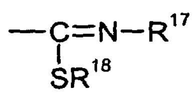

steht,

$R^{11}$ für Wasserstoff, $C_1$-$C_6$-Alkyl, $C_2$-$C_6$-Alkenyl, jeweils durch Fluor oder Chlor substituiertes $C_1$-$C_4$-Alkyl oder $C_2$-$C_6$-Alkenyl, gegebenenfalls durch Fluor, Chlor, $C_1$-$C_4$-Alkyl oder durch Fluor oder Chlor subsituiertes $C_1$-$C_4$-Alkyl subsituiertes $C_3$-$C_6$-Cycloalkyl oder für gegebenenfalls einfach bis dreifach durch $C_1$-$C_4$-Alkylcarbonylamino, $C_1$-$C_4$-Alkylcarbonyl-$C_1$-$C_4$-alkylamino und/oder Reste aus der Liste $W^3$ substituiertes Phenyl steht,

$R^{12}$ für Wasserstoff, $C_1$-$C_4$-Alkyl, $C_3$-$C_6$-Alkenyl, jeweils durch Fluor oder Chlor substituiertes $C_1$-$C_4$-Alkyl oder $C_2$-$C_6$-Alkenyl, jeweils gegebenenfalls durch Fluor, Chlor, $C_1$-$C_4$-Alkyl oder durch Fluor oder Chlor subsituiertes $C_1$-$C_4$-Alkyl substituiertes $C_3$-$C_6$-Cycloalkyl oder $C_3$-$C_6$-Cycloalkyl-$C_1$-$C_4$-alkyl, oder für jeweils gegebenenfalls einfach bis dreifach durch Reste aus der Liste $W^3$ substituiertes Phenyl-$C_1$-$C_4$-alkyl oder Naphthyl-$C_1$-$C_4$-alkyl steht,

$R^{13}$ und $R^{14}$ unabhängig voneinander für Wasserstoff, $C_1$-$C_4$-Alkyl, $C_3$-$C_6$-Alkenyl, jeweils durch Fluor oder Chlor substituiertes $C_1$-$C_4$-Alkyl oder $C_3$-$C_6$-Alkenyl, für $C_1$-$C_4$-Alkoxy, jeweils gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder $C_3$-$C_6$-Cycloalkyl-$C_1$-$C_4$-alkyl, für jeweils gegebenenfalls einfach bis dreifach durch Reste aus der Liste $W^3$ substituiertes Phenyl oder Phenyl-$C_1$-$C_4$-alkyl, für $-OR^{12}$ oder $-NR^{11}R^{12}$ oder gemeinsam für $-(CH_2)_5-$, $-(CH_2)_6-$ oder $-(CH_2)_2-O-(CH_2)_2-$ stehen,

$R^{15}$ für $-OR^{12}$, $-NR^{11}R^{12}$ oder $-N(R^{11})-COOR^{12}$ steht,

$R^{16}$, $R^{17}$ und $R^{18}$ unabhängig voneinander für $C_1$-$C_4$-Alkyl stehen,

$W^1$ für Wasserstoff, Fluor, Chlor, Brom, Iod, Cyano, Formyl, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für $C_1$-$C_4$-Alkylcarbonyl, $C_1$-$C_4$-Alkoxycarbonyl oder $-S(O)_oR^3$ steht,

$W^2$ für Fluor, Chlor, Brom, Cyano, Formyl, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für $C_1$-$C_4$-Alkylcarbonyl, $C_1$-$C_4$-Alkoxycarbonyl, $-S(O)_oR^3$ oder $-C(R^{11})=N-R^{15}$ steht,

$W^3$ für Fluor, Chlor, Brom, Cyano, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, für Di-$C_1$-$C_4$-alkylamino, $-S(O)_oR^3$, $-COOR^{19}$ oder $-CONR^{20}R^{21}$ steht,

$R^{19}$ für Wasserstoff, $C_1$-$C_4$-Alkyl, durch Fluor oder Chlor substituiertes $C_1$-$C_4$-Alkyl, gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder für gegebenenfalls einfach bis dreifach durch Reste aus der Liste $W^4$ substituiertes Phenyl steht,

$R^{20}$ und $R^{21}$ unabhängig voneinander für Wasserstoff, $C_1$-$C_4$-Alkyl, $C_3$-$C_6$-Alkenyl, jeweils durch Fluor oder Chlor substituiertes $C_1$-$C_4$-Alkyl oder $C_3$-$C_6$-Alkenyl, für $C_1$-$C_4$-Alkoxy, jeweils gegebenenfalls durch Fluor, Chlor, $C_1$-$C_4$-Alkyl oder durch Fluor oder Chlor substituiertes $C_1$-$C_4$-Alkyl substituiertes $C_3$-$C_6$-Cycloalkyl oder $C_3$-$C_6$-Cycloalkyl-$C_1$-$C_4$-alkyl oder für jeweils gegebenenfalls einfach bis dreifach durch Reste aus der Liste $W^4$ substituiertes Phenyl oder Phenyl-$C_1$-$C_4$-alkyl, für $-OR^{16}$ oder $-NR^{17}R^{18}$ oder gemeinsam für $-(CH_2)_5-$, $-(CH_2)_6-$ oder $-(CH_2)_2O-(CH_2)_2$-stehen, und

$W^4$ für Fluor, Chlor, Brom, Cyano, Nitro, $C_1$-$C_4$-Alkyl, $C_1$-$C_4$-Alkoxy, jeweils durch Fluor oder Chlor substituiertes $C_1$-$C_4$-Alkyl oder $C_1$-$C_4$-Alkoxy, Di-$C_1$-$C_4$-alkylamino, $C_1$-$C_4$-Alkoxycarbonyl, Di-$C_1$-$C_6$-alkylaminocarbonyl oder $-S(O)_oR^3$ steht.

**5.** Verbindungen der Formel (I) gemäß Anspruch 4, in welcher

A für jeweils gegebenenfalls einfach bis dreifach durch Reste aus der Liste $W^1$ substituiertes Phenyl, Naphthyl oder Tetrahydronaphthyl oder für jeweils gegebenenfalls einfach bis dreifach durch Reste aus der Liste $W^2$ substituiertes Furyl, Benzofuryl, Thienyl, Benzothienyl, Oxazolyl, Benzoxazolyl, Thiazolyl, Benzothiazolyl, Pyrrolyl, Pyridyl, Pyrimidyl, 1,3,5-Triazinyl, Quinolinyl, Isoquinolinyl, Indolyl, Purinyl, Benzodioxolyl, Indanyl, Benzodioxanyl oder Chromanyl steht,

D für Wasserstoff, $C_1$-$C_{16}$-Alkyl, $C_2$-$C_{16}$-Alkenyl, $C_2$-$C_6$-Alkinyl, jeweils durch Fluor oder Chlor substituiertes $C_1$-$C_4$-Alkyl oder $C_2$-$C_4$-Alkenyl, für jeweils gegebenenfalls durch Fluor, Chlor, Brom, $C_1$-$C_4$-Alkyl, $C_2$-$C_4$-Alkenyl, durch Fluor oder Chlor substituiertes $C_2$-$C_4$-Alkenyl, durch Phenyl, Styryl, jeweils durch Fluor, Chlor oder Brom substituiertes Phenyl oder Styryl substituiertes $C_3$-$C_6$-Cycloalkyl oder $C_3$-$C_6$-Cycloalkyl-$C_1$-$C_4$-alkyl, für jeweils gegebenenfalls durch Fluor, Chlor, Brom oder $C_1$-$C_4$-Alkyl, substituiertes $C_5$-$C_6$-Cycloalkenyl oder $C_5$-$C_6$-Cycloalkenyl-$C_1$-$C_4$-alkyl, für jeweils gegebenenfalls durch Nitro, Fluor, Chlor, Brom, $C_1$-$C_6$-Alkyl, $C_1$-$C_6$-Alkoxy, jeweils durch Fluor oder Chlor substituiertes $C_1$-$C_4$-Alkyl oder $C_1$-$C_4$-Alkoxy substituiertes Phenyl-$C_1$-$C_4$-alkyl, Naphthyl-$C_1$-$C_4$-alkyl, Tetrahydronaphthyl-$C_1$-$C_6$-alkyl Furylmethyl, Thienylmethyl, Pyrrolylmethyl, Oxazolylmethyl, Isoxazolylmethyl, Thiazolylmethyl oder Pyridylmethyl, für $-CO-R^6$, $-CO-NR^7R^8$ oder die Gruppierung

$$-(CH_2)_p-(CR^9R^{10})_q-(CH_2)_r-G$$

steht,

E für Wasserstoff, $C_1$-$C_{16}$-Alkyl, $C_2$-$C_{16}$-Alkenyl, $C_2$-$C_6$-Alkinyl, jeweils durch Fluor oder Chlor substituiertes $C_1$-$C_4$-Alkyl oder $C_2$-$C_4$-Alkenyl, für jeweils durch Fluor, Chlor, Brom, $C_1$-$C_4$-Alkyl, $C_2$-$C_4$-Alkenyl, durch Fluor oder Chlor substituiertes $C_2$-$C_4$-Alkenyl, durch Phenyl, Styryl, jeweils durch Fluor, Chlor oder Brom substituiertes Phenyl oder Styryl substituiertes $C_3$-$C_6$-Cycloalkyl, für jeweils durch Fluor, Chlor, Brom oder $C_1$-$C_4$-Alkyl, substituiertes $C_5$-$C_6$-Cycloalkenyl, für gegebenenfalls einfach bis dreifach durch Reste aus der Liste W' substituiertes Phenyl oder für jeweils gegebenenfalls einfach oder zweifach durch Reste aus der Liste $W^2$ substituiertes Furyl, Thienyl, Pyrrolyl, Oxazolyl, Isoxazolyl, Thiazolyl oder Pyridyl oder für die Gruppierung

$$-(CH_2)_p-(CR^9R^{10})_q-(CH_2)_r-G$$

steht,

G für Cyano, für gegebenenfalls einfach bis dreifach durch Fluor, Chlor, Brom, $C_1$-$C_4$-Alkyl oder durch Fluor oder Chlor substituiertes $C_1$-$C_4$-Alkyl und gegebenenfalls an der Verknüpfungsstelle durch den Rest $R^{11}$ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl oder eine der folgenden Gruppierungen;

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

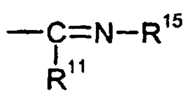

(f)

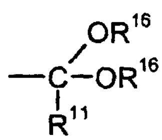

(g)

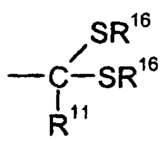

(h)

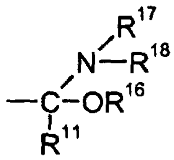

(i)

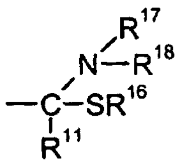

(j)

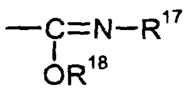

(k)

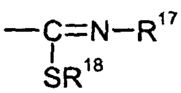

steht.

**6.** Verbindungen der Formel (I) gemäß Anspruch 1, in welcher

Ar für den Rest

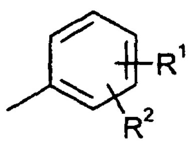

steht,

$R^1$ für einen Substituenten in meta- oder para-Position aus der Reihe Wasserstoff, Fluor, Chlor, Brom, Cyano, -CO-$NR^4R^5$, Tetrahydropyranyl oder eine der folgenden Gruppierungen

(l) -X-A

(m-a) 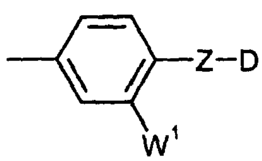

(n) -Y-E

steht,

$R^2$ für Wasserstoff, Fluor, Chlor, Brom, Iod, Methyl, Ethyl, Methoxy, Ethoxy, Methylthio, Ethylthio, Trifluormethyl, Difluormethoxy, Trifluormethoxy oder Trifluormethylthio steht, mit der Maßgabe, dass $R^1$ nicht für Wasserstoff steht, wenn m = 1 und $R^2$ = Iod, steht,

o für 0 oder 2 steht,

$R^3$ für Methyl, Ethyl, N-Propyl, Isopropyl, Difluormethyl oder Trifluormethyl steht,

$R^4$ und $R^5$ unabhängig voneinander für Wasserstoff, Methyl, Ethyl, n-Propyl, Isopropyl, n-Butyl, Isobutyl, sec-Butyl, *tert*-Butyl oder für jeweils gegebenenfalls einfach durch einen Rest aus der Liste $W^1$ substituiertes Phenyl oder Benzyl stehen,

X für eine direkte Bindung, Sauerstoff, Schwefel. Carbonyl, $-CH_2-$, $-(CH_2)_2-$, -CH=CH- (E oder Z), -C≡C-, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, $C_1$-$C_4$-Alkylendioxy, insbesondere $-OCH_2O-$, $-O(CH_2)_2O-$ oder $-OCH(CH_3)O-$ steht,

A für gegebenenfalls einfach oder zweifach durch Reste aus der Liste $W^1$ substituiertes Phenyl oder für jeweils gegebenenfalls einfach oder zweifach durch Reste aus der Liste $W^2$ substituiertes Furyl, Benzofuryl, Thienyl, Benzothienyl, Oxazolyl, Benzoxazolyl, Thiazolyl, Benzothiazolyl, Pyrrolyl, Pyridyl, Pyrimidyl, 1,3,5-Triazinyl, Quinolinyl, Isoquinolinyl, Indolyl, Purinyl, Benzodioxolyl, Indanyl, Benzodioxanyl oder Chromanyl, steht,

Z für Sauerstoff oder Schwefel steht,

D für Wasserstoff, Methyl, Ethyl, n-Propyl, Isopropyl, n-Butyl, Isobutyl, sec-Butyl, tert-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, für jeweils gegebenenfalls einfach bis dreifach durch Fluor, Chlor, Brom, Methyl, Ethyl, N-Propyl, Isopropyl, n-Butyl, Isobutyl, sec-Butyl, tert-Butyl, Ethenyl, 1-Propenyl, 2,2-Dimethylethenyl, $-CH=CCl_2$, Phenyl, Styryl, jeweils durch Fluor, Chlor oder Brom substituiertes Phenyl oder 4-Chlorstyryl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor, Chlor, Methyl, Ethyl, n-Propyl, Isopropyl, n-Butyl, Isobutyl, sec-Butyl oder tert-Butyl substituiertes Cyclopentenyl, Cyclohexenyl, Cyclohexenylmethyl oder Cyclopentenylmethyl, für jeweils gegebenenfalls einfach oder zweifach durch Nitro, Fluor, Chlor, Brom, Methyl, Ethyl, n-Propyl, Isopropyl, n-Butyl, Isobutyl, sec-Butyl, tert-Butyl, Methoxy, Ethoxy, n-Propoxy, Isopropoxy, n-Butoxy, Isobutoxy, sec-Butoxy, tert-Butoxy, Trifluormethyl, Trifluormethoxy, Difluormethoxy oder Chlordifluormethoxy substituiertes Benzyl, Phenethyl, Naphthylmethyl, Tetrahydronaphthylmethyl, Furylmethyl, Thienylmethyl, Pyrrolylmethyl, Oxazolylmethyl, Isoxazolylmethyl, Thiazolylmethyl oder Pyridylmethyl, für $-CO-R^6$, $-CO-NR^7R^8$ oder die Gruppierung

$$-(CH_2)_p-(CR^9R^{10})_q-(CH_2)_r-G$$

steht,

Z und D können auch gemeinsam für gegebenenfalls einfach oder zweifach durch Nitro, Fluor, Chlor, Brom, Methyl, Ethyl, n-Propyl, 1-Propyl, Methoxy, Ethoxy, n-Propoxy, i-Propoxy, Trifluormethyl, Trifluormethoxy, Difluormethoxy oder Chlordifluormethoxy substituiertes Phenoxymethyl stehen,

Y für eine direkte Bindung, Sauerstoff, Schwefel, Carbonyl, $-CH_2-$, $-(CH_2)_2-$, -CH=CH- (E oder Z), -C≡C-, $-CH_2O-$,$-(CH_2)_2O-$, -CH(CH3)O-, $-OCH_2-$, $-O(CH_2)_2-$, $-SCH_2-$, $-S(CH_2)_2-$, $-SCH(CH_3)-$, $C_1$-$C_4$-Alkylendioxy, insbesondere $-OCH_2O-$ oder $-O(CH_2)_2O-$ oder für gegebenenfalls einfach durch Reste aus der Liste $W^1$ substituiertes p-Phenylen steht,

E für Wasserstoff, Methyl, Ethyl, n-Propyl, Isopropyl, n-Butyl, Isobutyl, sec-Butyl, tert-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, für

jeweils gegebenenfalls einfach bis dreifach durch Fluor, Chlor, Brom, Methyl, Ethyl, n-Propyl, Isopropyl, n-Butyl, Isobutyl, sec-Butyl, tert-Butyl, Ethenyl, 1-Propenyl, 2,2-Dimethylethenyl, $-CH=CCl_2$, Phenyl, Styryl, jeweils durch Fluor, Chlor oder Brom substituiertes Phenyl oder durch 4-Chlorstyryl substituiertes Cyclopropyl, Cyclobutyl, Cyclopentyl oder Cyclohexyl, für jeweils gegebenenfalls durch Fluor, Chlor, Methyl, Ethyl, n-Propyl, Isopropyl, n-Butyl, Isobutyl, sec-Butyl oder tert-Butyl substituiertes Cyclopentenyl oder Cyclohexenyl, für gegebenenfalls einfach oder zweifach durch Reste aus der Liste $W^1$ substituiertes Phenyl, für jeweils gegebenenfalls einfach oder zweifach durch Reste aus der Liste $W^2$ substituiertes Furyl, Thienyl, Pyrrolyl, Oxazolyl, Isoxazolyl, Thiazolyl oder Pyridyl, oder für dir Gruppierung

$$-(CH_2)p-(CR^9R^{10}q-(CH_2)_r-G$$

steht,

$R^6$ für Methyl, Ethyl, n-Propyl, Isopropyl, n-Butyl, Isobutyl, sec-Butyl, tert-Butyl, Methoxy, Ethoxy, n-Propoxy, Isopropoxy, n-Butoxy, Isobutoxy, sec-Butoxy, tert-Butoxy, Cyclopropyl, Cyclohexyl, Cyclohexyloxy, Cyclohexylmethyloxy, Phenyl, 2-Chlorphenyl, 3-Chlorphenyl, 2,6-Difluorphenyl, 2,4-Dichlorphenyl, 3,4-Dichlorphenyl, 2-Trifluormethoxyphenyl oder 4-Trifluormethoxyphenyl steht,

$R^7$ für Wasserstoff steht,

$R^8$ für Methyl, Ethyl oder gegebenenfalls einfach durch Chlor substituiertes Phenyl steht,

p, q und r unabhängig voneinander für 0, 1, 2 oder 3 stehen, wobei deren Summe kleiner als 4 und größer als 1 ist,

$R^9$ und $R^{10}$ unabhängig voneinander für Wasserstoff, Methyl, Ethyl, n-Propyl, Isopropyl, n-Butyl, Isobutyl, sec-Butyl, tert-Butyl stehen,

G für Cyano, für jeweils gegebenenfalls einfach bis dreifach durch Fluor, Chlor, Brom, Methyl, Ethyl, n-Propyl, Isopropyl oder Trifluormethyl und gegebenenfalls an der Verknüpfungsstelle durch den Rest R" substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl, oder für eine der folgenden Gruppierungen:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

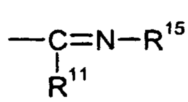

(f)

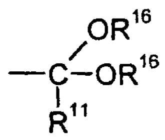

(g)

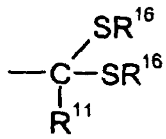

(h)

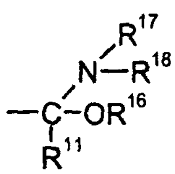

(i)

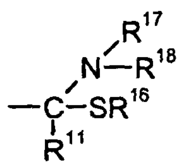

steht,

$R^{11}$ für Wasserstoff, Methyl, Ethyl, n-Propyl, Isopropyl, n-Butyl, Isobutyl, sec-Butyl, tert-Butyl, die isomeren Pentyle, die isomeren Hexyle, $-CF_3$, $-CHF_2$,$-CClF_2$, $-CF_2CHFCl$, $-CF_2CH_2F$, $-CF_2CHF_2$, $-CF_2CCl_3$, $-CH_2CF_3$, $C_3$-$C_6$-Alkenyl, einfach bis dreifach durch Fluor oder Chlor substituiertes $C_3$-$C_6$-Alkenyl, für jeweils gegebenenfalls einfach oder zweifach durch Fluor, Chlor, Methyl, Ethyl, n-Propyl, Isopropyl, $-CF_3$, $-CHF_2$, $-CClF_2$, $-CF_2CHFCl$, $-CF_2CH_2F$, $-CF_2CHF_2$, $-CF_2CCl_3$ oder $-CH_2CF_3$ substituiertes Cyclopropyl, Cyclopentyl oder Cyclohexyl oder für gegebenenfalls einfach oder zweifach durch Methylcarbonylamino, Ethylcarbonylamino, Methylcarbonyl-methylamino und/oder Reste aus der Liste $W^3$ substituiertes Phenyl steht,

$R^{12}$ für Wasserstoff, Methyl, Ethyl, n-Propyl, Isopropyl, n-Butyl, Isobutyl, sec-Butyl, tert-Butyl, $-CH_2CF_3$, Allyl, für jeweils gegebenenfalls einfach oder zweifach durch Fluor, Chlor, Methyl, Ethyl, n-Propyl, Isopropyl, $-CF_3$,$-CHF_2$, $-CClF_2$, $-CF_2CHFCl$, $-CF_2CH_2F$, $-CF_2CHF_2$, $-CF_2CCl_3$ oder $-CH_2CF_3$ substituiertes Cyclopropyl, Cyclopentyl, Cyclohexyl, Cyclopropylmethyl, Cyclopentylmethyl, Cyclohexylmethyl, Cyclopropylethyl, Cyclopentylethyl oder Cyclohexylethyl oder für jeweils gegebenenfalls einfach oder zweifach durch Reste aus der Liste $W^3$ substituiertes Benzyl oder Phenethyl steht,

$R^{13}$ und $R^{14}$ unabhängig voneinander für Wasserstoff, Methyl, Ethyl, n-Propyl, Isopropyl, n-Butyl, Isobutyl, sec-Butyl, tert-Butyl, $-CH_2CF_3$, Methoxy, Ethoxy, Allyl, jeweils gegebenenfalls einfach oder zweifach durch Fluor, Chlor, Methyl, Ethyl, n-Propyl, Isopropyl oder Trifluormethyl substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste $W^3$ substituiertes Phenyl, Benzyl oder Phenethyl, für $-OR^{12}$ oder $-NR^{11}R^{12}$ stehen,

$R^{15}$ für $-OR^{12}$, $-NR^{11}R^{12}$ oder $-N(R^{11})-COOR^{12}$ steht,

$R^{16}$, $R^{17}$ und $R^{18}$ unabhängig voneinander für Methyl, Ethyl, N-Propyl oder Isopropyl stehen,

$W^1$ für Wasserstoff, Fluor, Chlor, Brom, Cyano, Formyl, Nitro, Methyl, Ethyl, n-Propyl, Isopropyl, n-Butyl, Isobutyl, sec-Butyl, tert-Butyl, Methoxy, Ethoxy, n-Propoxy, Isopropoxy, n-Butoxy, Isobutoxy, sec-Butoxy, tert-Butoxy, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, Trifluormethoxy, Difluormethoxy, Chlordifluormethoxy, Acetyl, Propionyl, Butyryl, Isobutyryl, Methoxycarbonyl, Ethoxycarbonyl, n-Propoxycarbonyl, Isopropoxycarbonyl, n-Butoxycarbonyl, Isobutoxycarbonyl, sec-Butoxycarbonyl, tert-Butoxycarbonyl oder $-S(O)_oR^3$ steht,

$W^2$ für Fluor, Chlor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, $-CH=N-OCH_3$, $-CH=N-OC_2H_5$, $-CH=N-OC_3H_7$, $-C(CH_3)=N-OCH_3$, $-C(CH_3)=N-OC_2H_5$, $-C(CH_3)=N-OC_3H_7$, $-C(C_2H_5)=N-OCH_3$, $-C(C_2H_5)=N-OC_2H_5$ oder $-C(C_2H_5)=N-OC_3H_7$ steht,

$W^3$ für Fluor, Chlor, Cyano, Nitro, Methyl, Ethyl, Methoxy, Ethoxy, Methylthio, Trifluormethyl, Trifluormethoxy, Trifluormethylthio, Dimethylamino, Diethylamino, $-COOR^{19}$ oder $-CONR^{20}R^{21}$ steht,

$R^{19}$ für Wasserstoff, Methyl, Ethyl, n-Propyl, Isopropyl. tert-Butyl, $-CH_2CF_3$, für jeweils gegebenenfalls einfach oder zweifach durch Fluor, Chlor, Methyl, Ethyl, n-Propyl, Isopropyl oder $-CF_3$ substituiertes Cyclopropyl, Cyclopentyl oder Cyclohexyl, oder für gegebenenfalls einfach oder zweifach durch Reste aus der Liste $W^4$ substituiertes Phenyl steht,

$R^{20}$ und $R^{21}$ unabhängig voneinander für Wasserstoff, Methyl, Ethyl, n-Propyl, Isopropyl, n-Butyl, Isobutyl, sec-Butyl, tert-Butyl, $-CH_2CF_3$, Methoxy, Ethoxy, Allyl, für jeweils gegebenenfalls einfach oder zweifach durch Fluor oder Chlor substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste $W^4$ substituiertes Phenyl, Benzyl oder Phenethyl, für $-OR^{16}$ oder $-NR^{17}R^{18}$ steht, und

$W^4$ für Fluor, Chlor, Brom, Cyano, Nitro, Methyl, Ethyl, tert-Butyl, Methoxy, Ethoxy, Methylthio, Trifluormethyl, Trifluormethoxy oder Trifluormethylthio steht.

7. Verfahren zur Herstellung von Verbindungen der Formel (I) gemäß Anspruch 1, **dadurch gekennzeichnet, dass** man

A) Cyclische Imine der Formel (I)

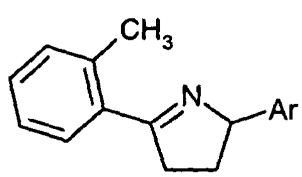

(I)

in welcher

Ar die in Anspruch 1 angegebenen Bedeutungen hat,

erhält, indem man

a) Aminoketonderivative der Formel (VIII)

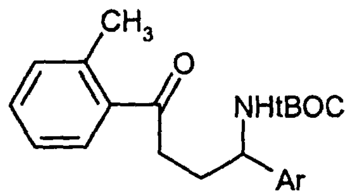

(VIII)

in welcher
Ar die in Anspruch 1 angegebenen Bedeutungen hat,
mit einer Säure umsetzt und anschließend gegebenenfalls in Gegenwart eines Säurebindemittels cyclokondensiert, oder

b) die Nitrogruppe von Nitroketonen der Formel (XVIII)

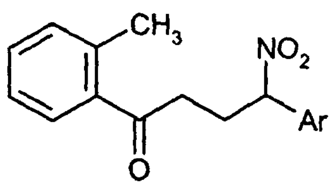

(XVIII)

in welcher
Ar die in Anspruch 1 angegebenen Bedeutungen hat, reduziert, wobei

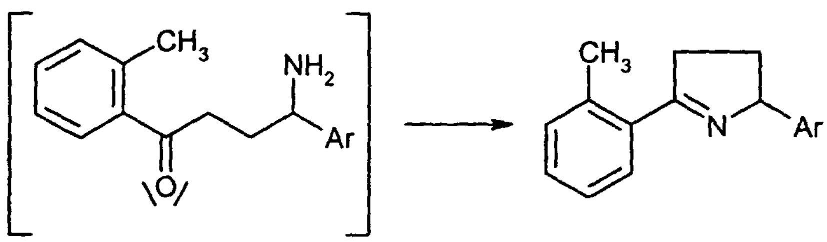

(II) (I)

intermediär ein Aminoketon der Formel (II) auftritt, welches jedoch, insbesondere in saurem Medium, in situ zu (I) cyclokondensiert wird, oder

c) Imines der Formel (XXVII)

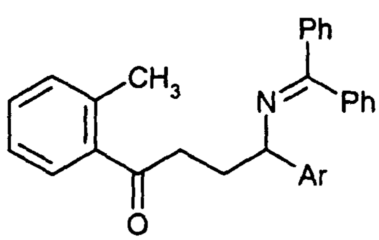

(XXVII)

in welcher

Ar die in Anspruch 1 angegebenen Bedeutungen hat, mit wässrigen Säuren hydrolysiert,

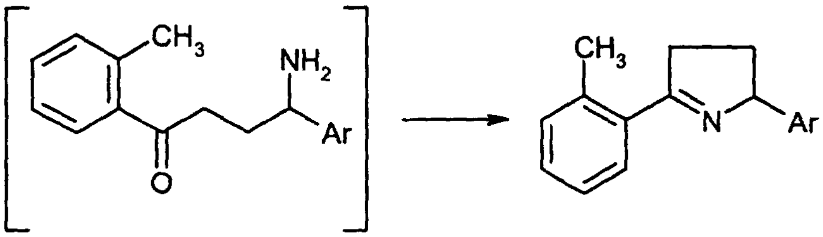

(II) (I)

wobei intermediär ein Aminoketon der Formel (II) auftritt, welches jedoch in situ zu (I) cyclokondensiert wird, oder

B) Verbindungen der Formel (III)

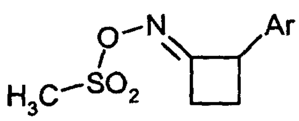

(III)

in welcher

Ar die in Anspruch 1 angegebenen Bedeutungen hat,

mit der Aryl-Grignard-Verbindung der Formel (IV)

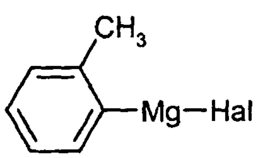

(IV)

in welcher

Hal für Chlor, Brom oder Iod steht,

in Gegenwart eines Verdünnungsmittels umsetzt, oder

C) Verbindungen der Formel (I-b)

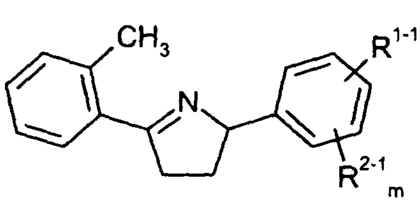

(I-b)

in welcher

m die in Anspruch 1 angegebenen Bedeutungen hat,
$R^{1-1}$ für A oder eine der folgenden Gruppierungen

(m) -B-Z-D

(n-a) 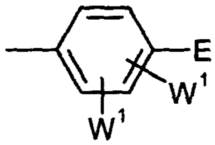

steht, wobei

A, B, D, E, W 1 und Z die in Anspruch 1 angegebenen Bedeutungen haben und

$R^{2-1}$ für Wasserstoff, Fluor, Cyano, Nitro, Alkyl, Alkoxy, Halogenalkyl, Halogenalkoxy, Alkoxyalkoxy oder $-SR^3$ steht, wobei

$R^3$ die in Anspruch 1 angegebenen Bedeutungen hat,

erhält, indem man Verbindungen der Formel (V)

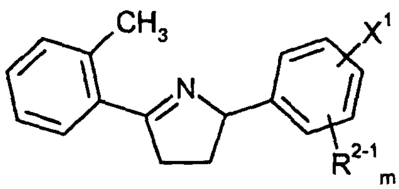

(V)

in welcher

$R^{2-1}$ die oben angegebenen Bedeutungen hat,

m die in Anspruch 1 angegebenen Bedeutungen hat, und

$X^1$ für Brom, Iod oder $-OSO_2CF_3$

mit Boronsäuren der Formel (VI)

$$R^{1-1}\text{-}B(OH)_2 \qquad (VI)$$

in welcher

$R^{1-1}$ die oben angegebenen Bedeutungen hat,

in Gegenwart eines Katalysators und in Gegenwart eines Säurebindemittels und in Gegenwart eines Lösungsmittels kuppelt, oder

D) Cyclische Imine der Formel (I-c)

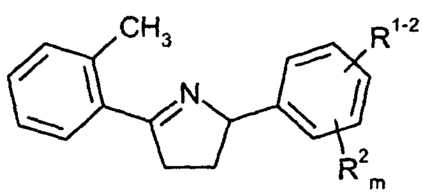

(I-c)

in welcher

$R^2$ und m die in Anspruch 1 angegebenen Bedeutungen haben,

$R^{1-2}$ für eine der folgenden Gruppierungen

(m-b) $-B-Z-D^1$

(n-b) $-Y^1-E^1$

steht, in denen

B und Z die in Anspruch 1 angegebenen Bedeutungen haben,
$Y^1$ für Sauerstoff oder Schwefel steht und
$D^1$ und $E^1$ für die Gruppierung

$$-(CH_2)p-(CR^9R^{10})_q-(CH_2)_r-G$$

stehen, in der
$R^9$, $R^{10}$, G, p, q und r die in Anspruch 1 angegebenen Bedeutungen haben,
erhält, indem man cyclische Imine der Formel (I-d)

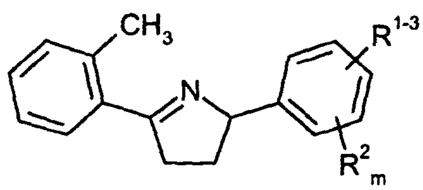

**(I-d)**

in welcher

$R^2$ und m die in Anspruch 1 angegebenen Bedeutungen haben und
$R^{1-3}$ für eine der folgenden Gruppierungen

(m-c) -B-Z-H

(n-c) $-Y^1-H$

steht, in denen
B und Z die in Anspruch 1 angegebenen Bedeutungen haben und
$Y^1$ die oben angegebenen Bedeutungen hat,
mit Verbindungen der Formel (VII)

$$Ab-(CH_2)_p-(CR^9R^{10})_q-(CH_2)_r-G \quad (VII)$$

in welcher

$R^9$, $R^{10}$, G, p, q und r die in Anspruch 1 angegebenen Bedeutungen haben und
Ab für a Abgangsgruppe steht,

kondensiert, oder

E) Cyclische Imine der Formel (I-e)

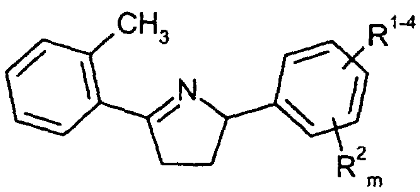

(I-e)

in welcher

| | |
|---|---|
| $R^2$ und m | die in Anspruch 1 angegebenen Bedeutungen haben und |
| $R^{1-4}$ | für eine den Rest G enthaltenden Gruppierung aus der Beschreibung der erfindungsgemäßen Verbindungen der Formel (I) steht, wobei |
| G | für eine der in Anspruch 1 genannten Gruppierungen (e) bis (k) steht, |

erhält durch allgemein übliche und bekannte Derivatisierungen der entsprechenden Keto-Derivate, Carbonsäure-Derivate oder Nitrile, d.h. Verbindungen der Formel (I), in denen G für Cyano oder eine der Gruppierungen (a) bis (d) steht.

**8.** Verbindungen der Formel (VIII)

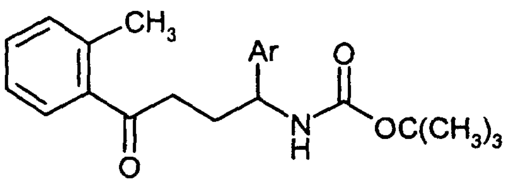

(VIII)

in welcher
Ar die in Anspruch 1 angegebenen Bedeutungen hat.

**9.** Verbindungen der Formel (XVIII)

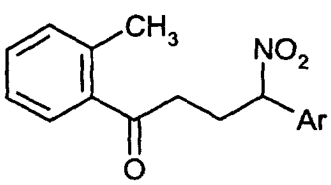

(XVIII)

in welcher
Ar die in Anspruch 1 angegebenen Bedeutungen hat.

**10.** Schädlingsbekämpfungsmittel, **gekennzeichnet durch** einen Gehalt an mindestens einer Verbindung der Formel (I) gemäß Anspruch 1.

**11.** Verwendung von Verbindungen der Formel (I) gemäß Anspruch 1 zur Bekämpfung von Schädlingen.

**12.** Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

**13.** Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

**14.** Verwendung von Verbindungen der Formel (I) gemäß Anspruch 1 zur Herstellung von Schädlingsbekämpfungsmitteln.

## Claims

**1.** Compounds of the formula (I)

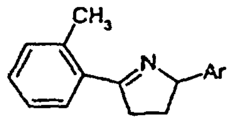

(I)

in which

Ar represents the radical

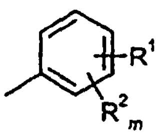

in which

m represents 0, 1, 2, 3 or 4,

$R^1$ represents a substituent in the meta or para position from the group consisting of hydrogen, F, Cl, Br, cyano, tri-($C_1$-$C_6$-alkyl)-silyl, -CO-$NR^4R^5$, tetrahydropyranyl or one of the following groupings

(l) -X - A

(m) -B - Z - D

(n) -Y - E,

$R^2$ represents hydrogen, halogen, cyano, nitro, $C_1$-$C_{16}$-alkyl, $C_1$-$C_{16}$-alkoxy, $C_1$-$C_6$-halogenoalkyl, $C_1$-$C_6$-halogenoalkoxy, $C_1$-$C_8$-alkoxy-$C_1$-$C_8$-alkoxy or -$S(O)_oR^3$,

with the proviso that $R^1$ does not represent hydrogen if m = 1 and $R^2$ = iodine,

o represents 0, 1 or 2,

$R^3$ represents optionally fluorine- or chlorine-substituted $C_1$-$C_6$-alkyl,

$R^4$ and $R^5$ independently of one another each represent hydrogen, $C_1$-$C_6$-alkyl, $C_1$-$C_6$-halogenoalkyl or represent phenyl or phenyl-$C_1$-$C_4$-alkyl, each of which is optionally mono- to trisubstituted by radicals from the list $W^1$,

X represents a direct bond, oxygen, sulphur, carbonyl, carbonyloxy, oxycarbonyl, $C_1$-$C_4$-alkylene, $C_2$-$C_4$-alkenylene, $C_2$-$C_4$-alkinylene, $C_1$-$C_4$-alkyleneoxy, $C_1$-$C_4$-oxyalkylene, $C_1$-$C_4$-thioalkylene, $C_1$-$C_4$-alkylenedioxy or di-$C_1$-$C_4$-alkylsilylene,

A represents phenyl, naphthyl or tetrahydronaphthyl, each of which is optionally mono- to tetrasubstituted by radicals from the list $W^1$, or represents 5- to 10-membered heterocyclyl containing 1 or 2 aromatic rings and having 1 to 4 heteroatoms, which contains 0 to 4 nitrogen atoms, 0 to 2 oxygen atoms and 0 to 2 sulphur atoms, and is in each case optionally mono- to tetrasubstituted by radicals from the list $W^2$,

B represents p-phenylene which is optionally mono- or disubstituted by radicals from the list $W^1$,

Z represents oxygen or sulphur,

D represents hydrogen, $C_1$-$C_{16}$-alkyl, $C_2$-$C_{16}$-alkenyl, $C_2$-$C_6$-alkinyl, $C_1$-$C_{16}$-halogenoalkyl, $C_2$-$C_{16}$-halogenoalkenyl, in each case optionally halogen-, $C_1$-$C_4$-alkyl-, $C_2$-$C_4$-alkenyl-, $C_2$-$C_4$-halogenoalkenyl-, phenyl-, styryl-, halogenophenyl- or halogenostyryl-substituted $C_3$-$C_8$-cycloalkyl or $C_3$-$C_8$-cycloalkyl-$C_1$-$C_6$-alkyl, represents in each case optionally halogen- or $C_1$-$C_4$-alkyl-substituted $C_5$-$C_8$-cycloalkenyl or $C_5$-$C_8$-cycloalkenyl-$C_1$-$C_4$-alkyl, represents in each case optionally nitro-, halogen-, $C_1$-$C_6$-alkyl-, $C_1$-$C_6$-alkoxy-, $C_1$-$C_6$-halogenoalkyl- or $C_1$-$C_6$-halogenoalkoxy-substituted phenyl-$C_1$-$C_6$-alkyl, naphthyl-$C_1$-$C_6$-alkyl, tetrahydronaphthyl-$C_1$-$C_6$-alkyl or hetaryl-$C_1$-$C_6$-alkyl having 5 or 6 ring members and 1 or 2 heteroatoms from the group consisting of nitrogen, oxygen and sulphur represents -CO-$R^6$, -CO-$NR^7R^8$ or represents the grouping

$$-(CH_2)_p - (CR^9R^{10})_q - (CH_2)_r - G,$$

Z and D together may also represent in each case optionally nitro-, halogen-, $C_1$-$C_6$-alkyl-, $C_1$-$C_6$-alkoxy-, $C_1$-$C_6$-halogenoalkyl- or $C_1$-$C_6$-halogenoalkoxy-substituted phenoxy-$C_1$-$C_4$-alkyl,

Y represents a direct bond, oxygen, sulphur, carbonyl, carbonyloxy, oxycarbonyl, $C_1$-$C_4$-alkylene, $C_2$-$C_4$-alkenylene, $C_2$-$C_4$-alkinylene, $C_1$-$C_4$-alkyleneoxy, $C_1$-$C_4$-oxyalkylene, $C_1$-$C_4$-thioalkylene, $C_1$-$C_4$-alkylenedioxy or represents p-phenylene which is optionally mono- or disubstituted by radicals from the list $W^1$,

E represents hydrogen, $C_1$-$C_{16}$-alkyl, $C_2$-$C_{16}$-alkenyl, $C_2$-$C_6$-alkinyl, $C_1$-$C_{16}$-halogenoalkyl, $C_2$-$C_{16}$-halogenoalkenyl, optionally halogen-, $C_1$-$C_4$-alkyl-, $C_2$-$C_4$-alkenyl-, $C_2$-$C_4$-halogenoalkenyl, phenyl-, styryl-, halogenophenyl- or halogenostyryl-substituted $C_3$-$C_8$-cycloalkyl, represents optionally halogen- or $C_1$-$C_4$-alkyl-substituted $C_5$-$C_8$-cycloalkenyl, represents phenyl which is optionally mono- to tetrasubstituted by radicals from the list $W^1$ or represents 5- or 6-membered hetaryl having 1 or 2 heteroatoms from the group consisting of nitrogen, oxygen and sulphur which is optionally mono- to tetrasubstituted by radicals from the list $W^2$ or represents the grouping

$$-(CH_2)_p - (CR^9R^{10})_q - (CH_2)_r - G,$$

$R^6$ represents $C_1$-$C_{12}$-alkyl, $C_1$-$C_{12}$-alkoxy, $C_2$-$C_{12}$-alkenyl, $C_2$-$C_{12}$-alkenyloxy, in each case optionally halogen-, $C_1$-$C_4$-alkyl-, $C_2$-$C_4$-alkenyl-, $C_1$-$C_4$-halogenoalkyl- or $C_2$-$C_4$-halogenoalkenyl-substituted $C_3$-$C_8$-cycloalkyl, $C_3$-$C_8$-cycloalkyloxy or $C_3$-$C_8$-cycloalkyl-$C_1$-$C_6$-alkyloxy or represents phenyl or naphthyl which is in each case optionally mono- to tetrasubstituted by nitro, halogen, $C_1$-$C_{12}$-alkyl, $C_1$-$C_{12}$-alkoxy, $C_1$-$C_{12}$-halogenoalkyl or $C_1$-$C_{12}$-halogenoalkoxy,

$R^7$ represents hydrogen or $C_1$-$C_{12}$-alkyl,

$R^8$ represents $C_1$-$C_{12}$-alkyl, $C_1$-$C_{12}$-halogenoalkyl, in each case optionally halogen-, $C_1$-$C_4$-alkyl-, $C_2$-$C_4$-alkenyl-, $C_1$-$C_4$-halogenoalkyl- or $C_2$-$C_4$-halogenoalkenyl-substituted $C_3$-$C_8$-cycloalkyl or $C_3$-$C_8$-cycloalkyl-$C_1$-$C_6$-alkyl or represents phenyl or phenyl-$C_1$-$C_6$-allcyl, each of which is optionally mono- to tetra-substituted by halogen, $C_1$-$C_{12}$-alkyl, $C_1$-$C_{12}$-alkoxy, $C_1$-$C_{12}$-halogenoalkyl or $C_1$-$C_{12}$-halogenoalkoxy,

| | |
|---|---|
| p, q and r | independently of one another each represent 0, 1, 2 or 3, their sum being smaller than 6 and greater than 1, |
| $R^9$ and $R^{10}$ | independently of one another each represent hydrogen or $C_1$-$C_4$-alkyl, |
| G | represents cyano, represents a 5- or 6-membered heterocycle having 1 to 3 identical or different heteroatoms from the group consisting of nitrogen, oxygen and sulphur and being optionally mono- to trisubstituted by halogen, $C_1$-$C_4$-alkyl or $C_1$-$C_4$-halogenoalkyl and optionally, at the point of linkage, by the radical $R^{11}$, or represents one of the following groupings: |

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

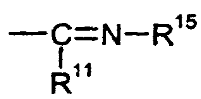

(f)

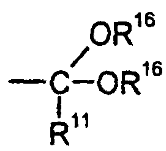

(g)

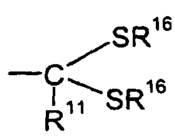

(h)

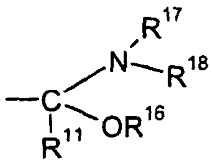

(i)

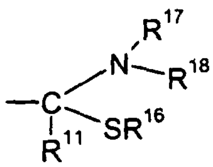

(j)

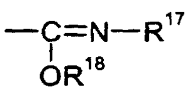

(k)

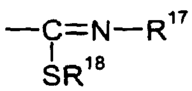

,

$R^{11}$ represents hydrogen, $C_1$-$C_6$-alkyl, $C_2$-$C_6$-alkenyl, $C_1$-$C_4$-halogenoalkyl, $C_2$-$C_6$-halogenoalkenyl, optionally halogen-, $C_1$-$C_4$-alkyl- or $C_1$-$C_4$-halogenoalkyl-substituted $C_3$-$C_6$-cycloalkyl or represents phenyl which is optionally mono- to pentasubstituted by $C_1$-$C_4$-alkylcarbonylamino, $C_1$-$C_4$-alkylcarbonyl-$C_1$-$C_4$-alkylamino and/or radicals from the list $W^3$,

$R^{12}$ represents hydrogen, $C_1$-$C_4$-alkyl, $C_2$-$C_6$-alkenyl, $C_1$-$C_4$-halogenoalkyl, $C_2$-$C_6$-halogenoalkenyl, in each case optionally halogen-, $C_1$-$C_4$-alkyl- or $C_1$-$C_4$-halogenoalkyl-substituted $C_3$-$C_6$-cycloalkyl or $C_3$-$C_6$-cycloalkyl-$C_1$-$C_4$-alkyl or represents $C_6$-$C_{10}$-aryl-$C_1$-$C_4$-alkyl which is optionally mono- to tetrasubstituted by radicals from the list $W^3$,

$R^{13}$ and $R^{14}$ independently of one another each represent hydrogen, $C_1$-$C_4$-alkyl, $C_3$-$C_6$-alkenyl, $C_1$-$C_4$-halogenoalkyl, $C_3$-$C_6$-halogenoalkenyl, $C_1$-$C_4$-alkoxy, in each case optionally halogen-, $C_1$-$C_4$-alkyl- or $C_1$-$C_4$-halogenoalkyl-substituted $C_3$-$C_6$-cycloalkyl or $C_3$-$C_6$-cycloalkyl-$C_1$-$C_4$-alkyl, represent phenyl or phenyl-$C_1$-$C_4$-alkyl, each of which is optionally mono- to pentasubstituted by radicals from the list $W^3$, represent $-OR^{12}$ or $-NR^{11}R^{12}$ or together represent an alkylene chain having 4 to 6 members in which optionally one methylene group is replaced by oxygen,

$R^{15}$ represents $-OR^{12}$, $-NR^{11}R^{12}$ or $-N(R^{11})-COOR^{12}$,

$R^{16}$, $R^{17}$ and $R^{18}$ independently of one another each represent $C_1$-$C_6$-alkyl,

$W^1$ represents hydrogen, halogen, cyano, formyl, nitro, $C_1$-$C_6$-alkyl, tri-$C_1$-$C_4$-alkylsilyl, $C_1$-$C_{16}$-alkoxy, $C_1$-$C_6$-halogenoalkyl, $C_1$-$C_6$-halogenoalkoxy, $C_2$-$C_6$-halogenoalkenyloxy, $C_1$-$C_6$-alkylcarbonyl, $C_1$-$C_{16}$-alkoxycarbonyl, pentafluorothio or $-S(O)_oR^3$,

$W^2$ represents halogen, cyano, formyl, nitro, $C_1$-$C_6$-alkyl, tri-$C_1$-$C_4$-alkylsilyl, $C_1$-$C_{16}$-alkoxy, $C_1$-$C_6$-halogenoalkyl, $C_1$-$C_6$-halogenoalkoxy, $C_1$-$C_6$-alkylcarbonyl, $C_1$-$C_{16}$-alkoxycarbonyl, pentafluorothio, $-S(O)_oR^3$ or$-C(R^{11})N-R^{15}$,

$W^3$ represents halogen, cyano, nitro, $C_1$-$C_4$-alkyl, $C_1$-$C_4$-alkoxy, $C_1$-$C_4$-halogenoalkyl, $C_1$-$C_4$-halogenoalkoxy, di-$C_1$-$C_4$-alkylamino, -$S(O)_oR^3$, -$COOR^{19}$ or -$CONR^{20}R^{21}$,

$R^{19}$ represents hydrogen, $C_1$-$C_4$-alkyl, $C_1$-$C_4$-halogenoalkyl, optionally halogen-, $C_1$-$C_4$-alkyl- or $C_1$-$C_4$-halogenoalkyl-substituted $C_3$-$C_7$-cycloalkyl or represents phenyl which is optionally mono- to pentasubstituted by radicals from the list $W^4$,

$R^{20}$ and $R^{21}$ independently of one another each represent hydrogen, $C_1$-$C_4$-alkyl, $C_3$-$C_6$-alkenyl, $C_1$-$C_4$-halogenoalkyl, $C_3$-$C_6$-halogenoalkenyl, $C_1$-$C_4$-alkoxy, in each case optionally halogen-, $C_1$-$C_4$-alkyl- or $C_1$-$C_4$-halogenoalkyl-substituted $C_3$-$C_6$-cycloalkyl or $C_3$-$C_6$-cycloalkyl-$C_1$-$C_4$-alkyl or represent phenyl or phenyl-$C_1$-$C_4$-alkyl, each of which is optionally mono- to pentasubstituted by radicals from the list $W^4$, represent -$OR^{16}$ or -$NR^{17}R^{18}$ or together represent an alkylene chain having 4 to 6 members in which optionally one methylene group is replaced by oxygen, and

$W^4$ represents halogen, cyano, nitro, $C_1$-$C_6$-alkyl, $C_1$-$C_6$-alkoxy, $C_1$-$C_6$-halogenoalkyl, $C_1$-$C_6$-halogenoalkoxy, di-$C_1$-$C_4$-alkylamino, $C_1$-$C_6$-alkoxycarbonyl, di-$C_1$-$C_6$-alkylaminocarbonyl or -$S(O)_oR^3$ .

**2.** Compounds of the formula (I) according to Claim 1, in which

A represents phenyl, naphthyl or tetrahydronaphthyl, each of which is optionally mono- to tetrasubstituted by radicals from list $W^1$, or represents furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl, each of which is subsequently mono- to tetrasubstituted by radicals from the list $W^2$,

D represents hydrogen, $C_1$-$C_{16}$-alkyl, $C_2$-$C_{16}$-alkenyl, $C_2$-$C_{16}$-alkinyl, $C_1$-$C_{16}$-haloalkyl, $C_2$-$C_{16}$-haloalkenyl, in each case optionally halogen-, $C_1$-$C_4$-alkyl-, $C_2$-$C_4$-alkenyl-, $C_2$-$C_4$-haloalkenyl-, phenyl-, styryl-, halogenophenyl- or halogenostyryl-substituted $C_3$-$C_8$-cycloalkyl or $C_3$-$C_8$-cycloakyl-$C_1$-$C_6$-alkyl, represents in each case optionally halogen- or $C_1$-$C_4$-alkyl-substituted $C_5$-$C_8$-cycloalkenyl or $C_5$-$C_8$-cycloalkenyl-$C_1$-$C_4$-alkyl, represents in each case optionally nitro-, halogen-, $C_1$-$C_6$-alkyl, $C_1$-$C_6$-alkoxy, $C_1$-$C_6$-halogenoalkyl- or $C_1$-$C_6$-halogenoalkoxy-substituted phenyl-$C_1$-$C_6$-alkyl, naphthyl-$C_1$-$C_6$-alkyl, tetrahydronaphthyl-$C_1$-$C_6$-alkyl, furylmethyl, thienylmethyl, pyrrolymethyl, oxazolylmethyl, isoxazolymethyl, thiazolymethyl or pyridylmethyl, represents -$CO$-$R^6$, -$CO$-$NR^7R^8$ or represents the grouping

$$-(CH_2)_p-(CR^9CR^{10})_q-(CH_2)_r-G,$$

E represents hydrogen, $C_1$-$C_{16}$-alkyl, $C_2$-$C_{16}$-alkenyl, $C_2$-$C_6$-alkinyl, $C_1$-$C_{16}$-halogenoalkyl, $C_2$-$C_{16}$-halogenoalkenyl, optionally halogen-, $C_1$-$C_4$-alkyl-, $C_2$-$C_4$-alkenyl-, $C_2$-$C_4$-halogenalkenyl-, phenyl-, styryl-, halogenophenyl-, or halogenostyryl-substituted $C_3$-$C_8$-cycloalkyl, represents optionally halogen- or Ci-$C_4$-alkyl-substituted $C_5$-$C_8$-cycloalkenyl, represents phenyl which is optionally mono- to tetrasubstituted by radicals from the list $W^1$ or represents furyl, thienyl, pyrrolyl, oxazolyl, isoxazolyl, thiazolyl or pyridyl, each of which is optionally mono- to tetrasubstituted by radicals from the list $W^2$, or represents the grouping

$$-(CH_2)_p-(CR^9CR^{10})_q-(CH_2)_r-G,$$

G represents cyano, represents 5,6-dihydrodioxazin-2-yl, 2-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl, each of which is optionally mono- to trisubstituted by halogen, $C_1$-$C_4$-alkyl or $C_1$-$C_4$-halogenoalkyl and, at the point of linkage, optionally by the radical $R^{11}$, or one of the following groupings:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

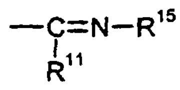

(f)

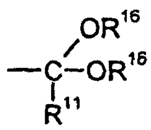

(g)

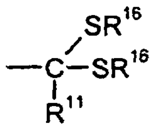

(h)

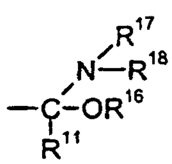

(i)

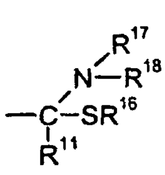

(j)

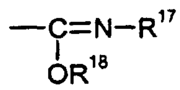

(k)

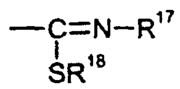

,

$R^{12}$ represents hydrogen, $C_1$-$C_4$-alkyl, $C_2$-$C_6$-alkenyl, $C_1$-$C_4$-halogenoalkyl, $C_2$-$C_6$-halogenoalkenyl, in each case optionally halogen-, $C_1$-$C_4$-alkyl- or $C_1$-$C_4$-halogenoalkyl-substituted $C_3$-$C_6$-cycloalkyl or $C_3$-$C_6$-cycloalkyl-$C_1$-$C_4$-alkyl or represents phenyl-$C_1$-$C_4$-alkyl or naphthyl-$C_1$-$C_4$-alkyl, each of which is optionally mono- to tetrasubstituted by radicals from the list $W^3$.

**3.** Compounds of the formula (I) according to Claim 1 or 2, in which

m represents 0, 1, 2 or 3.

**4.** Compounds of the formula (I) according to Claim 1, in which

Ar represents the radical

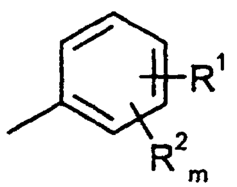

,

m represents 0, 1 or 2,

$R^1$ represents a substituent in the meta or para position from the group consisting of hydrogen, fluorine, chlorine, bromine, cyano, tri-($C_1$-$C_4$-alkyl)-silyl, -CO-$NR^4R^5$, tetrahydropyranyl or one of the following groupings

(l) -X-A

(m) -B-Z-D

(n) - Y - E,

$R^2$ represents hydrogen, fluorine, chlorine, bromine, iodine, cyano, nitro, $C_1$-$C_{16}$-alkyl, $C_1$-$C_{16}$-alkoxy, in each case fluorine- or chlorine-substituted $C_1$-$C_6$-alkyl or $C_1$-$C_6$-alkoxy, represents $C_1$-$C_8$-alkoxy-$C_1$-$C_8$-alkoxy or -$S(O)_oR^3$,

with the proviso that $R^1$ does not represent hydrogen if m = 1 and $R^2$ = iodine,

o represents 0, 1 or 2,

$R^3$ represents $C_1$-$C_4$-alkyl or in each case fluorine- or chlorine- substituted methyl or ethyl,

$R^4$ and $R^5$ independently of one another each represent hydrogen, $C_1$-$C_6$-alkyl, fluorine- or chlorine-substituted $C_1$-$C_6$-alkyl or represent phenyl or benzyl, each of which is optionally mono- or disubstituted by radicals from the list $W^1$,

X represents a direct bond, oxygen, sulphur, carbonyl, carbonyloxy, oxycarbonyl, $C_1$-$C_4$-alkylene $C_2$-$C_4$-alkenylene $C_2$-$C_4$-alkinylene $C_{1}$-$_{4}$-alkylenoxy, $C_1$-$C_4$-oxyalkylene, $C_1$-$C_4$-thioalkylene, $C_1$-$C_4$-alkylenedioxy or di-$C_1$-$C_4$-alkylsilylene,

A represents phenyl, naphthyl or tetrahydronaphthyl, each of which is optionally mono- to trisubstituted by radicals from the list $W^1$, or represents 5- to 10-membered heterocyclyl containing one or two aromatic rings and having 1 to 4 heteroatoms which contains 0 to 4 nitrogen atoms, 0 to 2 oxygen atoms and 0 to 2 sulphur atoms and is in each case optionally mono- to trisubstituted by radicals from the list $W^2$,

B represents p-phenylene which is optionally mono- or disubstituted by radicals from the list $W^1$,

Z represents oxygen or sulphur,

D represents hydrogen, $C_1$-$C_{16}$-alkyl, $C_2$-$C_{16}$-alkenyl, $C_2$-$C_6$-alkinyl, in each case fluorine- or chlorine-substituted $C_1$-$C_4$-alkyl or $C_2$-$C_4$-alkenyl, represents $C_3$-$C_6$-cycloalkyl or $C_3$-$C_6$-cycloalkyl-$C_1$-$C_4$-alkyl, each of which is optionally substituted by fluorine, chlorine, bromine, $C_1$-$C_4$-alkyl, $C_2$-$C_4$-alkenyl, by fluorine- or chlorine-substituted $C_2$-$C_4$-alkenyl, by phenyl, styryl, in each case fluorine-, chlorine- or bromine-substituted phenyl or styryl, represents in each case optionally fluorine-, chlorine-, bromine- or $C_1$-$C_4$-alkyl-substituted $C_5$-$C_6$-cycloalkenyl or $C_5$-$C_6$-cycloalkenyl-$C_1$-$C_4$-alkyl, represents phenyl-$C_1$-$C_4$-alkyl, napthyl-$C_1$-$C_4$-alkyl, tetrahydronaphthyl-$C_1$-$C_6$-alkyl or hetaryl-$C_1$-$C_4$-alkyl having 5 or 6 ring members and one or two heteroatoms from the group consisting of nitrogen, oxygen and sulphur, each of which is optionally substituted by nitro, fluorine, chlorine, bromine, $C_1$-$C_6$-alkyl, $C_1$-$C_6$-alkoxy, in each case fluorine- or chlorine-substituted $C_1$-$C_4$-alkyl or $C_1$-$C_4$-alkoxy, represents -CO-$R^6$, -CO-$NR^7R^8$ or the grouping

$$-(CH_2)_p-(CR^9R^{10})_q-(CH_2)_r-G,$$

Z and D together may also represent substituted phenoxy-$C_1$-$C_3$-alkyl which is optionally substituted by nitro, fluorine, chlorine, bromine, $C_1$-$C_4$-alkyl, $C_1$-$C_4$-alkoxy, in each case fluorine-, or chlorine- substituted $C_1$-$C_4$-alkyl or $C_1$-$C_4$-alkoxy,

Y represents a direct bond, oxygen, sulphur, carbonyl, carbonyloxy, oxycarbonyl, $C_1$-$C_4$-alkylene, $C_2$-$C_4$-alkenylene, $C_2$-$C_4$-alkinylene, $C_1$-$C_4$-alkyleneoxy, $C_1$-$C_4$-oxyalkylene, $C_1$-$C_4$-thioalkylene, $C_1$-$C_4$-alkylenedioxy or represents p-phenylene which is optionally mono- or disubstituted by radicals from the list $W^1$,

E represents hydrogen, $C_1$-$C_{16}$-alkyl, $C_2$-$C_{16}$-alkenyl, $C_2$-$C_6$-alkinyl, in each case fluorine- or chlorine-substituted $C_1$-$C_4$-alkyl or $C_2$-$C_4$-alkenyl, represents $C_3$-$C_6$-cycloalkyl which is in each case substituted by fluorine, chlorine, bromine, $C_1$-$C_4$-alkyl, $C_2$-$C_4$-alkenyl, by fluorine- or chlorine-substituted $C_2$-$C_4$-alkenyl, by phenyl, styryl or in each case fluorine-, chlorine- or bromine- substituted phenyl or styryl, represents in each case fluorine-, chlorine-, bromine- or $C_1$-$C_4$-alkyl-substituted $C_5$-$C_6$-cycloalkenyl, represents phenyl which is optionally mono- to trisubstituted by radicals from the list $W^1$ or represents 5- or 6-membered hetaryl having I or 2 heteroatoms from the group consisting of nitrogen, oxygen and sulphur, each of which is optionally mono- or disubstituted by radicals from the list $W^2$, or represents the grouping

$$-(CH_2)_p-(CR^9R^{10})_q-(CH_2)_r-G,$$

$R^6$ represents $C_1$-$C_6$-alkyl, $C_1$-$C_6$-alkoxy, $C_2$-$C_6$-alkenyl, $C_2$-$C_6$-alkenyloxy, represents $C_3$-$C_6$-cycloalkyl, $C_3$-$C_6$-cycloalkyloxy or $C_3$-$C_6$-cycloalkyl-$C_1$-$C_2$-alkyloxy, each of which is optionally substituted by fluorine, chlorine, $C_1$-$C_3$-alkyl or in each case fluorine- or chlorine-substituted $C_1$-$C_2$-alkyl or $C_2$-$C_3$-alkenyl, or represents phenyl which is optionally mono- or disubstituted by fluorine, chlorine, bromine, iodine, $C_1$-$C_4$-alkyl, $C_1$-$C_4$-alkoxy or by in each case fluorine- or chlorine-substituted $C_1$-$C_3$-alkyl or $C_1$-$C_4$-alkoxy,

$R^7$ represents hydrogen or $C_1$-$C_4$-alkyl,

$R^8$ represents $C_1$-$C_4$-alkyl or represents phenyl or benzyl, each of which is optionally mono- or disubstituted by fluorine, chlorine, bromine, $C_l$-$C_4$-alkyl or by in each case fluorine-, or chlorine-substituted $C_1$-$C_4$-alkyl or $C_1$-$C_4$-alkoxy,

p, q and r independently of one another each represent 0, 1, 2 or 3, their sum being smaller than 6 and greater than 1,

$R^9$ and $R^{10}$ independently of one another each represent hydrogen or $C_1$-$C_4$-alkyl,

G represents cyano, represents a 5- or 6-membered heterocycle having 1 to 3 identical or different heteroatoms from the group consisting of nitrogen, oxygen and sulphur and being optionally mono- to trisubstituted by fluorine, chlorine, bromine, $C_1$-$C_4$-alkyl or by fluorine- or chlorine-substituted $C_1$-$C_4$-alkyl and optionally, at the point of linkage, by the radical $R^{11}$, or represents one of the following groupings:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

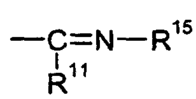

(f)

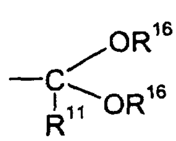

(g)

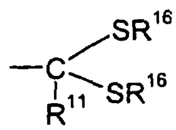

(h)

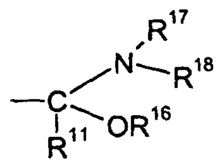

(i)

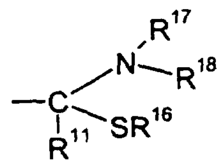

(j)

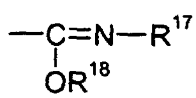

(k)

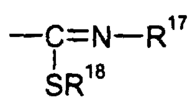

,

$R^{11}$ represents hydrogen, $C_1$-$C_6$-alkyl, $C_2$-$C_6$-alkenyl, in each case fluorine- or chlorine-substituted $C_1$-$C_4$-alkyl or $C_2$-$C_6$-alkenyl, represents $C_3$-$C_6$-cycloalkyl which is optionally substituted by fluorine, chlorine, $C_1$-$C_4$-alkyl or by fluorine- or chlorine-substituted $C_1$-$C_4$-alkyl, or represents phenyl which is optionally mono- to trisubstituted by $C_1$-$C_4$-alkylcarbonylamino, $C_1$-$C_4$-alkylcarbonyl-$C_1$-$C_4$-alkylamino and/or radicals from the list $W^3$,

$R^{12}$ represents hydrogen, $C_1$-$C_4$-alkyl, $C_3$-$C_6$-alkenyl, in each case fluorine- or chlorine-substituted $C_1$-$C_4$-alkyl or $C_3$-$C_6$-alkenyl, represents $C_3$-$C_6$-cycloalkyl or $C_3$-$C_6$-cycloalkyl-$C_1$-$C_4$-alkyl, each of which is optionally substituted by fluorine, chlorine, $C_1$-$C_4$-alkyl or by fluorine- or chlorine-substituted $C_1$-$C_4$-alkyl, or represents phenyl-$C_1$-$C_4$-alkyl or naphthyl-$C_1$-$C_4$-alkyl, each of which is optionally mono- to trisubstituted by radicals from the list $W^3$,

$R^{13}$ and $R^{14}$ independently of one another each represent hydrogen, $C_1$-$C_4$-alkyl, $C_3$-$C_6$-alkenyl, in each case fluorine- or chlorine- substituted $C_1$-$C_4$-alkyl or $C_3$-$C_6$-alkenyl, represent $C_1$-$C_4$-alkoxy, represent $C_3$-$C_6$-cycloalkyl or $C_3$-$C_6$-cycloalkyl-$C_1$-$C_4$-alkyl, each of which is optionally substituted by fluorine, chlorine, $C_1$-$C_4$-alkyl or by fluorine- or chlorine-substituted $C_1$-$C_4$-alkyl, represent phenyl or phenyl-$C_1$-$C_4$-alkyl, each of which is optionally mono- to trisubstituted by

radicals from the list $W^3$, represent $-OR^{12}$ or $-NR^{11}R^{12}$ or together represent $-(CH_2)_5-$, $-(CH_2)_6-$ or $-(CH_2)_2-O-(CH_2)_2-$,

$R^{15}$ represents $-OR^{12}$, $-NR^{11}R^{12}$ or $-N(R^{11})-COOR^{12}$,

$R^{16}$, $R^{17}$ and $R^{18}$ independently of one another each represent $C_1$-$C_4$-alkyl,

$W^1$ represents hydrogen, fluorine, chlorine, bromine, iodine, cyano, formyl, nitro, $C_1$-$C_4$-alkyl, $C_1$-$C_4$-alkoxy, in each case fluorine- or chlorine-substituted $C_1$-$C_4$-alkyl or $C_1$-$C_4$-alkoxy, represents $C_1$-$C_4$-alkylcarbonyl, $C_1$-$C_4$-alkoxycarbonyl or $-S(O)_oR^3$,

$W^2$ represents fluorine, chlorine, bromine, cyano, formyl, nitro, $C_1$-$C_4$-alkyl, $C_1$-$C_4$-alkoxy, in each case fluorine- or chlorine- substituted $C_1$-$C_4$-alkyl or $C_1$-$C_4$-alkoxy, represents $C_1$-$C_4$-alkylcarbonyl, $C_1$-$C_4$-alkoxycarbonyl, $-S(O)_oR^3$ or $-C(R^{11})=N-R^{15}$,

$W^3$ represents fluorine, chlorine, bromine, cyano, nitro, $C_1$-$C_4$-alkyl, $C_1$-$C_4$-alkoxy, in each case fluorine- or chlorine-substituted $C_1$-$C_4$-alkyl or $C_1$-$C_4$-alkoxy, represents di-$C_1$-$C_4$-alkylamino, $-S(O)_oR^3$, $-COOR^{19}$ or $-CONR^{20}R^{21}$,

$R^{19}$ represents hydrogen, $C_1$-$C_4$-alkyl, fluorine- or chlorine-substituted $C_1$-$C_4$-alkyl, represents $C_3$-$C_6$-cycloalkyl which is optionally substituted by fluorine, chlorine, $C_1$-$C_4$-alkyl or by fluorine- or chlorine-substituted $C_1$-$C_4$-alkyl, or represents phenyl, which is optionally mono- to trisubstituted by radicals from the list $W^4$,

$R^{20}$ and $R^{21}$ independently of one another each represent hydrogen, $C_1$-$C_4$-alkyl, $C_3$-$C_6$-alkenyl, in each case fluorine- or chlorine- substituted $C_1$-$C_4$-alkyl or $C_3$-$C_6$-alkenyl, represent $C_1$-$C_4$-alkoxy, represent $C_3$-$C_6$-cycloalkyl or $C_3$-$C_6$-cycloalkyl-$C_1$-$C_4$-alkyl, each of which is optionally substituted by fluorine, chlorine, $C_1$-$C_4$-alkyl or by fluorine- or chlorine-substituted-$C_1$-$C_4$-alkyl, or represent phenyl or phenyl-$C_1$-$C_4$-alkyl, each of which is optionally mono- to trisubstituted by radicals from the list $W^4$, represent $-OR^{16}$ or $-NR^{17}R^{18}$ or together represent $-(CH_2)_5-$, $-(CH_2)_6-$ or $-(CH_2)_2-O-(CH_2)_2-$, and

$W^4$ represents fluorine, chlorine, bromine, cyano, nitro, $C_1$-$C_4$-alkyl, $C_1$-$C_4$-alkoxy, in each case fluorine- or chlorine-substituted $C_1$-$C_4$-alkyl or $C_1$-$C_4$-alkoxy, di-$C_1$-$C_4$-alkylamino, $C_1$-$C_4$-alkoxycarbonyl, di-$C_1$-$C_6$-alkylaminocarbonyl or-$S(O)_oR^3$.

**5.** Compounds of the formula (I) according to Claim 4, in which

A represents phenyl, naphthyl or tetrahydronapthyl, each of which is optionally mono- to trisubstituted by radicals from the list $W^1$, or represents furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl, each of which is optionally mono- to trisubstituted by radicals from the list $W^2$,

D represents hydrogen, $C_1$-$C_{16}$-alkyl, $C_2$-$C_{16}$-alkenyl, $C_2$-$C_6$-alkinyl, in each case fluorine- or chlorine-substituted $C_1$-$C_4$-alkyl or $C_2$-$C_4$-alkenyl, represents $C_3$-$C_6$-cycloalkyl or $C_3$-$C_6$-cycloalkyl-$C_1$-$C_4$-alkyl, each of which is optionally substituted by fluorine, chlorine, bromine, $C_1$-$C_4$-alkyl, $C_2$-$C_4$-alkenyl, by fluorine- or chlorine- substituted $C_2$-$C_4$-alkenyl, by phenyl, styryl, in each case fluorine-, chlorine- or bromine-substituted phenyl or styryl, represents in each case optionally fluorine-, chlorine-, bromine- or $C_1$-$C_4$-alkyl-substituted $C_5$-$C_6$-cycloalkenyl or $C_5$-$C_6$-cycloalkenyl-$C_1$-$C_4$-alkyl, represents phenyl-$C_1$-$C_4$-alkenyl, naphthyl-$C_1$-$C_4$-alkyl, tetrahydronapthyl-$C_1$-$C_6$-alkyl, furylmethyl, thienylmethyl, pyrrolylmethyl, oxazolylmethyl, isoxazolylmethyl, thiazolymethyl or pyridylmethyl, each of which is optionally substituted by nitro, fluorine, chlorine, bromine, $C_1$-$C_6$-alkyl, $C_1$-$C_6$-alkoxy, in each case fluorine- or chlorine-substituted $C_1$-$C_4$-alkyl or $C_1$-$C_4$-alkoxy, represents $-CO-R^6$, $-CO-NR^7R^8$ or the grouping

$$-(CH_2)_p-(CR^9R^{10})_q-(CH_2)_r-G,$$

E represents hydrogen, $C_1$-$C_{16}$-alkyl, $C_2$-$C_{16}$-alkenyl $C_2$-$C_6$-alkinyl, in each case fluorine- or chlorine-substituted $C_1$-$C_4$-alkyl or $C_2$-$C_4$-alkenyl, represents $C_3$-$C_6$-cycloalkyl substituted by fluorine, chlorine, bromine, $C_1$-$C_4$-alkyl, $C_2$-$C_4$-alkenyl, by fluorine- or chlorine-substituted $C_2$-$C_4$-alkenyl, by phenyl, styryl, in each case fluorine-, chlorine- or bromine-substituted phenyl or styryl, represents fluorine-, chlorine- or bromine- or $C_1$-$C_4$-alkyl-substituted $C_5$-$C_6$-cycloalkenyl, represents phenyl which is optionally mono- to trisubstituted by radicals from the list W' or represents furyl, thienyl, pyrrolyl, oxazolyl, isoxazolyl, thiazolyl or pyridyl, each of which is optionally mono- or disubstituted by radicals from the list $W^2$, or represents the grouping

$$-(CH_2)_p-(CR^9R^{10})_q-(CH_2)_r-G,$$

G represents cyano, represents 5,6-dihydrodioxazin-2-yl, 3-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl, each of which is optionally mono- to trisubstituted by fluorine, chlorine, bromine, $C_1$-$C_4$-alkyl or by fluorine- or chlorine-substituted $C_1$-$C_4$-alkyl and optionally, at the point of linkage, by the radical $R^{11}$, or represents one of the following groupings:

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

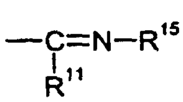

(f)

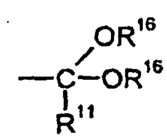

(g)

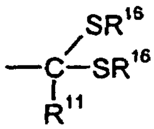

(h)

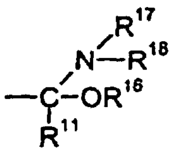

(i)

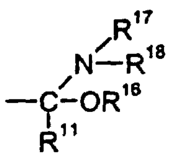

(j)

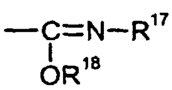

(k)

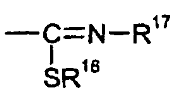

.

**6.** Compounds of the formula (I) according to Claim 1, in which

Ar represents the radical

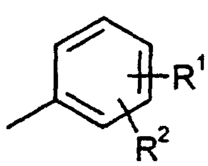

,

$R^1$ represents a substituent in the meta or para position from the group consisting of hydrogen, fluorine, chlorine, bromine, cyano, -CO-NR$^4$R$^5$, tetrahydropyranyl or one of the groupings below

(l) - X - A

(m-a) 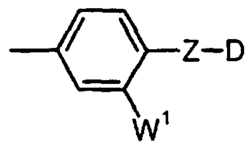

(n) -Y-E,

$R^2$ represents hydrogen, fluorine, chlorine, bromine, iodine, methyl, ethyl, methoxy, ethoxy, methylthio, ethylthio, trifluoromethyl, difluoromethoxy, trifluoromethoxy or trifluormethylthio,

with the proviso that $R^1$ does not represent hydrogen if m = 1 and $R^2$ = iodine,

o represents 0 or 2,

$R^3$ represents methyl, ethyl, n-propyl, isopropyl, difluoromethyl or trifluoromethyl,

$R^4$ and $R^5$ independently of one another each represent hydrogen, methyl, ethyl, n-propyl, isopropyl, n-butyl, isobutyl, sec-butyl, tert-butyl or represent phenyl or benzyl, each of which is optionally monosubstituted by a radical from the list $W^1$,

X represents a direct bond, oxygen, sulphur, carbonyl, $-CH_2-$, $-(CH_2)_2-$, -CH=CH- (E or Z), $-C{\equiv}C-$, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, $C_1$-$C_4$-alkylenedioxy, in particular $-OCH_2O-$, $-O(CH_2)_2O-$ or $-OCH(CH_3)O-$,

A represents phenyl which is optionally mono- or disubstituted by radicals from the list $W^1$ or represents furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl, each of which is optionally mono- or disubstituted by radicals from the list $W^2$,

Z represents oxygen or sulphur,

D represents hydrogen, methyl, ethyl, n-propyl, isopropyl, n-butyl, isobutyl, sec-butyl, tert-butyl, the isomeric pentyls, the isomeric hexyls, n-heptyl, n-octyl, n-isooctyl, n-nonyl, n-decyl, n-undecyl, n-dodecyl, n-tridecyl, n-tetradecyl, n-pentadecyl, n-hexadecyl, 2-propenyl, butenyl, pentenyl, hexenyl, propargyl, butinyl, pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, represents cyclopropyl, cyclobutyl, cyclopentyl, cyclohexyl, cyclopropylmethyl, cyclobutylmethyl, cyclopentylmethyl or cyclohexylmethyl, each of which is optionally mono- to trisubstituted by fluorine, chlorine, bromine, methyl, ethyl, n-propyl, isopropyl, n-butyl, isobutyl, sec-butyl, tert-butyl, ethenyl, 1-propenyl, 2,2-dimethylethenyl, $-CH=CCl_2$, phenyl, styryl, in each case fluorine-, chlorine- or bromine-substituted phenyl or 4-chlorostyryl, represents cyclopentenyl, cyclohexenyl, cyclohexenylmethyl or cyclopentenylmethyl, each of which is optionally substituted by fluorine, chlorine, methyl, ethyl, n-propyl, isopropyl, n-butyl, isobutyl, sec-butyl or tert-butyl, represents benzyl, phenethyl, naphthylmethyl, tetrahydronaphthylmethyl, furylmethyl, thienylmethyl, pyrrolylmethyl, oxazolylmethyl, isoxazolylmethyl, thiazolylmethyl or pyridylmethyl, each of which is optionally mono- or disubstituted by nitro, fluorine, chlorine, bromine, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, trifluoromethyl, trifluoromethoxy, difluoromethoxy or chlorodifluoromethoxy, represents $-CO-R^6$, $-CO-NR^7R^8$ or the grouping

$$-(CH_2)_p-(CR^9R^{10})_q-(CH_2)_r-G,$$

Z and D together may also represent phenoxymethyl which is optionally mono- or disubstituted by nitro, fluorine, chlorine, bromine, methyl, ethyl, n-propyl, i-propyl, methoxy, ethoxy, n-propoxy, i-propoxy, trifluoromethyl, trifluoromethoxy, difluoromethoxy or chlorodifluoromethoxy,

Y represents a direct bond, oxygen, sulphur, carbonyl, $-CH_2-$, $-(CH_2)_2-$, -CH=CH- (E or Z), $-C\equiv C-$, $-CH_2O-$ $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, $C_1$-$C_4$-alkylenedioxy, in particular $-OCH_2O-$ or $-O(CH_2)_2O-$ or represents p-phenylene which is optionally monosubstituted by radicals from the list $W^1$,

E represents hydrogen, methyl, ethyl, n-propyl, isopropyl, n-butyl, isobutyl, sec-butyl, tert-butyl, the isomeric pentyls, the isomeric hexyls, n-heptyl, n-octyl, n-isooctyl, n-nonyl, n-decyl, n-undecyl, n-dodecyl, n-tridecyl, n-tetradecyl, n-pentadecyl, n-hexadecyl, 2-propenyl, butenyl, pentenyl, hexenyl, propargyl, butinyl, pentinyl, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, represents cyclopropyl, cyclobutyl, cyclopentyl or cyclohexyl, each of which is optionally mono- to trisubstituted by fluorine, chlorine, bromine, methyl, ethyl, n-propyl, isopropyl, n-butyl, isobutyl, sec-butyl, tert-butyl, ethenyl, 1-propenyl, 2,2-dimethylethenyl, $-CH=CCl_2$, phenyl, styryl, in each case fluorine-, chlorine- or bromine-substituted phenyl or by 4-chlorostyryl, represents cyclopentenyl or cyclohexenyl, each of which is optionally substituted by fluorine, chlorine, methyl, ethyl, n-propyl, isopropyl, n-butyl, isobutyl, sec-butyl or tert-butyl, represents phenyl which is optionally mono- or disubstituted by radicals from the list $W^1$, represents furyl, thienyl, pyrrolyl, oxazolyl, isoxazolyl, thiazolyl or pyridyl, each of which is optionally mono- or disubstituted by radicals from the list $W^2$, or represents the grouping

$$-(CH_2)_p-(CR^9R^{10})q-(CH_2)_r-G,$$

$R^6$ represents methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, cyclopropyl, cyclohexyl, cyclohexyloxy, cyclohexylmethyloxy, phenyl, 2-chlorophenyl, 3-chlorophenyl, 2,6-difluorophenyl, 2,4-dichlorophenyl, 3,4-dichlorophenyl, 2-trifluoromethoxyphenyl or 4-trifluoromethoxyphenyl,

$R^7$ represents hydrogen,

$R^8$ represents methyl, ethyl or phenyl which is optionally monosubstituted by chlorine,

p, q and r independently of one another each represent 0, 1, 2 or 3, their sum being smaller than 4 and greater than 1,

$R^9$ and $R^{10}$ independently of one another each represent hydrogen, methyl, ethyl, n-propyl, isopropyl, n-butyl, isobutyl, sec-butyl, tert-butyl,

G represents cyano, represents 5,6-dihydrodioxazin-2-yl, 3-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl, each of which is optionally mono- to trisubstituted by fluorine, chlorine, bromine, methyl, ethyl, n-propyl, isopropyl or trifluoromethyl and optionally, at the point of linkage, by the radical $R^{11}$, or represents one of the groupings below:

(a)

$$-CO-R^{11}$$

(b)

$-CO-OR^{12}$

(c)

$-CO-NR^{13}R^{14}$

(d)

$-CS-NR^{13}R^{14}$

(e)

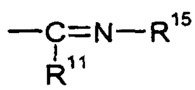

(f)

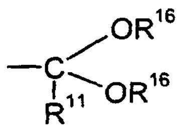

(g)

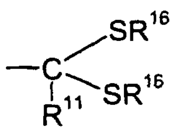

(h)

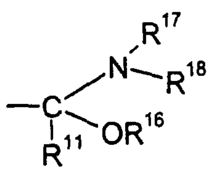

(i)

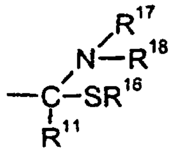

$R^{11}$ represents hydrogen, methyl, ethyl, n-propyl, isopropyl, n-butyl, isobutyl, sec-butyl, tert-butyl, the isomeric pentyls, the isomeric hexyls, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $C_3$-$C_6$-alkenyl, $C_3$-$C_6$-alkenyl which is mono- to trisubstituted

by fluorine or chlorine, represents cyclopropyl, cyclopentyl or cyclohexyl, each of which is optionally mono- or disubstituted by fluorine, chlorine, methyl, ethyl, n-propyl, isopropyl, $-CF_3$, $-CHF_2$, $-CClF_2$, $-CF_2CHFCl$, $-CF_2CH_2F$, $-CF_2CHF_2$, $-CF_2CCl_3$ or $-CH_2CF_3$, or represents phenyl which is optionally mono- or disubstituted by methylcarbonylamino, ethylcarbonylamino, methylcarbonyl-methylamino and/or radicals from the list $W^3$,

$R^{12}$ represents hydrogen, methyl, ethyl, n-propyl, isopropyl, n-butyl, isobutyl, sec-butyl, tert-butyl, $-CH_2CF_3$, allyl, represents cyclopropyl, cyclopentyl, cyclohexyl, cyclopropylmethyl, cyclopentylmethyl, cyclohexylmethyl, cyclopropylethyl, cyclopentylethyl or cyclohexylethyl, each of which is optionally mono- or disubstituted by fluorine, chlorine, methyl, ethyl, n-propyl, isopropyl, $-CF_3$, $-CHF_2$, $-CClF_2$, $-CF_2CHFCl$, $-CF_2CH_2F$, $-CF_2CHF_2$, $-CF_2CCl_3$ or $-CH_2CF_3$, or represents benzyl or phenethyl, each of which is optionally mono- or disubstituted by radicals from the list $W^3$,

$R^{13}$ and $R^{14}$ independently of one another each represent hydrogen, methyl, ethyl, n-propyl, isopropyl, n-butyl, isobutyl, sec-butyl, tert-butyl, $-CH_2CF_3$, methoxy, ethoxy, allyl, represent cyclopropyl, cyclopentyl, cyclohexyl, cyclopropylmethyl, cyclopentylmethyl or cyclohexylmethyl, each of which is optionally mono- or disubstituted by fluorine, chlorine, methyl, ethyl, n-propyl, isopropyl or trifluoromethyl, represent phenyl, benzyl or phenethyl, each of which is optionally mono- or disubstituted by radicals from the list $W^3$, represent $-OR^{12}$ or $-NR^{11}R^{12}$,

$R^{15}$ represents $-OR^{12}$, $-NR^{11} R^{12}$ or $-N(R^{11})-COOR^{12}$,

$R^{16}$, $R^{17}$ and $R^{18}$ independently of one another each represent methyl, ethyl, n-propyl or isopropyl,

$W^1$ represents hydrogen, fluorine, chlorine, bromine, cyano, formyl, nitro, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, trifluoromethoxy, difluoromethoxy, chlorodifluoromethoxy, acetyl, propionyl, butyryl, isobutyryl, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl or $-S(O)_oR^3$,

$W^2$ represents fluorine, chlorine, bromine, cyano, methyl, ethyl, n-propyl, isopropyl, trifluoromethyl, trifluoromethoxy, difluoromethoxy, chlorodifluoromethoxy, acetyl, trifluoromethylthio, $-CH=N-OCH_3$, $-CH=N-OC_2H_5$, $-CH=N-OC_3H_7$, $-C(CH_3)=N-OCH_3$, $-C(CH_3)=N-OC_2H_5$, $-C(CH_3)=N-OC_3H_7$, $-C(C_2H_5)=N-OCH_3$, $-C(C_2H_5)=N-OC_2H_5$ or $-C(C_2H_5)=N-OC_3H_7$,

$W^3$ represents fluorine, chlorine, cyano, nitro, methyl, ethyl, methoxy, ethoxy, methylthio, trifluoromethyl, trifluoromethoxy, trifluoromethylthio, dimethylamino, diethylamino, $-COOR^{19}$ or $-CONR^{20}R^{21}$,

$R^{19}$ represents hydrogen, methyl, ethyl, n-propyl, isopropyl, tert-butyl, $-CH_2CF_3$, represents cyclopropyl, cyclopentyl or cyclohexyl, each of which is optionally mono- or disubstituted by fluorine, chlorine, methyl, ethyl, n-propyl, isopropyl or $-CF_3$, or represents phenyl which is optionally mono- or disubstituted by radicals from the list $W^4$,

$R^{20}$ and $R^{21}$ independently of one another each represent hydrogen, methyl, ethyl, n-propyl, isopropyl, n-butyl, isobutyl, sec-butyl, tert-butyl, $-CH_2CF_3$, methoxy, ethoxy, allyl, represent cyclopropyl, cyclopentyl, cyclohexyl, cyclopropylmethyl, cyclopentylmethyl or cyclohexylmethyl, each of which is optionally mono- or disubstituted by fluorine or chlorine, represent phenyl, benzyl or phenethyl, each of which is optionally mono- or disubstituted by the radicals from the list $W^4$, represent $-OR^{16}$ or $-NR^{17}R^{18}$, and

$W^4$ represents fluorine, chlorine, bromine, cyano, nitro, methyl, ethyl, tert-butyl, methoxy, ethoxy, methylthio, trifluoromethyl, trifluoromethoxy or trifluoromethylthio.

**7.** Process for preparing compounds of the formula (I) according to Claim 1, **characterized in that**

A) cyclic imines of the formula (I)

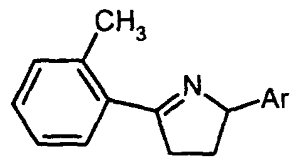

(I)

in which
Ar is as defined in Claim 1
are obtained by

a) reacting aminoketone derivatives of the formula (VIII)

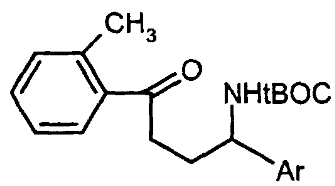

(VIII)

in which

Ar is as defined in Claim 1

with an acid, followed by cyclocondensation, if appropriate in the presence of an acid binder, or

b) reducing the nitro group of nitroketones of the formula (XVIII)

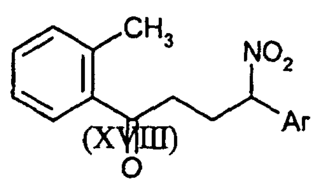

(XVIII)

in which
Ar is as defined in Claim 1, where

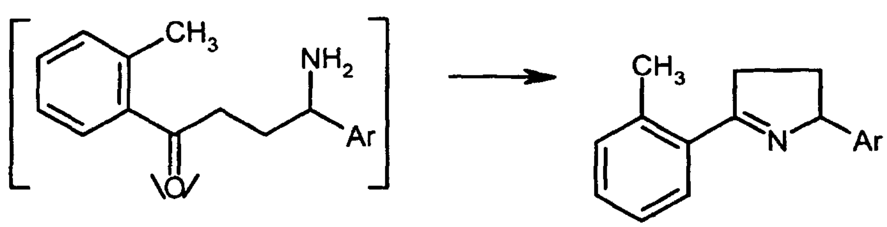

(II) (I)

an aminoketone intermediate of the formula (II) is formed which, however, is cyclocondensed in situ to (I), in particular in an acidic medium, or

c) hydrolysing imines of the formula (XXVII)

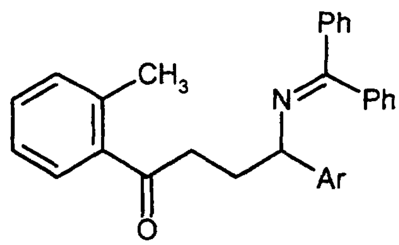

(XXVII)

in which
Ar is as defined in Claim 1
with aqueous acids

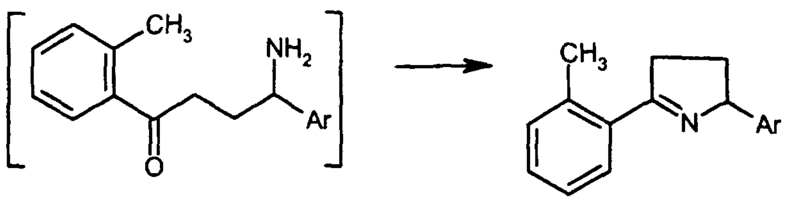

(II) (I)

where an aminoketone intermediate of the formula (II) is formed which, however, is cyclocondensed in situ to (I), or

B) reacting compounds of the formula (III)

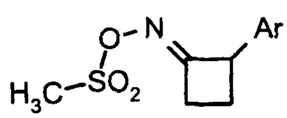

(III)

in which

Ar is as defined in Claim 1

with aryl Grignard compounds of the formula (IV)

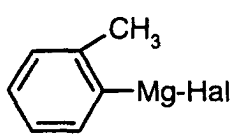

(IV)

in which

Hal represents chlorine, bromine or iodine

in the presence of a diluent, or

C) compounds of the formula (I-b)

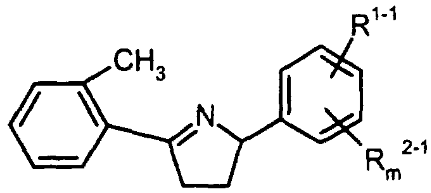 (I-b)

in which

m is as defined in Claim 1

$R^{1-1}$ represents A or one of the groupings below

(m) -B-Z-D

(n-a) 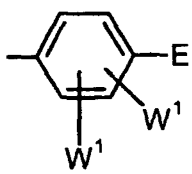

where

A, B, D, E, $W^1$ and Z are each as defined in Claim 1 and

$R^{2-1}$ represents hydrogen, fluorine, cyano, nitro, alkyl, alkoxy, halogenoalkyl, halogenoalkoxy, alkoxyalkoxy or $-SR^3$ where

$R^3$ is as defined in Claim 1

are obtained by coupling compounds of the formula (V)

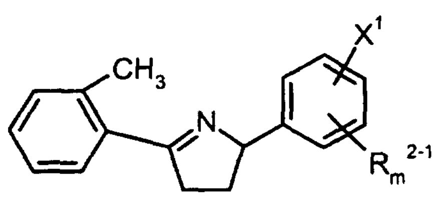 (V)

in which

$R^{2-1}$ is as defined above,
m is as defined in Claim 1, and
$X^1$ represents bromine, iodine or $-OSO_2CF_3$

with boronic acids of the formula (VI)

$$R^{1-1}\text{-}B(OH)_2 \quad \text{(VI)}$$

in which

$R^{1-1}$ is as defined above

in the presence of a catalyst and in the presence of an acid binder and in the presence of a solvent, or

D) cyclic imines of the formula (I-c)

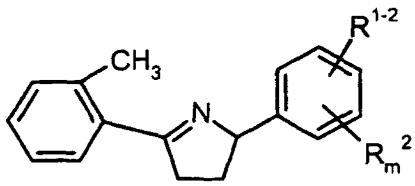

(I-c)

in which
$R^2$ and m are each as defined in Claim 1,

$R^{1-2}$ represents one of the groupings below

(m-b) $-B-Z-D^1$

(n-b) $-Y^1-E^1$

in which
B and Z are each as defined in Claim 1,

$Y^1$ represents oxygen or sulphur and

$D^1$ and $E^1$ represent the grouping

$$-(CH_2)_p-(CR^9R^{10})_q-(CH_2)_r-G$$

in which

$R^9$, $R^{10}$, G, p, q and r are each as defined in Claim 1

are obtained by condensing cyclic imines of the formula (I-d)

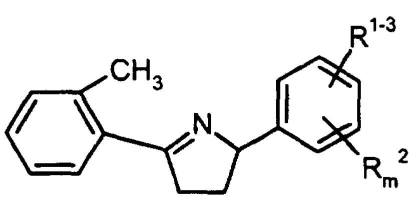

(I-d)

in which

$R^2$ and m are each as defined in Claim 1 and

$R^{1-3}$ represents one of the groupings below

(m-c) -B - Z - H

(n-c) $-Y^1$ - H

in which
B and Z are each as defined in Claim 1 and
$Y^1$ is as defined above

with compounds of the formula (VII)

$$Ab - (CH_2)p - (CR^9R^{10})_q - (CH_2)_r - G \quad (VII)$$

in which

$R^9$, $R^{10}$, G, p, q and r are each as defined in Claim 1 and

Ab represents a leaving group,

or

E) cyclic imines of the formula (I-e)

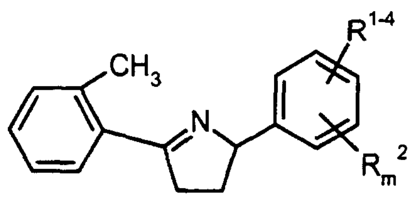

(I-e)

in which

$R^2$ und m are each as defined in Claim 1 and

$R^{1-4}$ represents a grouping from the description of the compounds of the formula (I) according to the invention which contains the radical G, where

G represents one of the groupings (e) to (k) mentioned in Claim 1,

are obtained by generally customary and known derivatizations of the corresponding keto derivatives, carboxylic acid derivatives or nitriles, i.e. of compounds of the formula (I) in which G represents cyano or one of the groupings (a) to (d).

**8.** Compounds of the formula (VIII)

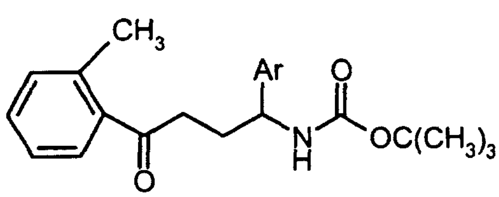

(VIII),

in which

Ar is as defined in Claim 1.

**9.** Compounds of the formula (XVIII)

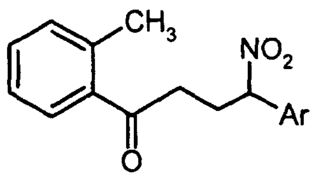

(XVIII),

in which

Ar is as defined in Claim 1.

**10.** Pesticides, **characterized in that** they contain at least one compound of the formula (I) according to Claim 1.

**11.** Use of compounds of the formula (I) according to Claim I for controlling pests.

**12.** Method of controlling pests, **characterized in that** compounds of the formula (I) according to Claim 1 are allowed to act on pests and/or their habitat.

**13.** Process for preparing pesticides, **characterized in that** compounds of the formula (I) according to Claim 1 are mixed with extenders and/or surfactants.

**14.** Use of compounds of the formula (I) according to Claim 1 for preparing pesticides.

**Revendications**

**1.** Composés de formule (I)

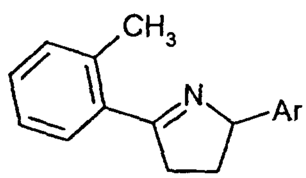

(I)

dans laquelle

Ar représente le reste

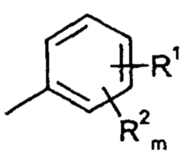

dans lequel

m a la valeur 0, 1, 2, 3 ou 4,

$R^1$ représente un substituant, en position méta ou para, de la série hydrogène, F, Cl, Br, cyano, tri-(alkyle en $C_1$ à $C_6$)-silyle, -CO-$NR^4R^5$, tétrahydropyrannyle ou l'un des groupements suivants

(l) -X-A

(m) -B-Z-D

(n) -Y-E,

$R^2$ représente l'hydrogène, un halogène, un groupe cyano, nitro, un reste alkyle en $C_1$ à $C_{16}$, alkoxy en $C_1$ à $C_{16}$, halogénalkyle en $C_1$ à $C_6$, halogénalkoxy en $C_1$ à $C_6$, (alkoxy en $C_1$ à $C_8$)-(alkoxy en $C_1$ à $C_8$) ou -S $(O)_oR^3$, sous réserve que $R^1$ ne représente pas l'hydrogène lorsque m est égal à 1 et $R^2$ représente l'iode,

o a la valeur 0, 1 ou 2,

$R^3$ est un reste alkyle en $C_1$ à $C_6$ éventuellement substitué par du fluor ou du chlore,

$R^4$ et $R^5$ représentent, indépendamment l'un de l'autre, l'hydrogène, un reste alkyle en $C_1$ à $C_6$, halogénalkyle en $C_1$ à $C_6$ ou un reste phényle ou phényle-(alkyle en $C_1$ à $C_4$) chacun éventuellement substitué une à trois fois par des restes de la liste $W^1$,

X représente une liaison simple, l'oxygène, le soufre, un groupe carbonyle, carbonyloxy, oxycarbonyle, un reste alkylène en $C_1$ à $C_4$, alcénylène en $C_2$ à $C_4$, alcynylène en $C_2$ à $C_4$, alkylénoxy en $C_1$ à $C_4$, oxyalkylène en $C_1$ à $C_4$, thioalkylène en $C_1$ à $C_4$, alkylènedioxy en $C_1$ à $C_4$ ou di-(alkyle en $C_1$ à $C_4$)-silylène,

A est un reste phényle, naphtyle ou tétrahydronaphtyle, chacun éventuellement substitué une à quatre fois par des restes de la liste $W^1$, ou bien un reste hétérocyclyle à 1 ou 2 noyaux aromatiques et de 5 à 10 chaînons et de 1 à 4 hétéroatomes, éventuellement substitués dans chaque cas 1 à 4 fois par des restes de la liste $W^2$ et contenant 0 à 4 atomes d'azote, 0 à 2 atomes d'oxygène et 0 à 2 atomes de soufre,

B est un reste p-phénylène éventuellement substitué une ou deux fois par des restes de la liste $W^1$,

Z représente l'oxygène ou le soufre,

D représente l'hydrogène, un reste alkyle en $C_1$ à $C_{16}$, alcényle en $C_2$ à $C_{16}$, alcynyle en $C_2$ à $C_6$, halogénalkyle en $C_1$ à $C_{16}$, halogénalcényle en $C_2$ à $C_{16}$, un reste cycloalkyle en $C_3$ à $C_8$ ou (cycloalkyle en $C_3$ à $C_8$)-(alkyle en $C_1$ à $C_6$) chacun éventuellement substitué par un radical halogéno, alkyle en $C_1$ à $C_4$, alcényle en $C_2$ à $C_4$, halogénalcényle en $C_2$ à $C_4$, phényle, styryle, halogénophényle ou halogénostyryle, un reste cycloalcényle en $C_5$ à $C_8$ ou (cycloalcényle en $C_5$ à $C_8$)-(alkyle en $C_1$ à $C_4$) chacun éventuellement substitué par un radical halogène ou alkyle en $C_1$ à $C_4$, un reste phényle-(alkyle en $C_1$ à $C_6$), naphtyle-(alkyle en $C_1$ à $C_6$), tétrahydronaphtyl-(alkyle en $C_1$ à $C_6$) ou (hétaryle pentagonal ou hexagonal)-(alkyle en $C_1$ à $C_6$) ayant un ou deux hétéroatomes de la série azote, oxygène et soufre et chacun étant éventuellement substitué par un radical nitro, halogéno, alkyle en $C_1$ à $C_6$, alkoxy en $C_1$ à $C_6$, halogénalkyle en $C_1$ à $C_6$ ou halogénalkoxy en $C_1$ à $C_6$, un groupe $-CO-R^6$, $-CO-NR^7R^8$ ou le groupement

$$-(CH_2)_p-(CR^9R^{10})_q-(CH_2)_r-G,$$

Z et D peuvent aussi former ensemble un reste phénoxy-(alkyle en $C_1$ à $C_4$) éventuellement substitué dans chaque cas par un radical nitro, halogéno, alkyle en $C_1$ à $C_6$, alkoxy en $C_1$ à $C_6$, halogénalkyle en $C_1$ à $C_6$ ou halogénalkoxy en $C_1$ à $C_6$,

Y est une liaison directe, l'oxygène, le soufre, un groupe carbonyle, carbonyloxy, oxycarbonyle, un reste alkylène en $C_1$ à $C_4$, alcénylène en $C_2$ à $C_4$, alcynylène en $C_2$ à $C_4$, alkylénoxy en $C_1$ à $C_4$, oxyalkylène en $C_1$ à $C_4$, thioalkylène en $C_1$ à $C_4$, alkylènedioxy en $C_1$ à $C_4$ ou un reste p-phénylène éventuellement substitué une ou deux fois par des restes de la liste $W^1$,

E représente l'hydrogène, un reste alkyle en $C_1$ à $C_{16}$, alcényle en $C_2$ à $C_{16}$, alcynyle en $C_2$ à $C_6$, halogénalkyle en $C_1$ à $C_{16}$, halogénalcényle en $C_2$ à $C_{16}$, un reste cycloalkyle en $C_3$ à $C_8$, éventuellement substitué par un radical halogéno, alkyle en $C_1$ à $C_4$, alcényle en $C_2$ à $C_4$, halogénalkyle en $C_2$ à $C_4$, phényle, styryle, halogénophényle ou halogénostyryle, un reste cycloalcényle en $C_5$ à $C_8$ éventuellement substitué par un radical halogéno ou alkyle en $C_1$ à $C_4$, un reste phényle éventuellement 1 à 4 fois substitué par des restes de la liste $W^1$ ou un reste hétaryle pentagonal ou hexagonal ayant 1 ou 2 hétéroatomes de la série azote, oxygène et

soufre et éventuellement 1 à 4 fois par des restes de la liste $W^2$, ou le groupement

$$-(CH_2)_p-(CR^9R^{10})q-(CH_2)_r-G,$$

$R^6$ représente un reste alkyle en $C_1$ à $C_{12}$, alkoxy en $C_1$ à $C_{12}$, alcényle en $C_2$ à $C_{12}$, alcényloxy en $C_2$ à $C_{12}$, un reste cycloalkyle en $C_3$ à $C_8$, cycloalkyloxy en $C_3$ à $C_8$ ou (cycloalkyle en $C_3$ à $C_8$) - (alkyloxy en $C_1$ à $C_6$) chacun éventuellement substitué par un radical halogéno, alkyle en $C_1$ à $C_4$, alcényle en $C_2$ à $C_4$, halogénalkyle en $C_1$ à $C_4$ ou halogénalcényle en $C_2$ à $C_4$, ou un reste phényle ou naphtyle, chacun éventuellement substitué 1 à 4 fois par un radical nitro, halogéno, alkyle en $C_1$ à $C_{12}$, alkoxy en $C_1$ à $C_{12}$, halogénalkyle en $C_1$ à $C_{12}$ ou halogénalkoxy en $C_1$ à $C_{12}$,

$R^7$ représente l'hydrogène ou un reste alkyle en $C_1$ à $C_{12}$,

$R^8$ représente un reste alkyle en $C_1$ à $C_{12}$, halogénalkyle en $C_1$ à $C_{12}$, un reste cycloalkyle en $C_3$ à $C_8$ ou (cycloalkyle en $C_3$ à $C_8$) - (alkyle en $C_1$ à $C_6$) chacun éventuellement substitué par un radical halogéno, alkyle en $C_1$ à $C_4$, alcényle en $C_2$ à $C_4$, halogénalkyle en $C_1$ à $C_4$ ou halogénalcényle en $C_2$ à $C_4$, ou un reste phényle ou phényl-(alkyle en $C_1$ à $C_6$) chacun éventuellement substitué 1 à 4 fois par un radical halogéno, alkyle en $C_1$ à $C_{12}$, alkoxy en $C_1$ à $C_{12}$, halogénalkyle en $C_1$ à $C_{12}$ ou halogénalkoxy en $C_1$ à $C_{12}$,

p, q et r ont indépendamment l'un de l'autre la valeur 0, 1, 2 ou 3, leur soinme étant inférieure à 6 et supérieure à 1,

$R^9$ et $R^{10}$ représentent indépendamment l'un de l'autre l'hydrogène ou un reste alkyle en $C_1$ à $C_4$,

G est un groupe cyano, un cycle pentagonal ou hexagonal ayant 1 à 3 hétéroatomes identiques ou différents de la série azote, oxygène et soufre et chacun étant éventuellement substitué 1 à 3 fois par un radical halogéno, alkyle en $C_1$ à $C_4$ ou halogénalkyle en $C_1$ à $C_4$ et, le cas échéant, par le reste $R^{11}$ au niveau de la jonction, ou bien l'un des groupements suivants :

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

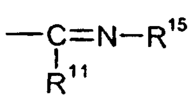

(f)

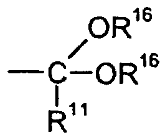

(g)

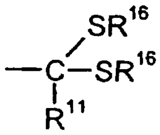

(h)

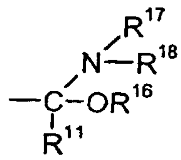

(i)

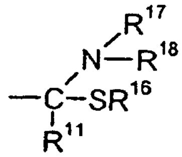

(j)

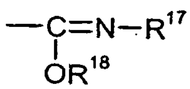

(k)

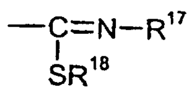

$R^{11}$ représente l'hydrogène, un reste alkyle en $C_1$ à $C_6$, alcényle en $C_2$ à $C_6$, halogénalkyle en $C_1$ à $C_4$, halogénalcényle en $C_2$ à $C_6$, un reste cycloalkyle en $C_3$ à $C_6$, éventuellement substitué par un radical halogéno, alkyle en $C_1$ à $C_4$ ou halogénalkyle en $C_1$ à $C_4$ ou un reste phényle éventuellement substitué 1 à 5 fois par un radical (alkyle en $C_1$ à $C_4$)-carbonylamino, (alkyle en $C_1$ à $C_4$)-carbonyle-(alkylamino en $C_1$ à $C_4$) et/ou des restes de la liste $W^3$,

$R^{12}$ représente l'hydrogène, un reste alkyle en $C_1$ à $C_4$, alcényle en $C_2$ à $C_6$, halogénalkyle en $C_1$ à $C_4$, halogénalcényle en $C_2$ à $C_6$, un reste cycloalkyle en $C_3$ à $C_6$ ou (cycloalkyle en $C_3$ à $C_6$) - (alkyle en $C_1$ à $C_4$) chacun éventuellement substitué par un radical halogéno, alkyle en $C_1$ à $C_4$, halogénalkyle en $C_1$ à $C_4$, ou un reste (aryle en $C_6$ à $C_{10}$) - (alkyle en $C_1$ à $C_4$), éventuellement substitué 1 à 4 fois par les restes de la liste $W^3$,

$R^{13}$ et $R^{14}$ représentent indépendamment l'un de l'autre l'hydrogène, un reste alkyle en $C_1$ à $C_4$, alcényle

en $C_3$ à $C_6$, halogénalkyle en $C_1$ à $C_4$, halogénalcényle en $C_3$ à $C_6$, alkoxy en $C_1$ à $C_4$, un reste cycloalkyle en $C_3$ à $C_6$ ou (cycloalkyle en $C_3$ à $C_6$) - (alkyle en $C_1$ à $C_4$) chacun éventuellement substitué par un radical halogéno, alkyle en $C_1$ à $C_4$ ou halogénalkyle en $C_1$ à $C_4$, un reste phényle ou phényl-(alkyle en $C_1$ à $C_4$) chacun éventuellement substitué 1 à 5 fois par des restes de la liste $W^3$, un groupe $-OR^{12}$ ou $-NR^{11}R^{12}$ ou forment ensemble une chaîne alkylénique de 4 à 6 chaînons, dans laquelle un groupe méthylène est éventuellement remplacé par de l'oxygène,

$R^{15}$ est un groupe $-OR^{12}$, $-NR^{11}R^{12}$ ou $-N(R^{11})-COOR^{12}$,

$R^{16}$, $R^{17}$ et $R^{18}$ représentent indépendamment les uns des autres un reste alkyle en $C_1$ à $C_6$,

$W^1$ représente l'hydrogène, un halogène, un groupe cyano, formyle, nitro, un reste alkyle en $C_1$ à $C_6$, trialkylsilyle en $C_1$ à $C_4$, alkoxy en $C_1$ à $C_{16}$, halogénalkyle en $C_1$ à $C_6$, halogénalkoxy en $C_1$ à $C_6$, halogénalcényloxy en $C_2$ à $C_6$, (alkyle en $C_1$ à $C_6$)-carbonyle, (alkoxy en $C_1$ à $C_{16}$)-carbonyle, pentafluorothio ou $-S(O)_oR^3$,

$W^2$ représente un halogène, un groupe cyano, formyle, nitro, un reste alkyle en $C_1$ à $C_6$, trialkylsilyle en $C_1$ à $C_4$, alkoxy en $C_1$ à $C_{16}$, halogénalkyle en $C_1$ à $C_6$, halogénalkoxy en $C_1$ à $C_6$, (alkyle en $C_1$ à $C_6$)-carbonyle, (alkoxy en $C_1$ à $C_{16}$)-carbonyle, pentafluorothio, $-S (O)\ _oR^3$ ou $-C (R^{11}) =N-R^{15}$,

$W^3$ représente un halogène, un groupe cyano, nitro, un reste alkyle en $C_1$ à $C_4$, alkoxy en $C_1$ à $C_4$, halogénalkyle en $C_1$ à $C_4$, halogénalkoxy en $C_1$ à $C_4$, di-(alkyle en $C_1$ à $C_4$)-amino, $-S(O)\ _oR^3$, $-COOR^{19}$ ou $-CONR^{20}R^{21}$,

$R^{19}$ représente l'hydrogène, un reste alkyle en $C_1$ à $C_4$, halogénalkyle en $C_1$ à $C_4$, un reste cycloalkyle en $C_3$ à $C_7$ éventuellement substitué par un radical halogène ou alkyle en $C_1$ à $C_4$ ou halogénalkyle en $C_1$ à $C_4$ ou un reste phényle éventuellement substitué 1 à 5 fois par des restes de la liste $W^4$,

$R^{20}$ et $R^{21}$ représentent indépendamment l'un de l'autre l'hydrogène, un reste alkyle en $C_1$ à $C_4$, alcényle en $C_3$ à $C_6$, halogénalkyle en $C_1$ à $C_4$, halogénalcényle en $C_3$ à $C_6$, alkoxy en $C_1$ à $C_4$, un reste cycloalkyle en $C_3$ à $C_6$ ou (cycloalkyle en $C_3$ à $C_6$)-(alkyle en $C_1$ à $C_4$) chacun éventuellement substitué par un radical halogéno ou alkyle en $C_1$ à $C_4$ ou halogénalkyle en $C_1$ à $C_4$, ou un reste phényle ou phényl-(alkyle en $C_1$ à $C_4$) chacun éventuellement substitué 1 à 5 fois par des restes de la liste $W^4$, un groupe $-OR^{16}$ ou $-NR^{17}R^{18}$ ou forment ensemble une chaîne alkylénique de 4 à 6 chaînons, dans laquelle un groupe méthylène est éventuellement remplacé par de l'oxygène, et

$W^4$ représente un halogène, un groupe cyano, nitro, un reste alkyle en $C_1$ à $C_6$, alkoxy en $C_1$ à $C_6$, halogénalkyle en $C_1$ à $C_6$, halogénalkoxy en $C_1$ à $C_6$, di-(alkyle en $C_1$ à $C_4$)-amino, (alkoxy en $C_1$ à $C_6$)-carbonyle, di-(alkyle en $C_1$ à $C_6$)-aminocarbonyle ou $-S (O)_oR_3$.

**2.** Composés de formule (I) suivant la revendication 1, formule dans laquelle

A est un reste phényle, naphtyle ou tétrahydronaphtyle, chacun éventuellement substitué 1 à 4 fois par des restes de la liste $W^1$ ou un reste furyle, benzofuryle, thiényle, benzothiényle, oxazolyle, benzoxazolyle, thiazolyle, benzothiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle, chacun éventuellement substitué 1 à 4 fois par des restes de la liste $W^2$,

D représente l'hydrogène, un reste alkyle en $C_1$ à $C_{16}$, alcényle en $C_2$ à $C_{16}$, alcynyle en $C_2$ à $C_6$, halogénalkyle en $C_1$ à $C_{16}$, halogénalcényle en $C_2$ à $C_{16}$, un reste cycloalkyle en $C_3$ à $C_8$ ou (cycloalkyle en $C_3$ à $C_8$)-alkyle en $C_1$ à $C_6$, chacun éventuellement substitué par un radical halogéno, alkyle en $C_1$ à $C_4$, alcényle en $C_2$ à $C_4$, halogénalcényle en $C_2$ à $C_4$, phényle, styryle, halogénophényle ou halogénostyryle ou un reste cycloalcényle en $C_5$ à $C_8$ ou (cycloalcényle en $C_5$ à $C_8$)-(alkyle en $C_1$ à $C_4$) chacun éventuellement substitué par un radical halogéno ou alkyle en $C_1$ à $C_4$, un reste phényl-(alkyle en $C_1$ à $C_6$), naphtyl-(alkyle en $C_1$ à $C_6$), tétrahydronaphtyl-(alkyle en $C_1$ à $C_6$), furylméthyle, thiénylméthyle, pyrrolylméthyle, oxazolylméthyle, isoxazolylméthyle, thiazolylméthyle ou pyridylméthyle, chacun éventuellement substitué par un radical nitro, halogéno ou alkyle en $C_1$ à $C_6$, alkoxy en $C_1$ à $C_6$, halogénalkyle en $C_1$ à $C_6$ ou halogénalkoxy en $C_1$ à $C_6$, un groupe $-CO-R^6$, $-CO-NR^7R^8$ ou le groupement

$$- (CH_2)\ p\text{-}\ (CR^9R^{10})_q\text{-}\ (CH_2)\ _r\text{-}G,$$

E représente l'hydrogène, un reste alkyle en $C_1$ à $C_{16}$, alcényle en $C_2$ à $C_{16}$, alcynyle en $C_2$ à $C_6$, halogénalkyle en $C_1$ à $C_{16}$, halogénalcényle en $C_2$ à $C_{16}$, un reste cycloalkyle en $C_3$ à $C_8$ éventuellement substitué par un radical halogéno, alkyle en $C_1$ à $C_4$, alcényle en $C_2$ à $C_4$, halogénalkyle en $C_2$ à $C_4$, phényle, styryle, halogénophényle ou halogénostyryle, un reste cycloalcényle en $C_5$ à $C_8$ éventuellement substitué par un radical halogéno ou alkyle en $C_1$ à $C_4$, un reste phényle éventuellement substitué 1 à 4 fois par des restes de la liste $W^1$ ou un reste furyle, thiényle, pyrrolyle, oxazolyle, isoxazolyle, thiazolyle ou pyridyle, chacun éventuellement substitué par des restes de la liste $W^2$, ou le groupement

$$-(CH_2)p-(CR^9R^{10})q-(CH_2)_r-G,$$

G est un groupe cyano, un reste 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxanne-2-yle chacun éventuellement substitué par le reste $R^{11}$ au niveau de la jonction, ou l'un des groupements suivants

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

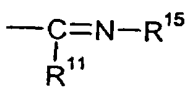

(f)

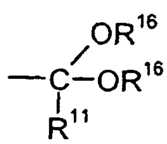

(g)

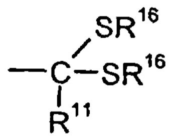

(h)

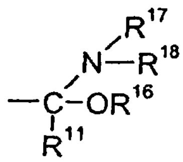

(i)

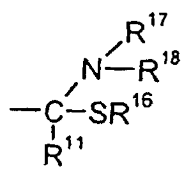

(j)

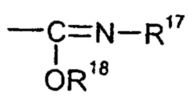

(k)

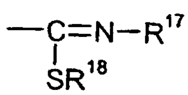

$R^{12}$ représente l'hydrogène, un reste alkyle en $C_1$ à $C_4$, alcényle en $C_2$ à $C_6$, halogénalkyle en $C_1$ à $C_4$, halogénalcényle en $C_2$ à $C_6$, un reste cycloalkyle en $C_3$ à $C_6$ ou (cycloalkyle en $C_3$ à $C_6$) - (alkyle en $C_1$ à $C_4$) chacun éventuellement substitué par un radical halogéno, alkyle en $C_1$ à $C_4$ ou halogénalkyle en $C_1$ à $C_4$ ou un reste phényl- (alkyle en $C_1$ à $C_4$) ou naphtyl-(alkyle en $C_1$ à $C_4$) chacun éventuellement substitué 1 à 4 fois par des restes de la liste $W^3$.

**3.** Composés de formule (I) suivant la revendication 1 ou 2, formule dans laquelle

m a la valeur 0, 1, 2 ou 3,

**4.** Composés de formule (I) suivant la revendication 1, formule dans laquelle

Ar représente le reste

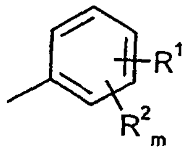

m a la valeur 0, 1 ou 2,

$R^1$ représente un substituant en position méta ou para de la série hydrogène, fluor, chlore, brome, cyano, tri-(alkyle en $C_1$ à $C_6$)-silyle, -CO-$NR^4R^5$, tétrahydropyrannyle ou l'un des groupements suivants

(l) -X-A

(m) -B-Z-D

(n) -Y-E

$R^2$ représente l'hydrogène, le fluor, le chlore, le brome, l'iode, un groupe cyano, nitro, un reste alkyle en $C_1$ à $C_{16}$, alkoxy en $C_1$ à $C_{16}$, un reste alkyle en $C_1$ à $C_6$ ou alkoxy en $C_1$ à $C_6$, chacun substitué par du fluor ou du chlore, un reste (alkoxy en $C_1$ à $C_8$) - (alkoxy en $C_1$ à $C_8$) ou -S $(O)_0$ $R^3$,
sous réserve que $R^1$ ne représente pas l'hydrogène lorsque m = 1 et $R^2$ représente l'iode,

o a la valeur 0, 1 ou 2,

$R^3$ est un reste alkyle en $C_1$ à $C_4$ ou un reste méthyle ou éthyle chacun substitué par du fluor ou du chlore,

$R^4$ et $R^5$ représentent, indépendamment l'un de l'autre, l'hydrogène, un reste alkyle en $C_1$ à $C_6$, un reste alkyle en $C_1$ à $C_6$ substitué par du fluor ou du chlore ou un reste phényle ou benzyle, chacun éventuellement substitué une ou deux fois par des restes de la liste $W^1$,

X représente une liaison directe, l'oxygène, le soufre, un groupe carbonyle, carbonyloxy, oxycarbonyle, un reste alkylène en $C_1$ à $C_4$, alcénylène en $C_2$ à $C_4$, alcynylène en $C_2$ à $C_4$, alcénylénoxy en $C_1$ à $C_4$, oxyalkylène en $C_1$ à $C_4$, thioalkylène en $C_1$ à $C_4$, alkylènedioxy en $C_1$ à $C_4$ ou di-(alkyle en $C_1$ à $C_4$)-silylène,

A représente un reste phényle, naphtyle, tétrahydronaphtyle, chacun éventuellement substitué une à trois fois par des restes de la liste $W^1$ ou un reste hétérocyclyle à 1 ou 2 noyaux aromatiques, de 5 à 10 chaînons et 1 à 4 hétéroatomes, qui contient 0 à 4 atomes d'azote, 0 à 2 atomes d'oxygène et 0 à 2 atomes de soufre,

B est un reste p-phénylène éventuellement substitué une ou deux fois par des restes de la liste $W^1$,

Z représente l'oxygène ou le soufre,

D représente l'hydrogène, un reste alkyle en $C_1$ à $C_{16}$, alcényle en $C_2$ à $C_{16}$, alcynyle en $C_2$ à $C_6$, un reste alkyle en $C_1$ à $C_4$ ou alcényle en $C_2$ à $C_4$ chacun substitué par du fluor ou du chlore, un reste cycloalkyle en $C_2$ à $C_4$ ou (cycloalkyle en $C_3$ à $C_6$)-(alkyle en $C_1$ à $C_4$) chacun éventuellement substitué par du fluor, du chlore, du brome, un radical alkyle en $C_1$ à $C_4$, alcényle en $C_2$ à $C_4$, alcényle en $C_2$ à $C_4$ substitué par du fluor ou du chlore, phényle, styryle, phényle ou styryle chacun substitué par du fluor, du chlore ou du brome, un reste cycloalcényle en $C_5$ ou $C_6$ ou (cycloalcényle en $C_5$ ou $C_6$)-(alkyle en $C_1$ à $C_4$) chacun éventuellement substitué par du fluor, du chlore, du brome ou un radical alkyle en $C_1$ à $C_4$, un reste phényl-(alkyle en $C_1$ à $C_4$), napthyl-(alkyle en $C_1$ à $C_4$), tétrahydronaphtyl-(alkyle en $C_1$ à $C_6$) ou (hétaryle pentagonal ou hexagonal)-(alkyle en $C_1$ à $C_4$) ayant 1 ou 2 hétéroatomes de la série azote, oxygène et soufre, chacun éventuellement substitué par un radical nitro, fluoro, chloro, bromo, alkyle en $C_1$ à $C_6$, alkoxy en $C_1$ à $C_6$, alkyle en $C_1$ à $C_4$ ou alkoxy en $C_1$ à $C_4$, chacun substitué par du fluor ou du chlore, un reste -CO-$R^6$, -CO-$NR^7R^8$ ou le groupement

$$-(CH_2)_p-(CR^9R^{10})_q-(CH_2)_r-G,$$

Z et D peuvent aussi former ensemble un reste phénoxy-(alkyle en $C_1$ à $C_3$) éventuellement subs-

titué par un radical nitro, fluoro, chloro, bromo, alkyle en $C_1$ à $C_4$, alkoxy en $C_1$ à $C_4$, alkyle en $C_1$ à $C_4$ ou alkoxy en $C_1$ à $C_4$ chacun substitué par du fluor ou du chlore,

Y représente une liaison directe, l'oxygène, le soufre, un groupe carbonyle, carbonyloxy, oxycarbonyle, un reste alkylène en $C_1$ à $C_4$, alcénylène en $C_2$ à $C_4$, alcynylène en $C_2$ à $C_4$, alkylénoxy en $C_1$ à $C_4$, oxyalkylène en $C_1$ à $C_4$, thioalkylène en $C_1$ à $C_4$, alkylènedioxy en $C_1$ à $C_4$ ou un reste p-phénylène éventuellement substitué une ou deux fois par des restes de la liste $W^1$,

E représente l'hydrogène, un reste alkyle en $C_1$ à $C_{16}$, alcényle en $C_2$ à $C_{16}$, alcynyle en $C_2$ à $C_6$, un reste alkyle en $C_1$ à $C_4$ ou alcényle en $C_2$ à $C_4$ chacun substitué par du fluor ou du chlore, un reste cycloalkyle en $C_3$ à $C_6$ substitué dans chaque cas par du fluor, du chlore, du brome, un radical alkyle en $C_1$ à $C_4$, alcényle en $C_2$ à $C_4$, un radical alcényle en $C_2$ à $C_4$ substitué par du fluor ou du chlore, un radical phényle ou styryle chacun substitué par du fluor, du chlore ou du brome, un reste cycloalcényle en $C_5$ ou $C_6$ substitué dans chaque cas par du fluor, du chlore, du brome ou un radical alkyle en $C_1$ à $C_4$, un reste phényle éventuellement substitué une à trois fois par des restes de la série $W^1$ ou un reste hétaryle pentagonal ou hexagonal contenant 1 ou 2 hétéroatomes de la série azote, oxygène et soufre, éventuellement substitué dans chaque cas une ou deux fois par des restes de la liste $W^2$, ou bien le groupement

$$-(CH_2)_p-(CR^9R^{10})_q-(CH_2)_r-G,$$

$R^6$ est un reste alkyle en $C_1$ à $C_6$, alkoxy en $C_1$ à $C_6$, alcényle en $C_2$ à $C_6$, alcényloxy en $C_2$ à $C_6$, un reste cycloalkyle en $C_3$ à $C_6$, cycloalkyloxy en $C_3$ à $C_6$ ou (cycloalkyle en $C_3$ à $C_6$)-(alkyloxy en $C_1$ ou $C_2$) chacun éventuellement substitué par du fluor, du chlore, un radical alkyle en $C_1$ à $C_3$ ou un radical alkyle en $C_1$ ou $C_2$ ou alcényle en $C_2$ ou $C_3$ chacun substitué par du fluor ou du chlore, ou bien un reste phényle éventuellement substitué une ou deux fois par un radical fluoro, chloro, bromo, iodo, alkyle en $C_1$ à $C_4$, alkoxy en $C_1$ à $C_4$ ou un radical alkyle en $C_1$ à $C_3$ ou alkoxy en $C_1$ à $C_4$ chacun substitué par du fluor ou du chlore,

$R^7$ représente l'hydrogène ou un reste alkyle en $C_1$ à $C_4$,

$R^8$ est un reste alkyle en $C_1$ à $C_4$ ou un reste phényle ou benzyle chacun éventuellement substitué une ou deux fois par un radical fluoro, chloro, bromo, alkyle en $C_1$ à $C_4$ ou un radical alkyle en $C_1$ à $C_4$ ou alkoxy en $C_1$ à $C_4$, chacun substitué par du fluor ou du chlore,

p, q et r ont indépendamment les uns des autres la valeur 0, 1, 2 ou 3, leur somme étant inférieure à 6 et supérieure à 1,

$R^9$ et $R^{10}$ représentent indépendamment l'un de l'autre l'hydrogène ou un reste alkyle en $C_1$ à $C_4$,

G est un groupe cyano, un hétérocycle pentagonal ou hexagonal contenant 1 à 3 hétéroatomes identiques ou différents de la série azote, oxygène et soufre, éventuellement substitué une à trois fois par du fluor du chlore, du brome, un radical alkyle en $C_1$ à $C_4$ ou un radical alkyle en $C_1$ à $C_4$ substitué par du fluor ou du chlore, et éventuellement substitué par le reste $R^{11}$ au niveau de la jonction, ou l'un des groupements :

(a)

$$-CO-R^{11}$$

(b)

$$-CO-OR^{12}$$

(c)

$$-CO-NR^{13}R^{14}$$

(d)

$-CS-NR^{13}R^{14}$

(e)

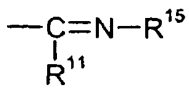

(f)

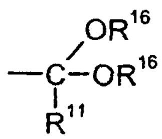

(g)

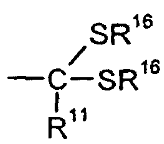

(h)

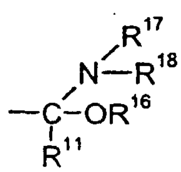

(i)

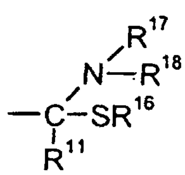

(j)

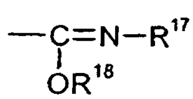

(k)

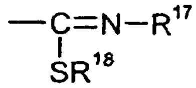

$R^{11}$ représente l'hydrogène, un reste alkyle en $C_1$ à $C_6$, alcényle en $C_2$ à $C_6$, un reste alkyle en $C_1$ à $C_4$ ou alcényle en $C_2$ à $C_6$, chacun substitué par du fluor ou du chlore, un reste cycloalkyle en $C_3$ à $C_6$ éventuellement substitué par un radical fluoro, chloro, alkyle en $C_1$ à $C_4$ ou par un radical alkyle en $C_1$ à $C_4$ substitué par du fluor ou du chlore, ou un reste phényle éventuellement substitué une à trois fois par un radical (alkyle en $C_1$ à $C_4$)-carbonylamino, (alkyle en $C_1$ à $C_4$)-carbonyle-(alkyle en $C_1$ à $C_4$)-amino et/ou par des restes de la liste $W^3$,

$R^{12}$ représente l'hydrogène, un reste alkyle en $C_1$ à $C_4$, alcényle en $C_3$ à $C_6$, un reste alkyle en $C_1$ à $C_4$ ou alcényle en $C_2$ à $C_6$ chacun substitué par du fluor ou du chlore, un reste cycloalkyle en $C_3$ à $C_6$ ou (cycloalkyle en $C_3$ à $C_6$) - (alkyle en $C_1$ à $C_4$) chacun substitué par un radical fluoro, chloro, alkyle en $C_1$ à $C_4$ ou par un radical alkyle en $C_1$ à $C_4$ substitué par du fluor ou du chlore, ou bien un reste phényl-(alkyle en $C_1$ à $C_4$) ou naphtyl-(alkyle en $C_1$ à $C_4$) chacun éventuellement substitué une à trois fois par des restes de la série $W^3$,

$R^{13}$ et $R^{14}$ représentent indépendamment l'un de l'autre l'hydrogène, un reste alkyle en $C_1$ à $C_4$, alcényle en $C_3$ à $C_6$, un reste alkyle en $C_1$ à $C_4$ ou alcényle en $C_3$ à $C_6$, chacun substitué par du fluor ou du chlore, un reste alkoxy en $C_1$ à $C_4$, un reste cycloalkyle en $C_3$ à $C_6$ ou (cycloalkyle en $C_3$ à $C_6$) - (alkyle en $C_1$ à $C_4$) chacun éventuellement substitué par un radical fluoro, chloro, alkyle en $C_1$ à $C_4$ ou par un radical alkyle en $C_1$ à $C_4$ substitué par du fluor ou du chlore, un reste phényle ou phényl-(alkyle en $C_1$ à $C_4$) chacun éventuellement substitué une à trois fois par des restes de la liste $W^3$, un reste $-OR^{12}$ ou $-NR^{11}R^{12}$ ou forment ensemble un groupe - $(CH_2)_5$-, -$(CH_2)_6$- ou - $(CH_2)_2$-O- $(CH_2)_2$-,

$R^{15}$ est un groupe $-OR^{12}$, $-NR^{11}R^{12}$ ou -N $(R^{11})$ $-COOR^{12}$,

$R^{16}$, $R^{17}$ et $R^{18}$ représentent indépendamment les uns des autres un reste alkyle en $C_1$ à $C_4$,

$W^1$ représente l'hydrogène, le fluor, le chlore, le brome, l'iode, un groupe cyano, formyle, nitro, un reste alkyle en $C_1$ à $C_4$, alkoxy en $C_1$ à $C_4$, un reste alkyle en $C_1$ à $C_4$ ou alkoxy en $C_1$ à $C_4$ chacun substitué par du fluor ou du chlore, un reste (alkyle en $C_1$ à $C_4$)-carbonyle, (alkoxy en $C_1$ à $C_4$)-carbonyle ou -S $(O)_oR^3$,

$W^2$ représente le fluor, le chlore, le brome, un groupe cyano, formyle, nitro, un reste alkyle en $C_1$ à $C_4$, alkoxy en $C_1$ à $C_4$, un reste alkyle en $C_1$ à $C_4$ ou alkoxy en $C_1$ à $C_4$ chacun substitué par du fluor ou du chlore, un reste (alkyle en $C_1$ à $C_4$)-carbonyle, (alkoxy en $C_1$ à $C_4$) -carbonyle, -S(O) $_oR^3$ ou -C $(R^{11})$ =N-$R^{15}$,

$W^3$ représente le fluor, le chlore, le brome, un groupe cyano, nitro, un reste alkyle en $C_1$ à $C_4$, alkoxy en $C_1$ à $C_4$, un reste alkyle en $C_1$ à $C_4$ ou alkoxy en $C_1$ à $C_4$ chacun substitué par du fluor ou du chlore, un reste di-(alkyle en $C_1$ à $C_4$)-amino, -S $(O)_oR^3$, $-COOR^{19}$ ou $-CONR^{20}R^{21}$,

$R^{19}$ représente l'hydrogène, un reste alkyle en $C_1$ à $C_4$, un reste alkyle en $C_1$ à $C_4$ substitué par du fluor ou du chlore, un reste cycloalkyle en $C_3$ à $C_6$ éventuellement substitué par du fluor, du chlore, un radical alkyle en $C_1$ à $C_4$ ou par un radical alkyle en $C_1$ à $C_4$ substitué par du fluor ou du chlore, ou un reste phényle éventuellement substitué une à trois fois par des restes de la liste $W^4$,

$R^{20}$ et $R^{21}$ représentent indépendamment l'un de l'autre l'hydrogène, un reste alkyle en $C_1$ à $C_4$, alcényle en $C_3$ à $C_6$, un reste alkyle en $C_1$ à $C_4$ ou alcényle en $C_3$ à $C_6$, chacun substitué par du fluor ou du chlore, un reste alkoxy en $C_1$ à $C_4$, un reste cycloalkyle en $C_3$ à $C_6$ ou (cycloalkyle en $C_3$ à $C_6$) - (alkyle en $C_1$ à $C_4$) chacun éventuellement substitué par du fluor, du chlore, un radical alkyle en $C_1$ à $C_4$ ou un radical alkyle en $C_1$ à $C_4$ substitué par du fluor ou du chlore, ou un reste phényle ou phényl-(alkyle en $C_1$ à $C_4$) chacun substitué le cas échéant une à trois fois par des restes de la liste $W^4$, un groupe $-OR^{16}$ ou $-NR^{17}R^{18}$, ou bien ils forment ensemble un groupement -$(CH_2)_5$-, -$(CH_2)_6$- ou -$(CH_2)_2$-O-$(CH_2)_2$-, et

$W^4$ représente le fluor, le chlore, le brome, un groupe cyano, nitro, un reste alkyle en $C_1$ à $C_4$, alkoxy en $C_1$ à $C_4$, un reste alkyle en $C_1$ à $C_4$ ou alkoxy en $C_1$ à $C_4$ chacun substitué par du fluor ou du chlore, un reste di-(alkyle en $C_1$ à $C_4$)-amino, (alkoxy en $C_1$ à $C_4$)-carbonyle, di- (alkyle en $C_1$ à $C_6$)-aminocarbonyle, ou -S$(O)_oR^3$.

**5.** Composés de formule (I) suivant la revendication 4, formule dans laquelle,

A représente un reste phényle, naphtyle ou tétrahydronaphtyle chacun éventuellement substitué une à trois fois par des restes de la liste $W^1$, ou un reste furyle, benzofuryle, thiényle, benzothiényle, oxazolyle, benzoxazolyle, thiazolyle, benzothiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle, chacun éventuellement substitué une à trois fois par des restes de la liste $W^2$,

D représente l'hydrogène, un reste alkyle en $C_1$ à $C_{16}$, alcényle en $C_2$ à $C_{16}$, alcynyle en $C_2$ à $C_{16}$, un reste alkyle en $C_1$ à $C_4$ ou alcényle en $C_2$ à $C_4$ chacun substitué par du fluor ou du chlore, un reste cycloalkyle en $C_3$ à $C_6$ ou (cycloalkyle en $C_3$ à $C_6$)-(alkyle en $C_1$ à $C_4$) chacun éventuellement substitué par du fluor, du chlore, du brome, un radical alkyle en $C_1$ à $C_4$, alcényle en $C_2$ à $C_4$, alcényle en $C_2$ à $C_4$ substitué par du fluor ou du chlore, phényle, styryle, phényle ou styryle chacun substitué par du fluor ou du brome, un reste phényl-(alkyle en $C_1$ à $C_4$), naphtyl-(alkyle en $C_1$ à $C_4$), tétrahydronaphtyl-(alkyle en $C_1$ à $C_4$), furylméthyle, thiénylméthyle, pyrrolylméthyle, oxazolylméthyle, isoxazolylméthyle, thiazolylméthyle ou pyridylméthyle, chacun éventuellement substitué par un radical nitrio, fluoro, chloro, bromo, alkyle en $C_1$ à $C_6$, alkoxy en $C_1$ à $C_6$, un radical alkyle en $C_1$ à $C_4$ ou alkoxy en $C_1$ à $C_4$, chacun substitué par du fluor ou du chlore, un reste $-CO-R^6$, $-CO-NR^7R^8$ ou le groupement

$$-(CH_2)_p-(CR^9R^{10})_r-G,$$

E représente l'hydrogène, un reste alkyle en $C_1$ à $C_{16}$, alcényle en $C_2$ à $C_{16}$, alcynyle en $C_2$ à $C_6$, un reste alkyle en $C_1$ à $C_4$ ou alcényle en $C_2$ à $C_4$ chacun substitué par du fluor ou du chlore, un reste cycloalkyle en $C_3$ à $C_6$, substitué dans chaque cas par du fluor, du chlore, du brome, un radical alkyle en $C_1$ à $C_4$, alcényle en $C_2$ à $C_4$, alcényle en $C_2$ à $C_4$ substitué par du fluor ou du chlore, phényle, styryle, phényle ou styryle chacun substitué par du fluor, du chlore ou du brome, un reste cycloalcényle en $C_5$ ou $C_6$, chacun substitué par du fluor, du chlore, du brome ou un radical alkyle en $C_1$ à $C_4$, un reste phényle éventuellement substitué une à trois fois par des restes de la liste $W^1$ ou un reste furyle, thiényle, pyrrolyle, oxazolyle, isoxazolyle, thiazolyle ou pyridyle, chacun éventuellement substitué une ou deux fois par des restes de la liste $W^2$, ou le groupement

$$-(CH_2)_p-(CR^9R^{10})\ q-(CH_2)\ _r-G,$$

G est un groupe cyano, un reste 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxanne-2-yle éventuellement substitué une à trois fois par du fluor, du chlore, du brome, un radical alkyle en $C_1$ à $C_4$ ou par un radical alkyle en $C_1$ à $C_4$ substitué par du fluor ou du chlore, et substitué le cas échéant par le reste $R^{11}$ au niveau de la jonction, ou l'un des groupements suivants :

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

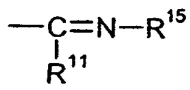

(f)

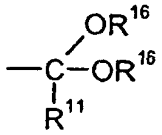

(g)

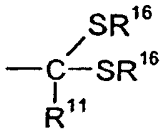

(h)

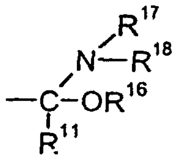

(i)

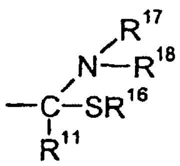

(j)

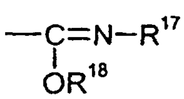

(k)

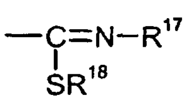

**6.** Composé de formule (I) suivant la revendication 1, formule dans laquelle

Ar représente le reste

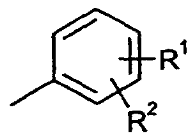

$R^1$ représente un substituant en position méta ou para de la série hydrogène, fluor, chlore, brome, cyano, -CO-$NR^4R^5$, tétrahydropyrannyle ou l'un des groupements suivants

(l) -X-A

(m-a) 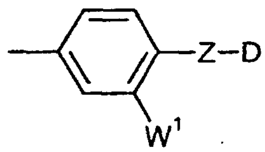

(n) -Y-E,

$R^2$ représente l'hydrogène, le fluor, le chlore, le brome, l'iode, un reste méthyle, éthyle, méthoxy, éthoxy, méthylthio, éthylthio, trifluorométhyle, difluorométhoxy, trifluorométhoxy ou trifluorométhylthio, sous réserve que $R^1$ ne représente pas l'hydrogène lorsque m est égal à 1 et $R^2$ est l'iode,

o a la valeur 0 ou 2,

$R^3$ est un reste méthyle, éthyle, n-propyle, isopropyle, difluorométhyle ou trifluorométhyle,

$R^4$ et $R^5$ représentent indépendamment l'un de l'autre l'hydrogène, un reste méthyle, éthyle, n-propyle, isopropyle, n-butyle, isobutyle, sec.-butyle, tertiobutyle ou un reste phényle ou benzyle chacun éventuellement substitué une fois par un reste de la liste $W^1$,

X représente une liaison simple, l'oxygène, le soufre, un groupe carbonyle, $-CH_2-$, $-(CH_2)_2-$, -CH=CH- (E ou Z), -C≡C-, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, alkylènedioxy en $C_1$ à $C_4$, notamment $-OCH_2O-$, $-O(CH_2)_2O-$ ou $-OCH(CH_3)O-$,

A représente un reste phényle éventuellement substitué une ou deux fois par des restes de la liste $W^1$ ou un reste furyle, benzofuryle, thiényle, benzothiényle, oxazolyle, benzoxazolyle, thiazolyle, benzothiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle, chacun éventuellement substitué une ou deux fois par des restes de la liste $W^2$,

Z représente l'oxygène ou le soufre,

D représente l'hydrogène, un reste méthyle, éthyle, n-propyle, isopropyle, n-butyle, isobutyle, sec.-butyle, tertiobutyle, les restes pentyle isomères, les restes hexyle isomères, les restes n-heptyle, n-octyle, n-isoctyle, n-nonyle, n-décyle, n-undécyle, n-dodécyle, n-tridécyle, n-tétradécyle, n-pentadécyle, n-hexadécyle, 2-propényle, butényle, pentényle, hexényle, propargyle, butynyle, pentynyle, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué une à trois fois par du fluor, du chlore, du brome, un radical méthyle, éthyle, n-propyle, isopropyle, n-butyle, isobutyle, sec.-butyle, tertiobutyle, éthényle, 1-propényle, 2,2-diméthyléthényle, $-CH=CCl_2$, phényle, styryle, phényle ou 4-chlorophényle chacun substitué par du fluor, du chlore ou du brome, un reste benzyle, phénéthyle, naphtylméthyle, tétrahydronaphtylméthyle, furylméthyle, thiénylméthyle, pyrrolylméthyle, oxazolylméthyle, isoxazolylméthyle, thiazolylméthyle ou pyridylméthyle chacun éventuellement substitué une ou deux fois par un radical nitro, fluoro, chloro, bromo, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, trifluorométhyle, trifluorométhoxy, difluorométhoxy ou chlorodifluorométhoxy, un groupe $-CO-R^6$, $-CO-NR^7R^8$ ou le groupe

$$-(CH_2)_p-(CR^9R^{10})_q-(CH_2)_r-G,$$

Z et D peuvent aussi former ensemble un reste phénoxyméthyle éventuellement substitué une ou deux fois par un radical nitro, fluoro, chloro, bromo, méthyle, éthyle, n-propyle, isopropyle, méthoxy, éthoxy, n-propoxy, isopropoxy, trifluorométhyle, trifluorométhoxy, difluorométhoxy ou chlorodifluorométhoxy,

Y désigne une liaison simple, l'oxygène, le soufre, un groupe carbonyle, $-CH_2-$, $-(CH_2)_2-$, -CH=CH- (E ou Z), -C≡C-, $-CH_2O-$, $-(CH_2)_2O-$, $-CH(CH_3)O-$, $-OCH_2-$, $-O(CH_2)_2-$, $-SCH_2-$, $-S(CH_2)_2-$, $-SCH(CH_3)-$, alkylènedioxy en $C_1$ à $C_4$, notamment $-OCH_2O-$ ou $-O(CH_2)_2O-$ ou un reste p-phénylène éventuellement substitué une fois par des restes de la série $W^1$,

E représente l'hydrogène, un reste méthyle, éthyle, n-propyle, isopropyle, n-butyle, isobutyle, sec.-butyle, tertiobutyle, les restes pentyle isomères, les restes hexyle isomères, un reste n-heptyle, n-octyle, n-isoctyle, n-nonyle, n-décyle, n-undécyle, n-dodécyle, n-tridécyle, n-tétradécyle, n-pentadécyle, n-hexadécyle, 2-propényle, butényle, pentényle, hexényle, propargyle, butynyle, pentynyle, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, un reste cyclopropyle, cyclobutyle, cyclopentyle ou cyclohexyle chacun éventuellement substitué jusqu'à trois fois par un radical fluoro, chloro, bromo, méthyle, éthyle, n-propyle, isopropyle, n-butyle, isobutyle, sec.-butyle, tertiobutyle, éthényle, 1-propényle, 2,2-diméthyléthényle, $-CH=CCl_2$, phényle, styryle, phényle substitué dans chaque cas par du fluor, du chlore ou du brome, ou 4-chlorostyryle, un reste cyclopentényle ou cyclohexényle chacun éventuellement substitué par du fluor, du chlore, un radical méthyle, éthyle, n-propyle, isopropyle, n-butyle, isobutyle, sec.-butyle ou tertiobutyle, un reste phényle éventuellement substitué une ou deux fois par des restes de la liste $W^1$, un reste furyle, thiényle, pyrrolyle, oxazolyle, isoxazolyle, thiazolyle ou pyridyle chacun éventuellement substitué une ou deux fois par des restes de la liste $W^2$, ou le groupement

$$-(CH_2)_p-(CR^9R^{10})q-(CH_2)_r-G,$$

$R^6$ est un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, cyclopropyle, cyclohexyle, cyclohexyloxy, cyclohexylméthyloxy, phényle, 2-chlorophényle, 3-chlorophényle, 2,6-difluorophényle, 2,4-dichlorophényle, 3,4-dichlorophényle, 2-trifluorométhoxyphényle ou 4-trifluorométhoxyphényle,

$R^7$ représente l'hydrogène,

$R^8$ est un reste méthyle, éthyle ou un reste phényle éventuellement substitué une fois par du chlore,

p, q et r ont indépendamment les uns des autres la valeur 0, 1, 2 ou 3, leur somme étant inférieure à 4 et supérieure à 1,

$R^9$ et $R^{10}$ représentent indépendamment l'un de l'autre l'hydrogène, un reste méthyle, éthyle, n-propyle, isopropyle, n-butyle, isobutyle, sec.-butyle ou tertiobutyle,

G est un groupe cyano, un reste 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxanne-2-yle, chacun éventuellement substitué une à trois fois par du fluor, du chlore, du brome, un radical méthyle, éthyle, n-propyle, isopropyle ou trifluorométhyle et le cas échéant par le reste $R^{11}$ au niveau de la jonction, ou bien l'un des groupements suivants :

(a)

$$-CO-R^{11}$$

(b)

$$-CO-OR^{12}$$

(c)

$$-CO-NR^{13}R^{14}$$

(d)

$$-CS-NR^{13}R^{14}$$

(e)

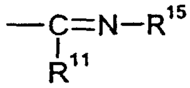

(f)

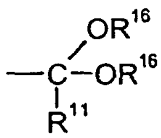

(g)

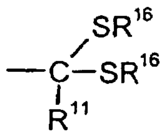

(h)

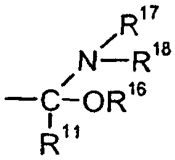

(i)

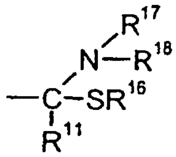

$R^{11}$ représente l'hydrogène, un reste méthyle, éthyle, n-propyle, isopropyle, n-butyle, isobutyle, sec.-butyle, tertiobutyle, les restes pentyle isomères, les restes hexyle isomères, un reste $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, alcényle en $C_3$ à $C_6$, un reste alcényle en $C_3$ à $C_6$ substitué une à trois fois par du fluor ou du chlore, un reste cyclopropyle, cyclopentyle ou cyclohexyle chacun éventuellement substitué une ou deux fois par du fluor, du chlore, un radical méthyle, éthyle, n-propyle, isopropyle, $-CF_3$, $-CHF_2$, $-CClF_2$, $-CF_2CHFCl$, $-CF_2CH_2F$, $-CF_2CHF_2$, $-CF_2CCl_3$ ou $-CH_2CF_3$ ou un reste phényle éventuellement substitué une ou deux fois par un radical méthylcarbonylamino, éthylcarbonylamino, méthylcarbonylméthylamino et/ou des restes de la liste $W^3$,

$R^{12}$ représente l'hydrogène, un reste méthyle, éthyle, n-propyle, isopropyle, n-butyle, isobutyle, sec.-butyle, tertiobutyle, $-CH_2CF_3$, allyle, un reste cyclopropyle, cyclopentyle, cyclohexyle, cyclopropylméthyle, cyclopentylméthyle, cylclohexylméthyle, cyclopropyléthyle, cyclopentyléthyle ou cyclohexyléthyle, chacun éventuellement substitué une ou deux fois par du fluor, du chlore, un radical méthyle, éthyle, n-propyle, isopropyle, $-CF_3$, $-CHF_2$, $-CClF_2$, $-CF_2CHFCl$, $-CF_2CH_2F$, $-CF_2CHF_2$, $-CF_2CCl_3$ ou $-CH_2CF_3$, ou un reste benzyle ou phénétyle chacun éventuellement substitué une ou deux fois par des restes de la liste $W^3$,

$R^{13}$ et $R^{14}$ représentent, indépendamment l'un de l'autre, l'hydrogène, un reste méthyle, éthyle, n-propyle, isopropyle, n-butyle, isobutyle, sec.-butyle, tertiobutyle, $-CH_2CF_3$, méthoxy, éthoxy, allyle, un reste cyclopropyle, cyclopentyle, cyclohexyle, cyclopropylméthyle, cyclopentylméthyle ou cyclohexylméthyle, chacun éventuellement substitué une ou deux fois par du fluor, du chlore, un radical méthyle, éthyle, n-propyle, isopropyle ou trifluorométhyle, un reste phényle, benzyle ou phénétyle éventuellement substitué une ou deux fois par des restes de la liste $W^3$, un reste $-OR^{12}$ ou $-NR^{11}R^{12}$,

$R^{15}$ représente, $-OR^{12}$, $-NR^{11}R^{12}$ ou $-N(R^{11})-COOR^{12}$,

$R^{16}$, $R^{17}$ et $R^{18}$ représentent indépendamment les uns des autres les restes méthyle, éthyle, n-propyle ou isopropyle,

$W^1$ représente l'hydrogène, le fluor, le chlore, le brome, un groupe cyano, formyle, nitro, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, $-CF_3$, $-CHF_2$, $-CClF_2$, $-CF_2CHFCl$, $-CF_2CH_2F$, $-CF_2CHF_2$, $-CF_2CCl_3$, $-CH_2CF_3$, $-CF_2CHFCF_3$, $-CH_2CF_2CHF_2$, $-CH_2CF_2CF_3$, trifluorométhoxy, difluorométhoxy, chlorodifluorométhoxy, acétyle, propionyle, butyryle, isobutyryle, méthoxycarbonyle, éthoxycarbonyle, n-propoxycarbonyle, isopropoxycarbonyle, n-butoxycarbonyle, isobutoxycarbonyle, sec.-butoxycarbonyle, tertio-butoxycarbonyle ou $-S(O)_oR^3$,

$W^2$ représente le fluor, le chlore, le brome, un groupe cyano, un reste méthyle, éthyle, n-propyle, isopropyle, trifluorométhyle, trifluorométhoxy, difluorométhoxy, chlorodifluorométhoxy, acétyle, trifluorométhylthio, $-CH{=}N{-}OCH_3$, $-CH{=}N{-}OC_2H_5$, $-CH{=}N{-}OC_3H_7$, $-C(CH_3){=}N{-}OCH_3$, $-C(CH_3){=}N{-}OC_2H_5$, $-C(CH_3){=}N{-}OC_3H_7$, $-C(C_2H_5){=}N{-}OCH_3$, $-C(C_2H_5){=}N{-}OC_2H_5$ ou $-C(C_2H_5){=}N{-}OC_3H_7$,

$W^3$ représente le fluor, le chlore, un groupe cyano, nitro, un reste méthyle, éthyle, méthoxy, éthoxy, méthylthio, trifluorométhyle, trifluorométhoxy, trifluorométhylthio, diméthylamino, diéthylamino, $-COOR^{19}$ ou $-CONR^{20}R^{21}$,

$R^{19}$ représente l'hydrogène, un reste méthyle, éthyle, n-propyle, isopropyle, tertiobutyle, $-CH_2CF_3$, un reste cyclopropyle, cyclopentyle ou cyclohexyle, chacun éventuellement substitué une ou deux fois par du fluor, du chlore, un radical méthyle, éthyle, n-propyle, isopropyle ou $-CF_3$, ou bien un reste phényle éventuellement substitué une ou deux fois par du fluor, du chlore, un radical méthyle, éthyle, n-propyle, isopropyle ou $-CF_3$, ou bien un reste phényle éventuellement substitué une ou deux fois par des restes de la liste $w^4$,

$R^{20}$ et $R^{21}$ représentent indépendamment l'un de l'autre de l'hydrogène, un reste méthyle, éthyle, n-propyle, isopropyle, n-butyle, isobutyle, sec.-butyle, tertiobutyle, $-CH_2CF_3$, méthoxy, éthoxy, allyle,

un reste cyclopropyle, cyclopentyle, cyclohexyle, cyclopropylméthyle, cyclopentylméthyle ou cyclohexylméthyle, chacun éventuellement substitué une ou deux fois par du fluor ou du chlore, un reste phényle, benzyle ou phénéthyle éventuellement substitué une ou deux fois par des restes de la liste $W^4$, un reste $-OR^{16}$ ou $-NR^{17}R^{18}$, et

$W^4$ représente le fluor, le chlore, le brome, un groupe cyano, nitro, un reste méthyle, éthyle, tertiobutyle, méthoxy, éthoxy, méthylthio, trifluorométhyle, trifluorométhoxy ou trifluorométhylthio.

**7.** Procédé de production de composés de formule (I) suivant la revendication 1, **caractérisé en ce que** :

A) on obtient des imines cycliques de formule (I)

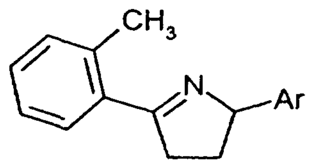

dans laquelle

Ar a les définitions indiquées dans la revendication 1,

a) en faisant réagir des dérivés aminocétoniques de formule (VIII)

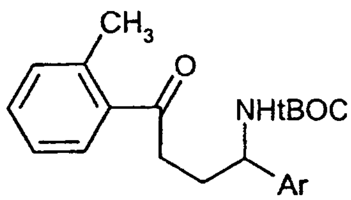

(VIII)

dans laquelle
Ar a les définitions indiquées dans la revendication 1,
avec un acide et en conduisant ensuite une cyclocondensation éventuellement en présence d'un accepteur d'acide, ou bien
b) en réduisant le groupe nitro de nitrocétones de formule (XVIII)

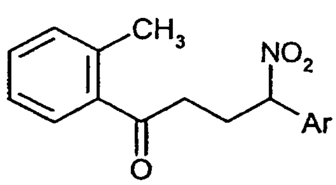

(XVIII)

dans laquelle
Ar a les définitions indiquées dans la revendication 1,

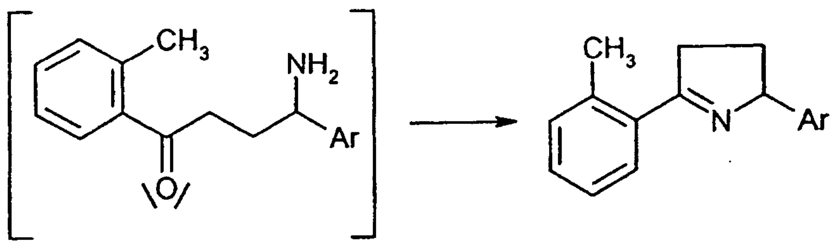

(II) (I)

avec formation intermédiaire d'une aminocétone de formule (II), mais que l'on cyclocondense in situ en composé (I), en particulier en milieu acide, ou bien

c) en hydrolysant avec des acides aqueux des imines de formule (XXVII)

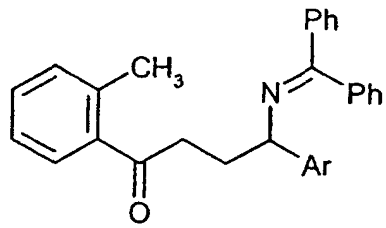

(XXVII)

dans laquelle

Ar a les définitions indiquées dans la revendication 1,

avec formation intermédiaire d'une aminocétone de formule (II),

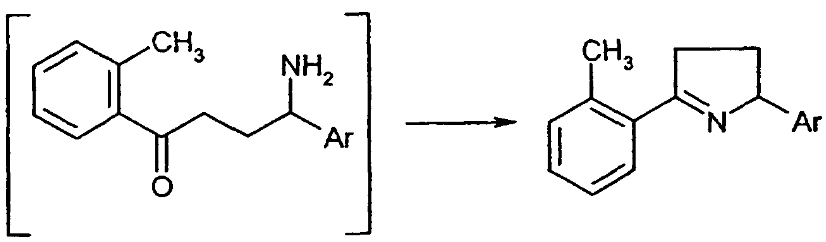

(II) (I)

mais que l'on cyclocondense in situ en composé (I), ou bien

B) on fait réagir des composés de formule (III)

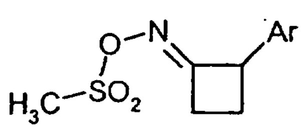

(III)

dans laquelle

Ar a les définitions indiquées dans la revendication 1,

avec un composé arylique de Grignard de formule (IV)

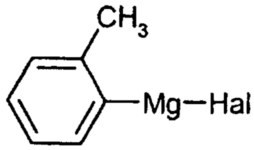

(IV)

dans laquelle

Hal représente le chlore, le brome ou l'iode,

en présence d'un diluant, ou bien
C) on obtient des composés de formule (I-b)

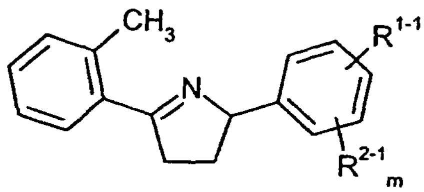

(I-b)

dans laquelle

m a les définitions indiquées dans la revendication 1,
$R^{1-1}$ représente A ou l'un des groupements suivants

(n-a)

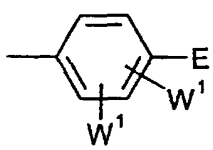

A, B, D, E, $W^1$ et Z ayant les définitions indiquées dans la revendication 1, et
$R^{2-1}$ représente l'hydrogène, le fluor, un groupe cyano, nitro, un reste alkyle, alkoxy, halogénalkyle, halogénalkoxy, alkoxyalkoxy ou $-SR_3$, où
$R_3$ a les définitions indiquées dans la revendication 1,

par couplage de composés de formule (V)

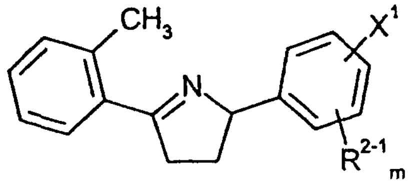

(V)

dans laquelle

$R^{2-1}$ a les définitions indiquées ci-dessus,
m a les définitions indiquées dans la revendication 1 et
$X^1$ représente le brome, l'iode ou un groupe $-OSO_2CF_3$, avec des acides boroniques de formule (VI)

$$R^{1-1}\text{-B-}(OH)2 \qquad (VI)$$

dans laquelle

$R^{1-1}$ a les définitions indiquées ci-dessus,

en présence d'un catalyseur et en présence d'un accepteur d'acide et en présence d'un solvant, ou bien D) on obtient des imines cycliques de formule (I-c)

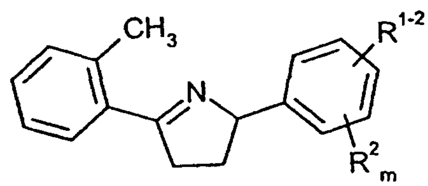

(I-c)

dans laquelle

$R^2$ et m ont les définitions indiquées dans la revendication 1,
$R^{1-2}$ représente l'un des groupements suivants

(m-b) $-B-Z-D^1$

(n-b) $-Y^1-E^1$

dans lesquels

B et Z ont les définitions indiquées dans la revendication 1,
$Y^1$ représente l'oxygène ou le soufre et
$D^1$ et $E^1$ représentent le groupement

$$-(CH_2)_p-(CR^9R^{10})_q-(CH^2)_r-G$$

dans lequel $R^9$, $R^{10}$, G, p, q et r ont les définitions indiquées dans la revendication 1, en condensant des imines cycliques de formule (I-d)

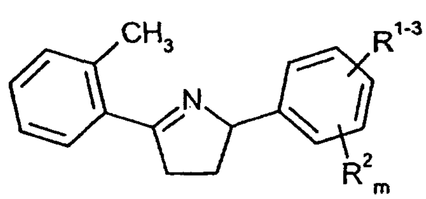

(I-d)

dans laquelle

$R^2$ et m ont les définitions indiquées dans la revendication 1 et
$R^{1-3}$ représentent l'un des groupements suivants

(m-c) -B-Z-H

(n-c) $-Y^1-H$

dans lesquels

B et Z ont les définitions indiquées dans la revendication 1 et
$Y^1$ a les définitions indiquées ci-dessus,

avec des composés de formule (VII)

$$Ab\text{-} (CH_2)_p\text{-} (CR^9R^{10})_q\text{-} (CH^2)_r\text{-}G \qquad (VII)$$

dans laquelle

$R^9$, $R^{10}$, G, p, q et r ont les définitions indiquées dans la revendication 1, et
Ab représente un groupe partant,

ou bien
E) on obtient des imines cycliques de formule (I-e)

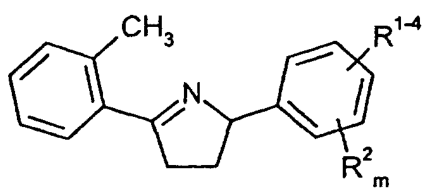

(I-e)

dans laquelle

$R^2$ et m ont les définitions indiquées dans la revendication 1 et
$R^{1-4}$ représente un groupement contenant le reste G venant de la description des composés de formule (I) conformes à l'invention,
G représentant l'un des groupements (e) à (k) mentionnés dans la revendication 1, par des dérivatisations généralement usuelles et connues des dérivés cétoniques, dérivés d'acides carbonyles ou nitryles correspondants, c'est-à-dire des composés de formule (I) dans lesquels G est un groupe cyano ou l'un des groupements (a) à (d).

**8.** Composés de formule (VIII)

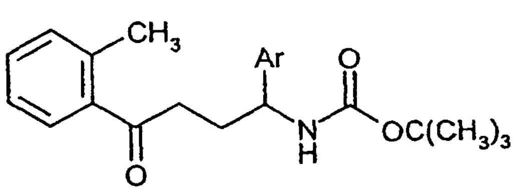

(VIII)

dans laquelle

Ar a les définitions indiquées dans la revendication 1.

**9.** Composés de formule (XVIII)

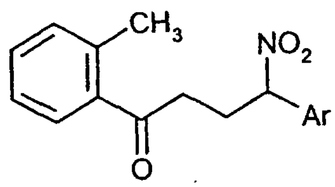

(XVIII)

dans laquelle

Ar a les définitions indiquées dans la revendication 1.

**10.** Composition pesticide, **caractérisée par** une teneur en au moins un composé de formule (I) suivant la revendication 1.

**11.** Utilisation de composés de formule (I) suivant la revendication 1 pour combattre des parasites.

**12.** Procédé de lutte contre des parasites, **caractérisé en ce qu'**on fait agir des composés de formule (I) suivant la revendication 1 sur les parasites et/ou sur leur milieu.

**13.** Procédé de préparation de compositions pesticides **caractérisé en ce qu'**on mélange des composés de formule (I) suivant la revendication 1 avec des diluants et/ou des agents tensioactifs.

**14.** Utilisation de composés de formule (I) suivant la revendication 1 pour la préparation de compositions pesticides.